# EUROPEAN PATENT SPECIFICATION

(11) **EP 3 972 965 B1**
(45) Date of publication and mention of the grant of the patent: **03.07.2024**
(21) Application number: 20809243.7
(22) Date of filing: 18.05.2020
(51) Int. Cl.: C07D 401/06, C07D 401/14, A61K 31/513, A61P 31/18

(54) **PYRIDINONE DERIVATIVES AS SELECTIVE CYTOTOXIC AGENTS AGAINST HIV INFECTED CELLS**
PYRIDINONDERIVATE ALS SELEKTIVE CYTOTOXISCHE MITTEL GEGEN HIV-INFIZIERTE ZELLEN
DÉRIVÉS DE PYRIDINONE UTILISÉS EN TANT QU'AGENTS CYTOTOXIQUES SÉLECTIFS CONTRE DES CELLULES INFECTÉES PAR LE VIH

(30) Priority: 22.05.2019 US 201962851269 P
(43) Date of publication of application: 30.03.2022
(73) Proprietor: Merck Sharp & Dohme LLC, Rahway, New Jersey 07065 (US)
(72) Inventor: CONVERSO, Antonella, West Point, Pennsylvania 19486 (US); EL MARROUNI, Abdellatif, West Point, Pennsylvania 19486 (US); FORSTER, Ashley, West Point, Pennsylvania 19486 (US); FRIE, Jessica, L., West Point, Pennsylvania 19486 (US); HUNTER, David, N., West Point, Pennsylvania 19486 (US); WANG, Cheng, West Point, Pennsylvania 19486 (US); WANG, Deping, West Point, Pennsylvania 19486 (US)
(74) Representative: Merck Sharp & Dohme LLC
(86) International application number: PCT/US2020/033358
(87) International publication number: WO 2020/236692

(56) References cited:
- WO-A1-2014/058747
- WO-A1-2015/153304
- WO-A1-2020/131597
- US-A1- 2010 056 535
- US-A1- 2010 256 181
- US-B2- 6 683 079

## Description

### BACKGROUND OF THE INVENTION

Human immunodeficiency virus (HIV) is the causative agent of acquired immunodeficiency syndrome (AIDS). In the absence of viral suppression, humans infected with HIV exhibit severe immunodeficiency which makes them highly susceptible to debilitating and ultimately fatal opportunistic infections. Multiple clinically approved antiretroviral drugs are available which demonstrate multi-log reductions in viral loads. Treated patients are at risk for acquiring mutations which render the virus in their bodies resistant to available therapies and rapid rebound of viremia is seen when therapy is removed, indicating that current regimens are not curative.

HIV is a retrovirus whose life cycle involves reverse transcription of a viral RNA genome into DNA via an enzyme known as reverse transcriptase and subsequent integration of the DNA copy into the host chromosomal DNA via the virally encoded integrase. Viral RNA is transcribed and viral proteins are translated using the host cellular machinery in conjunction with viral accessory proteins. Many viral proteins are contained within the GAG and GAG-POL proteins, with GAG containing structural proteins and GAG-POL resulting from a frameshift near the carboxy-terminus of GAG and containing protease (PR), reverse transcriptase (RT), and integrase (IN) viral enzymes, in addition to the structural proteins. GAG and GAG-POL are cleaved into individual proteins through the process of maturation which occurs during budding of virions from the infected cell. At this time GAG-POL dimerizes and the now dimeric HIV PR within the GAG-POL dimer forms an active enzyme which can cleave itself out of the polyprotein and catalyze further cleavage to form the remaining viral enzymes and structural proteins.

Available antiretroviral drugs act by blocking the virus at different stages in the viral life cycle. For example, reverse transcriptase inhibitors target the viral reverse transcriptase and prevent the RNA genome from being copied into DNA, integrase inhibitors block the ability of the copied DNA from being integrated into the host cell, and protease inhibitors prevent viral maturation so that virions produced from cells treated with protease inhibitors are immature and non-infectious. Once integration has occurred, a cell is infected until it dies through either normal cell death pathways, accelerated death due to viral factors, or is targeted by the immune system. While most infected cells are expected to die within ~2 days of being infected, the rapid rebound of viremia when therapy is removed is an indication that infected cells remain even after years on therapy (*See,* e.g., J. B. Dinoso et al., Proc. Natl. Acad. Sci. U.S.A., 2009, 106(23): 9403-9408). Thus, new therapies that can selectively kill the HIV infected cells would provide new treatment options for HIV infection. Treatment with compounds that can accelerate death of HIV infected cells and decrease the overall number of virally infected cells that persist within patients have the potential to decrease low-level viremia in suppressed patients and may also play a role in an HIV cure strategy. WO 2020/131597, WO 2015/153304 and WO 2014/058747 describe other compounds useful for the treatment of infection by HIV.

### SUMMARY OF THE INVENTION

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present disclosure is directed to pyridinone derivatives and their use as Small Molecule Activated Cell Kill (SMACK) agents which accelerate the death of HIV GAG-POL expressing cells without concomitant cytotoxicity to HIV naive cells. Accordingly, the compounds disclosed herein are useful for the treatment of infection by HIV, or for the treatment, prophylaxis or delay in the onset or progression of AIDS or AIDS Related Complex (ARC). Additionally, the compounds are useful for selectively killing HIV infected GAG-POL expressing cells in a subject infected with HIV. Compositions and methods of use comprising the compounds of this disclosure are also provided.

### DETAILED DESCRIPTION OF THE INVENTION

The present disclosure is directed to pyridinone derivative compounds and their use for accelerating the death of HIV GAG-POL expressing cells without concomitant cytotoxicity to HIV naive cells. In the absence of compounds such as those from the present invention, PR activation takes place during viral maturation and the concentration of mature PR in the cytoplasm is limited. In contrast, the present compounds promote the desired phenotype by catalyzing GAG-POL dimerization inside the infected cell by binding to the immature RT binding site and triggering premature activation of the HIV PR enzyme inside the host infected cell prior to budding. As a result, PR cleaves host substrates within the cell, leading to cytotoxicity and cell death. This effect can be blocked in the presence of an HIV protease inhibitor such as indinavir or darunavir demonstrating the role of HIV protease in the process.

The compounds presently disclosed herein also have activity as Non-Nucleoside Reverse Transcriptase Inhibitors (NNRTIs), due to the homology between the mature and immature RT pocket in HIV that allows the compounds to bind to the mature hydrophobic pocket near the active site of the viral RT enzyme. Binding to mature RT results in inhibition of enzymatic activity and production of the DNA provirus, which prevents infection of naive CD4+ T-cells.

While effects of NNRTIs on dimerization of RT and GAG-POL has been documented (Tachedjian et al. Proc. Natl. Acad. Sci. U.S.A. 2001, 98(13):7188; Tachedjian et al. FEES Lett. 2005, 579:379; Figueiredo etal. PLOS Path. 2006, 2(11):1051; Sudo etal. J. Virol. 2013, 87(6):3348), selective killing of HIV infected cells as a result of enhanced dimerization was first reported by Jochmans et al. (Jochmans et al. Retrovirology 2010, 7:89). The authors generated data showing these effects in chronically infected MT-4 cells, PBMCs, and CD4+ cells. Based on the potencies of tested molecules they concluded that "These data present proof of concept for targeted drug induced elimination of HIV producing cells. While NNRTIs themselves may not be sufficiently potent for therapeutic application, the results provide a basis for the development of drugs exploiting this mechanism of action." More recently, Zerbato *et al.* (Zerbato et al. Antimicrob. Agents Chemother. 2017, 61(3)) measured the activity of NNRTIs in a primary cell model for HIV latency. They saw significant reduction in virus production for certain NNRTIs compared to other classes of antiretrovirals and inferred that this was due to these compounds' ability to eliminate cells expressing HIV GAG-POL proteins.

The present disclosure is directed to a compound of Formula I: 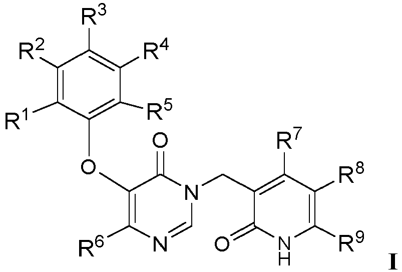 or a pharmaceutically acceptable salt thereof, wherein:
R¹ is H, halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F;
R2 is CN and R³ is H; or R² is H and R³ is CN;
R⁴ is -H, halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F;
R⁵ is -H, halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F;
R6 is halo or -C₁₋₆alkyl substituted with 1 to 9 of -F;
R⁷ is -H or -C₁₋₃alkyl;
R⁸ is -H, halo or -C₁₋₃alkyl; and
R⁹ is -H, pyrazolyl or -C₁₋₆alkyl unsubstituted or substituted with -OH or -OC₁₋₃alkyl.

In Embodiment 1 of this disclosure are compounds of Formula I having structural Formula II (wherein R² is CN and R³ is H): 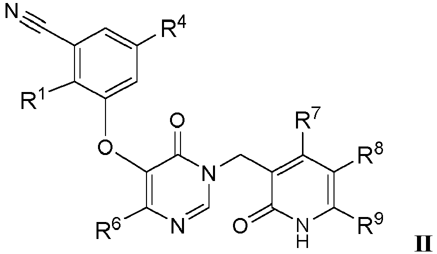 or pharmaceutically acceptable salts thereof, wherein R¹, R⁴, R⁶, R⁷, R⁸ and R⁹ are as defined in Formula I.

In Embodiment 2 of this disclosure are compounds of Formula I or Formula II, or pharmaceutically acceptable salts of each of the foregoing, wherein:
R⁴ is halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F. In class 1 thereof, R⁴ is halo or -C₁₋₃alkyl unsubstituted or substituted with 1 to 7 of F. In class 2 thereof thereof, R⁴ is halo, -CH₃ or -CH₃ substituted with 1 to 3 of F.

In Embodiment 3A of this disclosure are compounds of Formula I, Formula II, Embodiment 2, or any class thereof, or pharmaceutically acceptable salts of each of the foregoing, wherein R¹ is -H.

In Embodiment 3B of this disclosure are compounds of Formula I, Formula II, Embodiment 2, or any class thereof, or pharmaceutically acceptable salts of each of the foregoing, wherein R¹ is halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F. In class 1 thereof, R¹ is halo or -C₁₋₃alkyl unsubstituted or substituted with 1 to 7 of F. In class 2 thereof, R¹ is halo or CH₃.

In Embodiment 4 of this disclosure are compounds of Formula I having structural Formula III (wherein R² is H and R³ is CN): 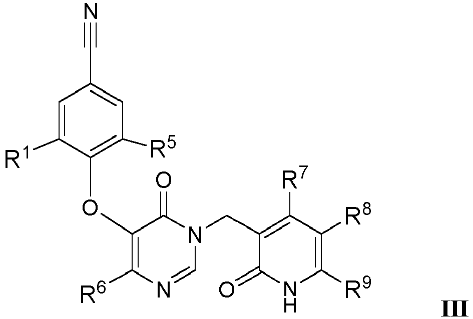 or pharmaceutically acceptable salts thereof, wherein R¹, R⁵, R⁶, R⁷ R⁸ and R⁹ are as defined in Formula I.

In Embodiment 5A of this disclosure are compounds of Formula I or Formula III, or pharmaceutically acceptable salts of each of the foregoing, wherein:
R¹ is halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F. In class 1 thereof, R¹ is halo or -C₁₋₃alkyl unsubstituted or substituted with 1 to 7 of F. In class 2 thereof, R¹ is halo or CH₃.

In Embodiment 5B of this disclosure are compounds of Formula I or Formula III, or pharmaceutically acceptable salts of each of the foregoing, wherein:
R5 is halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F. In class 1 thereof, R⁵ is halo or -C₁₋₃alkyl unsubstituted or substituted with 1 to 7 of F. In class 2 thereof, R⁵ is halo, -CH₃ or -CH₃ substituted with 1 to 3 of F.

In Embodiment 6 of this disclosure are compounds of Formula I, Formula II, Formula III, or Embodiment 2, 3A, 3B, 5A or 5B, or any class thereof, or pharmaceutically acceptable salts of each of the foregoing, wherein R⁶ is halo or -C₁₋₄alkyl substituted with 1 to 9 of -F. In class 1 thereof, R6 is halo or -C₁₋₃alkyl substituted with 1 to 7 of -F. In class 2 thereof, R⁶ is halo or -C₁₋₂alkyl substituted with 1 to 5 of -F.

In Embodiment 7 of this disclosure are compounds of Formula I, Formula II, Formula III, or Embodiment 2, 3A, 3B, 5A, 5B or 6, or any class thereof, or pharmaceutically acceptable salts of each of the foregoing, wherein R⁷ is H or -CH₃.

In Embodiment 8 of this disclosure are compounds of Formula I, Formula II, Formula III, Embodiment 2, 3A, 3B, 5A, 5B, 6 or 7, or any class thereof, or pharmaceutically acceptable salts of of each of the foregoing, wherein R⁸ is -H or halo.

In Embodiment 9 of this disclosure are compounds of Formula I, Formula II, Formula III, Embodiment 2, 3A, 3B, 5A, 5B, 6, 7 or 8, or any class thereof, or pharmaceutically acceptable salts of each of the foregoing, wherein R⁹ is -H, pyrazolyl or -C₁₋₄alkyl unsubstituted or substituted with -OH or -OC₁₋₃alkyl. In class 1 thereof, R⁹ is -H, pyrazolyl, or -C₁₋₃alkyl unsubstituted or substituted with -OH or -OC₁₋₃alkyl. In class 2 thereof, R⁹ is -H, pyrazolyl, or -C₁₋₃alkyl unsubstituted or substituted with -OH or -OCH₃.

In Embodiment 10 of this disclosure are compounds of Formula I or II, or pharmaceutically acceptable salts thereof, wherein
R¹ is H, halo or -C₁₋₃alkyl unsubstituted or substituted with 1 to 7 of F; for example R¹ is H, halo or CH₃;
R⁴ is halo, -CH₃ or -CH₃ substituted with 1 to 3 of F;
R⁶ is halo or -C₁₋₃alkyl substituted with 1 to 7 of -F, for example R⁶ is -C₁₋₂alkyl substituted with 1 to 5 of -F;
R⁷ is -H or -C₁₋₃alkyl, for example R⁷ is H or -CH₃;
R8 is -H, halo or -C₁₋₃alkyl, for example R⁸ is -H or halo; and
R⁹ is -H, pyrazolyl or -C₁₋₆alkyl unsubstituted or substituted with -OH or -OC₁₋₃alkyl, for example R⁹ is -H, pyrazolyl, or -C₁₋₃alkyl unsubstituted or substituted with -OH or -OCH₃.

In Embodiment 11 of this disclosure are compounds of Formula I or III, or pharmaceutically acceptable salts thereof, wherein
R¹ is halo or -C₁₋₃alkyl unsubstituted or substituted with 1 to 7 of F, for example R¹ is halo or CH3;
R5 is halo or -C₁₋₃alkyl unsubstituted or substituted with 1 to 7 of F, for example R⁵ is halo, -CH₃ or -CH₃ substituted with 1 to 3 of F, for example R⁵ is -CH₃;
R⁶ is halo or -C₁₋₃alkyl substituted with 1 to 7 of -F, for example R⁶ is -C₁₋₂alkyl substituted with 1 to 5 of -F;
R⁷ is -H or -C₁₋₃alkyl, for example R⁷ is H or -CH₃;
R⁸ is -H, halo or -C₁₋₃alkyl, for example R⁸ is -H or halo; and
R9 is -H, pyrazolyl or -C₁₋₆alkyl unsubstituted or substituted with -OH or -OC₁₋₃alkyl, for example R⁹ is -H, pyrazolyl, or -C₁₋₃alkyl unsubstituted or substituted with -OH or -OCH₃.

Reference to the compounds of Formula I herein encompasses the compounds of Formula I, II and III and the embodiments and classes thereof. The compounds of Formual I can be neutral compounds or in the form of a salt when such salts are possible, including pharmaceutically acceptable salts.

The term "e.g." means "for example." When the terms "e.g.," or "for example" are used herein, the example(s) recited are intended to be illustrative and are not intended to be an exhaustive list of all relevant examples.

As used herein, "alkyl" refers to both branched- and straight-chain saturated aliphatic hydrocarbon groups having the specified number of carbon atoms in a specified range. For example the term "C₁₋₆alkyl" means linear or branched chain alkyl groups, including all possible isomers, having 1, 2, 3, 4, 5 or 6 carbon atoms, and includes each of the hexyl and pentyl isomers as well as *n-, iso-, sec-* and *tert*-butyl (butyl, *i*-butyl, *s*-butyl, *t*-butyl, collectively "C₄alkyl"; Bu = butyl), *n*- and *iso*-propyl (propyl, *i*-propyl, collectively "C₃alkyl"; Pr = propyl), ethyl (Et) and methyl (Me). "C₁₋₄alkyl" has 1, 2, 3 or 4 carbon atoms and includes each of *n-, i-, s-* and *t*-butyl, *n-* and *i*-propyl, ethyl and methyl. "C₁₋₃alkyl" has 1, 2 or 3 carbon atoms and includes each of *n*-propyl, *i*-propyl, ethyl and methyl.

"Cycloalkyl" refers to a cyclized alkyl ring having the indicated number of carbon atoms in a specified range. Thus, for example, "C₃₋₆cycloalkyl" includes each of cyclopropyl, cyclobutyl, cyclopentyl and cyclohexyl, and "C₃₋₄cycloalkyl" includes each of cyclopropyl and cyclobutyl.

"Halo" or "halogen" refers to chloro, fluoro, bromo or iodo. Chloro, fluoro and bromo are a class of halogens of interest, and more particularly chloro and fluoro.

"HIV naive cell(s)" are cells that are not infected with HIV.

"Compatible anti-HIV agent(s)" are anti-HIV agents excluding HIV protease inhibitors.

A "stable" compound is a compound which can be prepared and isolated and whose structure and properties remain or can be caused to remain essentially unchanged for a period of time sufficient to allow use of the compound for the purposes described herein (e.g., therapeutic or prophylactic administration to a subject). The compounds of the present disclosure are limited to stable compounds embraced by Formula I and its embodiments. For example, certain moieties as defined in Formula I may be unsubstituted or substituted, and the latter is intended to encompass substitution patterns (i.e., number and kind of substituents) that are chemically possible for the moiety and that result in a stable compound.

This disclosure includes individual diastereomers, particularly epimers, i.e., compounds having the same chemical formula but which differ in the spatial arrangement around a single atom. This disclosure also includes mixtures of diastereomers, particularly mixtures of epimers, in all ratios. This disclosure encompasses compounds of Formula I having either the (*R*) or (*S*) stereo-configuration at an asymmetric center and at any additional asymmetric centers that may be present in a compound of Formula I, as well as stereo-isomeric mixtures thereof. Embodiments of this disclosure also include a mixture of enantiomers enriched with 51% or more of one of the enantiomers, including for example 60% or more, 70% or more, 80% or more, or 90% or more of one enantiomers. A single epimer is preferred. An individual or single enantiomers refers to an enantiomers obtained by chiral synthesis and/or using generally known separation and purification techniques, and which may be 100% of one enantiomers or may contain small amounts (e.g., 10% or less) of the opposite enantiomers. Thus, individual enantiomers are a subject of this disclosure in pure form, both as levorotatory and as dextrorotatory antipodes, in the form of racemates and in the form of mixtures of the two enantiomers in all ratios. In the case of a cis/trans isomerism this disclosure includes both the cis form and the trans form as well as mixtures of these forms in all ratios.

The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at an intermediate step during the synthesis of a compound of Formula I or it can be done on a final racemic product. Absolute stereochemistry may be determined by X-ray crystallography of crystalline products or crystalline intermediates which are derivatized, if necessary, with a reagent containing a stereogenic center of known configuration. Alternatively, absolute stereochemistry may be determined by Vibrational Circular Dichroism (VCD) spectroscopy analysis. The present disclosure includes all such isomers, as well as salts, solvates (which includes hydrates). and solvated salts of such racemates, enantiomers, diastereomers and tautomers and mixtures thereof.

As would be recognized by one of ordinary skill in the art, certain compounds of the present disclosure may be able to exist as tautomers. All tautomeric forms of such compounds, whether isolated individually or in mixtures, are within the scope of the present disclosure. For example, in instances where an oxo (=O) substituent is permitted on a heteroaromatic ring and keto-enol tautomerism is possible, it is understood that the substituent might in fact be present, in whole or in part, in the -OH form.

The atoms in a compound of Formula I may exhibit their natural isotopic abundances, or one or more of the atoms may be artificially enriched in a particular isotope having the same atomic number, but an atomic mass or mass number different from the atomic mass or mass number predominantly found in nature. The present disclosure is meant to include all suitable isotopic variations of the compounds of Formula I; for example, different isotopic forms of hydrogen (H) include protium (¹H) and deuterium (²H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds of Formula I can be prepared without undue experimentation by conventional techniques well known to those skilled in the art or by processes analogous to those described in the Schemes and Examples herein using appropriate isotopically-enriched reagents and/or intermediates.

The compounds can be administered in the form of pharmaceutically acceptable salts. The term "pharmaceutically acceptable salt" refers to a salt which possesses the effectiveness of the parent compound and which is not biologically or otherwise undesirable (e.g., is neither toxic nor otherwise deleterious to the recipient thereof). When the compounds of Formula I contain one or more acidic groups or basic groups, the invention includes the corresponding pharmaceutically acceptable salts.

Thus, the compounds of Formula I which contain one or more basic groups, i.e. groups which can be protonated, can be used according to the invention in the form of their acid addition salts with inorganic or organic acids as, for example but not limited to, salts with hydrogen chloride, hydrogen bromide, phosphoric acid, sulfuric acid, nitric acid, benzenesulfonic acid, methanesulfonic acid, p-toluenesulfonic acid, naphthalenedisulfonic acids, oxalic acid, acetic acid, trifluoroacetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, etc. If the compounds of Formula I simultaneously contain acidic and basic groups in the molecule the invention also includes, in addition to the salt forms mentioned, inner salts or betaines (zwitterions). Salts can be obtained from the compounds of Formula I by customary methods which are known to the person skilled in the art, for example by combination with an organic or inorganic acid or base in a solvent or dispersant, or by anion exchange or cation exchange from other salts. The present invention also includes all salts of the compounds of Formula I which, owing to low physiological compatibility, are not directly suitable for use in pharmaceuticals but which can be used, for example, as intermediates for chemical reactions or for the preparation of pharmaceutically acceptable salts.

The instant disclosure encompasses any composition comprised of a compound of Formula I or a compound that is a salt thereof, including for example but not limited to, a composition comprised of said compound associated together with one or more additional molecular and/or ionic component(s) which may be referred to as a "co-crystal." The term "co-crystal" as used herein refers to a solid phase (which may or may not be crystalline) wherein two or more different molecular and/or ionic components (generally in a stoichiometric ratio) are held together by non-ionic interactions including but not limited to hydrogen-bonding, dipole-dipole interactions, dipole-quadrupole interactions or dispersion forces (van der Waals). There is no proton transfer between the dissimilar components and the solid phase is neither a simple salt nor a solvate. A discussion of co-crystals can be found, e.g., in S. Aitipamula et al., Crystal Growth and Design, 2012, 12 (5), pp. 2147-2152.

Furthermore, compounds of the present disclosure may exist in amorphous form and/or one or more crystalline forms, and as such all amorphous and crystalline forms and mixtures thereof of the compounds of Formula I and salts thereof are intended to be included within the scope of the present disclosure. In addition, some of the compounds of the instant disclosure may form solvates with water (i.e., a hydrate) or common organic solvents. Such solvates and hydrates, particularly the pharmaceutically acceptable solvates and hydrates, of the compounds of this disclosure are likewise encompassed within the scope of the compounds defined by Formula I and the pharmaceutically acceptable salts thereof, along with un-solvated and anhydrous forms of such compounds.

Accordingly, the compounds of Formula I or salts thereof including pharmaceutically acceptable salts thereof, embodiments thereof and specific compounds described and claimed herein, encompass all possible stereoisomers, tautomers, physical forms (e.g., amorphous and crystalline forms), co-crystal forms, solvate and hydrate forms, and any combination of the foregoing forms where such forms are possible.

Another embodiment of the present disclosure is a compound of Formula I wherein the compound or its salt is in a substantially pure form. As used herein "substantially pure" means suitably at least about 60 wt.%, typically at least about 70 wt.%, preferably at least about 80 wt.%, more preferably at least about 90 wt.% (e.g., from about 90 wt.% to about 99 wt.%), even more preferably at least about 95 wt.% (e.g., from about 95 wt.% to about 99 wt.%, or from about 98 wt. % to 100 wt. %), and most preferably at least about 99 wt.% (e.g., 100 wt.%) of a product containing a compound of Formula I or its salt (e.g., the product isolated from a reaction mixture affording the compound or salt) consists of the compound or salt. The level of purity of the compounds and salts can be determined using a standard method of analysis such as, high performance liquid chromatography, and/or mass spectrometry or NMR techniques. If more than one method of analysis is employed and the methods provide experimentally significant differences in the level of purity determined, then the method providing the highest purity level governs. A compound or salt of 100% purity is one which is free of detectable impurities as determined by a standard method of analysis. With respect to a compound of the invention which has one or more asymmetric centers and can occur as mixtures of stereoisomers, a substantially pure compound can be either a substantially pure mixture of the stereoisomers or a substantially pure individual stereoisomer.

The compounds of Formula I herein, and pharmaceutically acceptable salts thereof, are useful for eliciting GAG-POL dimerization in HIV-infected cells and thereby selectively killing HIV infected GAG-POL expressing cells without concomitant cytotoxicity to HIV naive cells, referred to herein as Small Molecule Activated Cell Kill (SMACK) activity. Thus, the compounds of Formula I and pharmaceutically acceptable salts thereof are useful for:
(i) A method for the treatment of infection by HIV, or for the treatment, prophylaxis, or delay in the onset or progression of AIDS or ARC in a human subject in need thereof which comprises administering to the human subject an effective amount of the compound according to claim 1, or a pharmaceutically acceptable salt thereof;
(ii) A method for eliciting GAG-POL dimerization in HIV-infected cells in a human subject in need thereof which comprises administering to the human subject an effective amount of the compound according to claim 1, or a pharmaceutically acceptable salt thereof; and/or
(iii) A method for selectively killing HIV infected GAG-POL expressing cells without concomitant cytotoxicity to HIV naive cells in a human subject which comprises administering to the human subject an effective amount of the compound according to claim 1, or a pharmaceutically acceptable salt thereof.

Additionally, the compounds of Formula I and pharmaceutically acceptable salts thereof are useful for any of the methods (i), (ii) or (iii) above, further comprising administering to the human subject an effective amount of one or more compatible HIV antiviral agents selected from nucleoside or nucleotide HIV reverse transcriptase inhibitors, nucleoside reverse transcriptase translocation inhibitors, non-nucleoside HIV reverse transcriptase inhibitors, HIV integrase inhibitors, HIV fusion inhibitors, HIV entry inhibitors and HIV maturation inhibitors. In the methods of (i), (ii) and (iii) immediately above, the human subject is treated with a compound Formula I or a pharmaceutically acceptable salt thereof in addition to treatment with one or more compatible HIV antiviral agents.

The compounds of Formula I and pharmaceutically acceptable salts thereof are also useful for a method for augmenting the suppression of HIV viremia in a human subject whose viremia is being suppressed by administration of one or more compatible HIV antiviral agents, which comprises additionally administering to the human subject an effective amount of a compound according to claim 1, or a pharmaceutically acceptable salt thereof.

Other embodiments of the present disclosure include the following:
(a)A pharmaceutical composition comprising an effective amount of a compound of Formula I or a pharmaceutically acceptable salt thereof, and a pharmaceutically acceptable carrier.
(b) A pharmaceutical composition which comprises the product prepared by combining (e.g., mixing) an effective amount of a compound of Formula I or a pharmaceutically acceptable salt thereof, and a pharmaceutically acceptable carrier.
(c) The pharmaceutical composition of (a) or (b), further comprising an effective amount of one or more compatible anti-HIV agents selected from the group consisting of HIV antiviral agents, immunomodulators, and anti-infective agents.
(d) The pharmaceutical composition of (c), wherein the compatible anti-HIV agent is selected from one or more of an antiviral selected from the group consisting of nucleoside or nucleotide HIV reverse transcriptase inhibitors, nucleoside HIV reverse transcriptase translocation inhibitors, non-nucleoside HIV reverse transcriptase inhibitors, HIV integrase inhibitors, HIV fusion inhibitors, HIV entry inhibitors and HIV maturation inhibitors.
(e) A combination which is (i) a compound of Formula I or a pharmaceutically acceptable salt thereof and (ii) one or more compatible anti-HIV agents selected from the group consisting of HIV antiviral agents, immunomodulators, and anti-infective agents; wherein the compound and the compatible anti-HIV agent are each employed in an amount that renders the combination effective for the treatment of infection by HIV, or for the treatment, prophylaxis or delay in the onset or progression of AIDS or ARC.
(f) The combination of (e), wherein the compatible anti-HIV agent is an antiviral selected from the group consisting of nucleoside or nucleotide HIV reverse transcriptase inhibitors, nucleoside reverse transcriptase translocation inhibitors, non-nucleoside HIV reverse transcriptase inhibitors, HIV integrase inhibitors, HIV fusion inhibitors, HIV entry inhibitors and HIV maturation inhibitors.
(g) A method for eliciting GAG-POL dimerization in HIV-infected cells, a method for selectively killing HIV infected GAG-POL expressing cells without concomitant cytotoxicity to HIV naive cells, and/or a method for the treatment of infection by HIV, or for the treatment, prophylaxis, or delay in the onset or progression of AIDS or ARC, comprising administering an effective amount of a compound of Formula I or a pharmaceutically acceptable salt thereof to a subject in need of such treatment.
(h) The method of (g), wherein the compound of Formula I or a pharmaceutically acceptable salt thereof is administered in combination with an effective amount of at least one other compatible HIV antiviral selected from nucleoside or nucleotide HIV reverse transcriptase inhibitors, nucleoside reverse transcriptase translocation inhibitors, non-nucleoside HIV reverse transcriptase inhibitors, HIV integrase inhibitors, HIV fusion inhibitors, HIV entry inhibitors and HIV maturation inhibitors.
(i) The method of (g) or (h) comprising administering to the subject the pharmaceutical composition of (a), (b), (c) or (d) or the combination of (e) or (f).
(j) Use of a compound of Formula I or a pharmaceutically acceptable salt thereof in the manufacture of a medicament for (1) eliciting GAG-POL dimerization in HIV-infected cells in a subject; (2) selectively killing HIV infected GAG-POL expressing cells without concomitant cytotoxicity to HIV naive cells in a subject; (3) treatment of infection by HIV in a subject; (4) treatment, prophylaxis or delay in the onset or progression of AIDS or ARC in a subject; and/or (5) augmenting the suppression of HIV viremia in a subject undergoing treatment with a compatible anti-HIV agent.
(k) A compound of Formula I, or a pharmaceutically acceptable salt thereof, for use in (1) eliciting GAG-POL dimerization in HIV-infected cells; (2) selectively killing HIV infected GAG-POL expressing cells without concomitant cytotoxicity to HIV naive cells; (3) treatment of infection by HIV; (4) the treatment, prophylaxis or delay in the onset or progression of AIDS or ARC; and/or (5) augmenting the suppression of HIV viremia in a subject undergoing treatment with a compatible anti-HIV agent.

Additional embodiments of the present invention include each of the pharmaceutical compositions, methods and uses set forth in the preceding paragraphs, wherein the compound of Formula I or its salt employed therein in substantially pure. With respect to a pharmaceutical composition comprising a compound of Formula I or its salt and a pharmaceutically acceptable carrier and optionally one or more excipients, it is understood that the term "substantially pure" is in reference to a compound of Formula I or its salt per se.

An another embodiment of the present disclosure are the pharmaceutical compositions, methods and uses set forth above, wherein the HIV of interest is HIV-1. The term "administration" and variants thereof (e.g., "administering" a compound) in reference to a compound of Formula I means providing the compound to the individual in need of treatment or prophylaxis and includes both self-administration and administration to the patient by another person. When a compound is provided in combination with one or more other active agents (e.g., antiviral agents useful for treating or prophylaxis of HIV infection or AIDS), "administration" and its variants are each understood to include provision of the compound and other agents at the same time or at different times. When the agents of a combination are administered at the same time, they can be administered together in a single composition or they can be administered separately.

As used herein, the term "composition" is intended to encompass a product comprising the specified ingredients, as well as any product which results from combining the specified ingredients. Ingredients suitable for inclusion in a pharmaceutical composition are pharmaceutically acceptable ingredients, which means the ingredients must be compatible with each other and not deleterious to the recipient thereof.

The term "subject" or "patient" as used herein refers to an animal, preferably a mammal, most preferably a human, who has been the object of treatment, observation or experiment.

The term "effective amount" as used herein means an amount of a compound sufficient to elicit GAG-POL dimerization in HIV-infected cells and selectively kill HIV infected GAG-POL expressing cells without concomitant cytotoxicity to HIV naive cells; and/or exert a therapeutic effect, and/or a exert a prophylactic effect after administration. One embodiment of "effective amount" is a "therapeutically effective amount" which is an amount of a compound that is effective for selectively killing HIV infected GAG-POL expressing cells, treating HIV infection, or effective for the treatment or delay in the onset or progression of AIDS or ARC in a patient infected with HIV. Another embodiment of "effective amount" is a "prophylactically effective amount" which is an amount of the compound that is effective for prophylaxis of AIDS or ARC in an HIV-infected patient. It is understood that an effective amount can simultaneously be both a therapeutically effective amount, e.g., for treatment of HIV infection, and a prophylactically effective amount, e.g., for prevention or reduction of risk for developing AIDS or ARC in a subject infected with HIV. The term "prevention" as used herein refers to reducing the likelihood or severity of AIDS after HIV infection.

In the combination therapies of the present invention, an effective amount can refer to each individual agent or to the combination as a whole, wherein the amounts of all agents administered in the combination are together effective, but wherein a component agent of the combination may or may not be present individually in an effective amount with reference to what is considered effective for that component agent if it were administered alone.

In the methods of the present invention., (i.e., selectively killing HIV infected GAG-POL expressing cells, the treatment of infection by HIV, or the treatment, prophylaxis or delay in the onset or progression of AIDS or ARC), the compounds of this invention, or salts thereof, can be administered by means that produce contact of the active agent with the agent's site of action. They can be administered by conventional means available for use in conjunction with pharmaceuticals, either as individual therapeutic agents or in a combination of therapeutic agents. The compound can be administered itself, but typically is administered with a pharmaceutical carrier selected on the basis of the chosen route of administration and standard pharmaceutical practice. The compounds of the invention can, for example, be administered orally (e.g., via tablet or capsule), parenterally (including, e.g., subcutaneous injections, intravenous, intramuscular or intrasternal injection, or infusion techniques), by inhalation spray, or rectally, in the form of a unit dosage of a pharmaceutical composition containing an effective amount of the compound and conventional non-toxic pharmaceutically acceptable carriers, adjuvants and vehicles. The compound could also be administered via an implantable drug delivery device adapted to provide an effective amount of the compound or a pharmaceutical composition of the compound over an extended period of time.

### FORMULATIONS

Solid preparations suitable for oral administration (e.g., powders, pills, capsules and tablets) can be prepared according to techniques known in the art and can employ such solid excipients as starches, sugars, kaolin, lubricants, binders, disintegrating agents and the like. Liquid preparations suitable for oral administration (e.g., suspensions, syrups, elixirs and the like) can be prepared according to techniques known in the art and can employ any of the usual media such as water, glycols, oils, alcohols and the like. Parenteral compositions can be prepared according to techniques known in the art and typically employ sterile water as a carrier and optionally other ingredients, such as a solubility aid. Injectable solutions can be prepared according to methods known in the art wherein the carrier comprises a saline solution, a glucose solution or a solution containing a mixture of saline and glucose. Implantable compositions can be prepared according to methods known in the art wherein the carrier comprises the active chemical ingredient with polymers as suitable excipients, or utilizing an implantable device for drug delivery. Further description of methods suitable for use in preparing pharmaceutical compositions for use in the present invention and of ingredients suitable for use in said compositions is provided in Remington - The Science and Practice of Pharmacy, 22nd Edition, published by Pharmaceutical Press and Philadelphia College of Pharmacy at University of the Sciences, 2012, ISBN 978 0 85711-062-6 and prior editions.

Formulations of compounds of Formula I that result in drug supersaturation and/or rapid dissolution may be utilized to facilitate oral drug absorption. Formulation approaches to cause drug supersaturation and/or rapid dissolution include, but are not limited to, nanoparticulate systems, amorphous systems, solid solutions, solid dispersions, and lipid systems. Such formulation approaches and techniques for preparing them are known in the art. For example, solid dispersions can be prepared using excipients and processes as described in reviews (e.g., A.T.M. Serajuddin, J Pharm Sci, 88:10, pp. 1058-1066 (1999)). Nanoparticulate systems based on both attrition and direct synthesis have also been described in reviews such as Wu et al (F. Kesisoglou, S. Panmai, Y. Wu, Advanced Drug Delivery Reviews, 59:7 pp. 631-644 (2007)).

The compounds of Formula I may be administered in a dosage range of, e.g., 1 to 20 mg/kg, or 1 to 10 mg/kg, or about 5 mg/kg of mammal (e.g., human) body weight per day, or at other time intervals as appropriate, in a single dose or in divided doses. The compounds of Formula I may be administered in a dosage range of 0.001 to 2000 mg. per day in a single dose or in divided doses. Examples of dosage ranges are 0.01 to 1500 mg per day, or 0.1 to 1000 mg per day, administered orally or via other routes of administration in a single dose or in divided doses.

For oral (e.g., tablets or capsules) or other routes of administration, the dosage units may contain 100 mg to 1500 mg of the active ingredient, for example but not limited to, 100, 150, 200, 250, 300, 350, 400, 450, 500, 600, 700, 800, 900, 1000, 1100, 1200, 1300, 1400 or 1500 milligrams of the active ingredient for the symptomatic adjustment of the dosage to the patient to be treated. Furthermore, the compound may be formulated in oral formulations for immediate or modified release such as extended or controlled release. When the compound of Formula I is administered as a salt, reference to an amount of the compound in milligrams or grams is based on the free form (i.e., the non-salt form) of the compound.

Daily administration can be via any suitable route of administration but is preferably via oral administration and can be a single dose or more than one dose at staggered times (divided daily doses) within each 24 hour period. Each dose may be administered using one or multiple dosage units as appropriate.

The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy. In some cases, depending on the potency of the compound or the individual response, it may be necessary to deviate upwards or downwards from the given dose. The amount and frequency of administration will be regulated according to the judgment of the attending clinician considering such factors.

An "anti-HIV agent" is any agent which is directly or indirectly effective in the inhibition of HIV, the treatment or prophylaxis of HIV infection, and/or the treatment, prophylaxis or delay in the onset or progression of AIDS or ARC. It is understood that an anti-HIV agent is effective in treating, preventing, or delaying the onset or progression of HIV infection or AIDS and/or diseases or conditions arising therefrom or associated therewith. The present disclosure is additionally directed to use of a compound of Formula I with one or more compatible anti-HIV agents, i.e., anti-HIV agents excluding HIV protease inhibitors. For example, the compounds of Formula I may be administered in combination with effective amounts of one or more compatible anti-HIV agents selected from HIV antiviral agents, immunomodulators, antiinfectives, or vaccines useful for treating HIV infection or AIDS. Suitable compatible HIV antivirals for use in combination with the compounds of the present disclosure include, but are not limited to, those listed in Table A as follows:

**TABLE A: Antiviral Agents for Treating HIV infection or AIDS**

| Name | Type |
|---|---|
| abacavir, ABC, ZIAGEN^{®} | NRTI |
| abacavir +lamivudine, EPZICOM^{®} | NRTI |
| abacavir + lamivudine + zidovudine, TRIZIVIR^{®} | NRTI |
| AZT, zidovudine, azidothymidine, RETROVIR^{®} | NRTI |
| capravirine | NNRTI |
| ddC, zalcitabine, dideoxycytidine, HIVID^{®} | NRTI |
| ddI, didanosine, dideoxyinosine, VIDEX^{®} | NRTI |
| ddI (enteric coated), VIDEX EC^{®} | NRTI |
| delavirdine, DLV, RESCRIPTOR^{®} | NNRTI |
| dolutegravir, TIVICAY^{®} | InSTI |
| doravirine, PIFELTRO^{™} | NNRTI |
| doravirine/lamivudine/tenofovir disoproxil fumarate, DELSTRIGO^{™} | NNRTI/NRTI/NRTI |
| efavirenz, EFV, SUSTIVA^{®}, STOCRIN^{®} | NNRTI |
| Efavirenz/emtricitabine/tenofovir disoproxil fumarate, ATRIPLA^{®} | NNRTI/ NRTI/NRTI |
| EFdA (4'-ethynyl-2-fluoro-2'-deoxyadenosine) | NRTTI |
| Elvitegravir | InSTI |
| emtricitabine, FTC, EMTRIVA^{®} | NRTI |
| emtricitabine + tenofovir DF, TRUVADA^{®} | NRTI |
| emivirine, COACTINON^{®} | NNRTI |
| enfuvirtide, FUZEON^{®} | FI |
| enteric coated didanosine, VIDEX EC^{®} | NRTI |
| etravirine, TMC-125 | NNRTI |
| lamivudine, 3TC, EPIVIR^{®} | NRTI |
| lamivudine + zidovudine, COMBIVIR^{®} | NRTI |
| maraviroc, SELZENTRY^{®} | EI |
| nevirapine, NVP, VIRAMUNE^{®} | NNRTI |
| raltegravir, MK-0518, ISENTRESS^{™} | InSTI |
| Rilpivirine | NNRTI |
| stavudine, d4T,didehydrodeoxythymidine, ZERIT^{®} | NRTI |
| tenofovir disoproxil fumarate, VIREAD^{®} | NRTI |
| tenofovir alafenamide fumarate | NRTI |
| vicriviroc | EI |

| | |
|---|---|
| EI = entry inhibitor; FI = fusion inhibitor; InSTI = integrase inhibitor; NRTI = nucleoside or nucleotide reverse transcriptase inhibitor; NNRTI = non-nucleoside reverse transcriptase inhibitor; NRTTI = nucleoside reverse transcriptase translocation inhibitor. Some of the drugs listed in the table are used in a salt form; e.g., abacavir sulfate, delavirdine mesylate. | |

It is understood that the scope of combinations of the compounds of this invention with compatible anti-HIV agents is not limited to the HIV antivirals listed in Table A, but includes in principle any combination with any pharmaceutical composition useful for the treatment or prophylaxis of HIV AIDS, or ARC, with the exception of HIV protease inhibitors. The compatible HIV antiviral agents and other active agents will typically be employed in these combinations in their conventional dosage ranges and regimens as reported in the art, including, for example, the dosages described in the current Physicians' Desk Reference, Thomson PDR, 70th edition (2016), Montvale, NJ: PDR Network, or in prior editions thereof. The dosage ranges for a compound of the disclosure in these combinations can be the same as those set forth above.

The compounds of this invention are also useful in the preparation and execution of screening assays for antiviral compounds. For example, the compounds of this invention are useful for isolating enzyme mutants, which are excellent screening tools for more powerful antiviral compounds. Furthermore, the compounds of this invention are useful in establishing or determining the binding site of other antivirals to the reverse transcriptase region within GAG-POL, e.g., by competitive inhibition.

The following acronyms and abbreviations have the indicated meanings:

| |
|---|
| ACN = acetonitrile |
| AcOH = acetic acid; Ac = acetyl = -C(O)CH₃; aq. = aqueous |
| aq. = aqueous |
| B₂Pin₂ = bis(pinacolato)diboron |
| BPO = benzoyl peroxide |
| CAN = ceric amonium nitrate |
| cataCXium^{®} A Pd G2 precatalyst = chloro[(di(1-adamantyl)-*N*-butylphosphine)-2-(2-aminobiphenyl)]palladium(II) |
| DAST = (diethylamino)sulfur trifluoride |
| DCE = 1,2-dichloroethane |
| DCM = dichloromethane |
| DDQ = 2,3-Dichloro-5,6-dicyano-1,4-benzoquinone |
| DIAD = diisopropyl azodicarboxylate |
| DHP = 3,4-dihydro-2*H*-pyran |
| DIBAL-H = diisobutylaluminum hydride |
| DIPEA - diisopropylethylamine |
| DME = dimethoxyethane |
| DMF = *N*,*N*-dimethylformamide |
| Dess-Martin periodinane = 1,1,1-triacetoxy-1,1-dihydro-1,2-benziodoxol-3(1H)-one |
| DMA = Dimethylacetamide |
| DMSO = dimethyl sulfoxide |
| DMSO-*d₆*= deuterated dimethyl sulfoxide |
| e.g. = for example, but not limited to |
| EDC = 1-Ethyl-3-(3-dimethylaminopropyl)carbodiimide |
| EDTA = ethylenediaminetetraacetic acid |
| EtOAc = ethyl acetate |
| EtOH = ethanol |
| FBS = fetal bovine serum |
| GFP = Green fluorescent protein |
| HIV = human immunodeficiency virus |
| HOBt = hydroxybenzotriazole |
| HPLC = high performance liquid chromatography |
| h = hour |
| KI = potassium iodide |
| Ir [(Ome)(1,5-COD)]₂ = (1,5-Cyclooctadiene)(methoxy)iridium(I) dimer |
| L = liter; g = gram |
| LCAP = liquid chromatography area percent |
| LC-MS, LCMS = liquid chromatography-mass spectroscopy |
| LDA = lithium diisoproplyamide |
| LAH = lithium aluminium hydride |
| m-CPBA = 3-chloroperbenzoic acid |
| Me = methyl |
| MeI = methyl iodide |
| MeOH = methanol |
| Me-THF = 2-methyltetrahydrofuran |
| MTBE = methyl tertiary-butyl ether |
| min = minute |
| MS = mass spectroscopy |
| MsCl, Mesyl-Cl = methanesulfonyl chloride |
| NaIO₄ = sodium periodate |
| NBS = *N*-bromosuccinimide |
| n-BuLi = n-butyl lithium |
| NCS = *N*- chlorosuccinimide |
| NHS = normal human serum |
| NMP = *N*- methyl-2-pyrrolidinone |
| NMR = nuclear magnetic resonance |
| PBMC = peripheral blood mononuclear cell |
| PBS = phosphate buffered saline |
| Pd₂(dba)₃ = tris(dibenzylideneacetone)dipalladium(0) |
| PdCl₂(dppf) = [1,1'-bis(diphenylphosphino)ferrocene] dichloropalladium(II) |
| Pd(OAc)₂ = palladium(II) acetate |
| Pd(Ph₃P)₄ = tetrakis(triphenylphosphine)palladium(0) |
| PE, P.E. = petroleum ether |
| PHA = phytoheagglutinin |
| PMB = 4-methyoxybenzyl |
| PMBCl = 4-methoxybenzyl chloride |
| PPTS = 4-toluenesulfonic acid |
| RT = room temperature |
| sat. = saturated |
| SMACK - Small Molecule Activated Cell Kill |
| SN_{Ar} = nucleophilic aromatic substitution |
| TBAF = tetrabutylammonium fluoride |
| Tc = thiophene carboxylate |
| *t*-BuOH = *tert*-butanol |
| *t*-BuOK = potassum *tert*-butoxide |
| *t*-BuONO = *tert*-butyl nitrite |
| TEA = triethylamine |
| TFA = trifluoroacetic acid |
| TFAA = trifluoroacetic anhydride |
| THF = tetrahydrofuran |
| TLC = thin layer chromatography |
| TMS-Cl = trimethylsilyl chloride |
| X-Phos = 2-Dicyclohexylphosphino-2',4',6'-triisopropylbiphenyl |

Several methods for preparing the compounds of this invention are described in the following Schemes and Examples. Starting materials and intermediates are purchased or are made using known procedures, or as otherwise illustrated in the five Intermediate **(A, B**, **C, AB** and **BC)** sections that follow. A frequently applied route to the compounds of Formula I are described in the Schemes that follow.

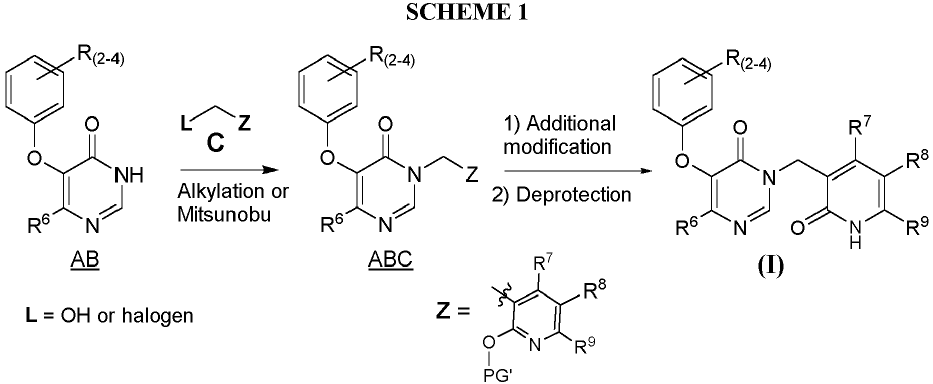

Scheme 1 depicts a method for preparing compounds of Formula I. Intermediate AB is prepared with procedures illustrated in the Intermediate **AB** section. Mitsunobu reaction or alkylation using an appropriate pyridinone benzylic alcohol or halide (Intermediate **C)** provides Intermediate **ABC.** Intermdiate **C** usually has a protected (PG') pyridinone moiety shown as **Z** in Scheme 1. Synthesis of **C** is illustrated in the Intermediate **C** section. The resulting Intermediate **ABC** may optionally undergo additional modification, followed by a deprotection step to afford compounds of Formula I.

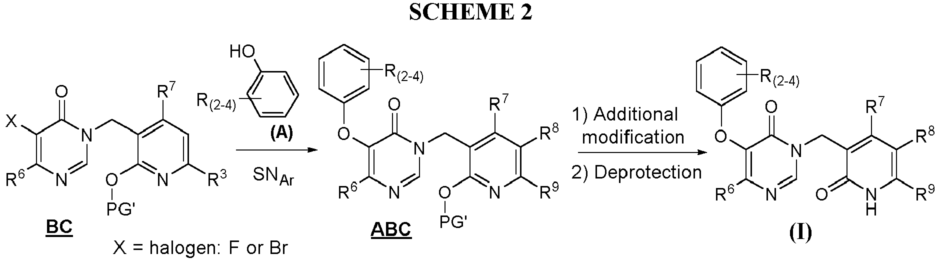

Scheme 2 illustrates another method for preparing compounds of Formula I. An SN_{A^{r}} reaction between an intermediate **A** (either commercially available or prepared using procedures in the Intermediate **A** section) and an Intermediate **BC** (made by procedures illustrated in the Intermediate **BC** section) is used to make Intermediate **ABC.** The pyridinone **C** ring is generally protected with a protecting group (PG') such as methyl or a PMB group. The resulting Intermediate **ABC** may then optionally undergo additional modification followed by a deprotection step to afford compounds of Formula I.

Reactions sensitive to moisture or air were performed under nitrogen or argon using anhydrous solvents and reagents. Reactions performed using microwave irradiation were normally carried out using an Emrys Optimizer manufactured by Personal Chemistry, or an Initiator manufactured by Biotage. Concentration of solutions was carried out on a rotary evaporator under reduced pressure.

The progress of reactions was determined by either analytical thin layer chromatography (TLC) usually performed with E. Merck pre-coated TLC plates, silica gel 60F-254, layer thickness 0.25 mm or analytical liquid chromatography-mass spectrometry (LC-MS). Typically, the analytical LC-MS system used consisted of a Waters ZQ^{™} platform with electrospray ionization in positive ion detection mode with an Agilent 1100 series HPLC with autosampler. The column was commonly a Waters Xterra MS C18, 3.0 × 50 mm, 5 *µ*m or a Waters Acquity UPLC^{®} BEH C18 1.0 × 50 mm, 1.7 *µ*m. The flow rate was 1 mL/min, and the injection volume was 10 ,uL. UV detection was in the range 210-400 nm. The mobile phase consisted of solvent A (water plus 0.05% TFA) and solvent B (acetonitrile plus 0.05% TFA) with a gradient of 100% solvent A for 0.7 min changing to 100% solvent B over 3.75 min, maintained for 1.1 min, then reverting to 100% solvent A over 0.2 min. LC/MS determinations were carried out on a Waters Classing Aquity system equipped with TUV and MS detectors and a Waters SQD mass spectrometer, a Shimadzu 20 UV 254 and 220nM with Shimadzu 2010 or 2020 mass spectrometer, or an Agilent 1200 HPLC quipped with DAD/ELSD and G6110 MSD using one of the following conditions: 1) Ascentis Express C18 (3 × 50 mm) 2.7µm column using mobile phase containing A: 0.05% Trifluoroacetic acid in water and B: 0.05% Trifluoroacetic acid in acetonitrile with a gradient from 90:10 (A:B) to 5:95 (A:B) over 6 min at a flow rate of 1.8 mL/min, UV detection at 210 nm; 2) Aquity BEH C18, (1.0 × 50 mm) 1.7 µm column using mobile phase containing A: 0.05% Trifluoroacetic acid in water and B: 0.05% Trifluoroacetic acid in acetonitrile with a gradient from 90:10 (A:B) to 5:95 (A:B) over 2 min at a flow rate of 0.3 mL/min, UV detection at 215 nm; 3) Agilent YMC J'Sphere H-80 (3 × 50 mm) 5µm column using mobile phase containing A: 0.1% Trifluoroacetic acid in water and B: acetonitrile with a gradient from 95:5 (A:B) to 0:100 (A:B) over 3.6 min and 0:100 (AB) for 0.4 min at a flow rate of 1.4 mL/min, UV detection at 254 and 220 nm and Agilent 1100 quadrupole mass spectrometer; 4) an Agilent TC-C18 (2.1 × 50 mm) 5µm column using mobile phase containing A: 0.0375% Trifluoroacetic acid in water and B: 0.01875% Trifluoroacetic acid in acetonitrile with a gradient from 90:10 (A:B) for 0.4 min to 90:10 to 0:100 (A:B) over 3 min and 10:90 (A:B) for 0.6 min at a flow rate of 0.8 mL/min, UV detection at 254 and 220 nm and Agilent 6110 quadrupole mass spectrometer.

Preparative HPLC purifications were usually performed using either a mass spectrometry directed system or a non-mass guided system. Usually they were performed on a Waters Chromatography Workstation configured with LC-MS System consisting of: Waters ZQ^{™} single quad MS system with Electrospray Ionization, Waters 2525 Gradient Pump, Waters 2767 Injecto /Collector, Waters 996 PDA Detector, the MS Conditions of: 150-750 amu, Positive Electrospray, Collection Triggered by MS, and a Waters SUNFIRE^{®} C-18 5 micron, 30 mm (id) × 100 mm column. The mobile phases consisted of mixtures of acetonitrile (10-100%) in water containing 0.1% TFA. Flow rates were maintained at 50 mL/min, the injection volume was 1800 ,uL, and the UV detection range was 210-400 nm. An alternate preparative HPLC system used was a Gilson Workstation consisting of: Gilson GX-281 Injector/Collector, Gilson UV/VIS-155 Detector, Gilson 333 and 334 Pumps, and equipped with a column selected from the following: Phenomenexd Synergi C18 (150mm × 30mm × 4 micron), YMC-Actus Pro C18 (150mm × 30mm × 5 micron), Xtimate C18 (150mm × 25mm × 5 micron), Boston Green ODS (150mm × 30mm × 5 micron), XSELECT C18 (150mm × 30mm × 5 micron), and Waters XSELECT C18 (150mm × 30mm × 5 micron). Conditions included either high pH (0-100% acetonitrile/water eluent comprising 0.1% v/v 10mM NH₄HCO₃ or 0.05% NH₄OH) or low pH (0-95% acetonitrile/water eluent comprising 0.1% v/v TFA). The injection volume ranged from 1000-8000 ,uL, and the UV detection range was 210-400 nm. Mobile phase gradients were optimized for the individual compounds.

Flash chromatography was usually performed using either a Biotage^{®} Flash Chromatography apparatus (Dyax Corp.), an ISCO CombiFlash^{®} Rf apparatus, or an ISCO CombiFlash^{®} Companion XL on silica gel (32-63 µm, 60 Å pore size) in pre-packed cartridges of the size noted.

SFC chiral resolution was carried out on a Sepiate Prep SFC 100, Multigram II (MG II) , THAR80 prep SFC, or a Waters SFC (80, 200, or 350) using conditions described in the experimental section.

Proton or ¹H NMR was acquired using a Varian Unity-Inova 400 MHz NMR spectrometer equipped with a Varian 400 ATB PFG 5mm, Nalorac DBG 400-5 or a Nalorac IDG 400-5 probe, a Varian-400MHz MR spectrometer equipped with an Auto X ID PFG Probe 5mm, a Varian 400MHz VNMRS spectrometer equipped with a PFG 4Nuc Probe 5 mm, or a Bruker AvanceIII 500MHz spectrometer equipped with a PABBO Probe 5 mm in accordance with standard analytical techniques, unless specified otherwise, and results of spectral analysis are reported. ¹H NMR spectra were acquired in CDCl₃ solutions unless otherwise noted. Chemical shifts were reported in parts per million (ppm). Tetramethylsilane (TMS) was used as internal reference in CD₃Cl solutions, and residual CH₃OH peak or TMS was used as internal reference in CD₃OD solutions. Coupling constants (J) were reported in hertz (Hz).

It is understood that a chiral center in a compound may exist in the "*S*" or "*R*" stereo-configuration, or as a mixture of both. Within a molecule, each bond drawn as a straight line from a chiral center encompasses each of the (*R*) and (*S*) stereoisomers as well as mixtures thereof unless otherwise noted. The compounds in Examples **32, 33** and **34** contain a chiral center. The isomer mixture made as described in each of the experimentals for these compounds was separated, providing one or both of an isomer A (the faster eluting isomer) and an isomer B (the slower eluting isomer), based on their observed elution order resulting from the separation as performed in the Example. Elution time and/or order of separated isomers may differ if performed under conditions different than those employed herein. Absolute stereochemistry (*R* or *S*) of the chiral center in each of the "A" and/or "B" separated stereoisomers in Examples **32, 33** and **34** was not determined, and "A" and "B" only refer to elution order resulting from the purification conditions as performed. An asterisk (*) may be used in the associated chemical structure drawings of the Example compounds to indicate a chiral center.

### Intermediate A Section

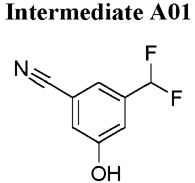

### 3-(difluoromethyl)-5-hydroxybenzonitrile

### Step 1: 3-chloro-5-((4-methoxybenzyl)oxy)benzonitrile

To a solution of 3-chloro-5-hydroxybenzonitrile (30 g, 0.20mol) in ACN (300 mL) was added PMBCl (34 g, 0.21 mol) and K₂CO₃ (55 g, 0.4 mmol), then the mixture was stirred at 70 °C for 4 h. The reaction mixture was filtered and concentrated in vacuum. The residue was purified by flash chromatography on silica (2% EtOAc/petroleum ether) to afford the title compound. ¹H NMR (400 MHz, CDCl₃): δ 7.33 (d, *J*= 8.0 Hz, 2H), 7.23 (s, 1H), 7.19 (s, 1H), 7.11 (s, 1H), 6.94 (d, *J*= 8.0 Hz, 2H), 5.00 (s, 2H), 3.83(s, 3H).

### Step 2: 3-((4-methoxybenzyl)oxy)-5-vinylbenzonitrile

To a solution of 3-chloro-5-((4-methoxybenzyl)oxy)benzonitrile, (50 g, 0.18 mol) in dioxane/H₂O (400 mL/80 mL) was added potassium vinyltrifluoroborate (25 g, 0.18 mol), K₂CO₃ (50 g, 0.36 mol), X-phos (17 g, 36 mmol) and Pd(OAc)₂ (4.1 g, 18 mmol) under N₂ atmosphere, the mixture was stirred at 80 °C for 2 h. After cooled to RT, the resulting mixture was filtered and extracted with EtOAc (3 × 400 mL). The combined organic layers were washed with brine, dried over Na₂SO₄, filtered and concentrated under reduced pressure. The residue was purified by flash chromatography on silica (1-2% EtOAc/petroluem ether) to afford the title compound. ¹H NMR (400 MHz, CDCl₃): δ 7.34 (d, *J*= 8.4 Hz, 2H), 7.28 (s, 1H), 7.20 (s, 1H), 7.09 (s, 1H), 6.93 (d, *J*= 8.4 Hz, 2H), 6.63 (dd, *J*= 17.6, 12.0 Hz, 1H), 5.78 (d, *J*= 17.6 Hz, 1H), 5.38 (d, *J*= 12.0 Hz, 1H), 5.01 (s, 2H), 3.82 (s, 3H).

### Step 3: 3-formyl-5-((4-methoxybenzyl)oxy)benzonitrile

To a stirred solution of compound 3-((4-methoxybenzyl)oxy)-5-vinylbenzonitrile, (28 g, 0.1 mol) in dioxane/H₂O (180 mL / 60 mL) was added 2,6-lutidine (22 g, 0.2 mol), OsO₄(1.3 g, 5 mmol) and NaIO₄ (43 g, 0.2 mol), the mixture was stirred at RT for 3 h. Upon reaction completion, the mixture was diluted with water, extracted with EtOAc (3×200 mL). The organic layer was washed with water and brine, dried over Na₂SO₄, filtered and concentrated under reduced pressure. The residue was purified by flash chromatography on silica (2-10% EtOAc/petroleum ether) to afford the title compound. ¹H NMR (400 MHz, DMSO-*d₆*): δ 9.95 (s, 1H), 7.90 (s, 1H), 7.82 (s, 1H), 7.76 (s, 1H), 7.37 (d, *J*= 8.4 Hz, 2H), 6.93 (d, *J*= 8.4 Hz, 2H), 5.14 (s, 2H), 3.73 (s, 3H).

### Step 4: 3-(difluoromethyl)-5-((4-methoxybenzyl)oxy)benzonitrile

To a stirred solution of 3-formyl-5-((4-methoxybenzyl)oxy)benzonitrile, (22 g, 82 mmol) in DCM (250 mL) was added DAST (106 g, 0.6 mol) at 0°C under N₂ atmosphere, then the mixture was stirred for 3 h at RT. Upon reaction completion, the mixture was quenched with water, extracted with DCM (3 × 200 mL). The organic layer was washed with water and brine, dried over Na₂SO₄, filtered and concentrated under reduce pressure to give the title compound.

### Step 5: 3-(difluoromethyl)-5-hydroxybenzonitrile

A solution of 3-(difluoromethyl)-5-((4-methoxybenzyl)oxy)benzonitrile (23.7 g, 82 mmol) in TFA/TFAA (100 mL/ 50 mL) was stirred at 110 °C for 3 h. When reaction was complete, the resulting mixture was quenched with sat. NaHCO₃ aq. (100 mL) and extracted with EtOAc (3 × 150 mL). The combined organic layers were washed with brine, dried over Na₂SO₄, filtered and concentrated under reduced pressure to afford the title compound. ¹H NMR (400 MHz, DMSO-*d₆*): δ 10.66 (s, 1H), 7.41 (s, 1H), 7.28 (s, 1H), 7.23 (s, 1H), 6.97 (t, *J*= 56.0 Hz, 1H).

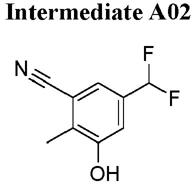

### 5-(difluoromethyl)-3-hydroxy-2-methylbenzonitrile

### Step 1: 5-formyl-2-methylbenzonitrile

Into a 20-L 4-necked round-bottom flask purged and maintained with an inert atmosphere of nitrogen, was placed a solution of 5-bromo-2-methylbenzonitrile (750 g, 38.26 mol) in tetrahydrofuran (6 L), n-BuLi (1.54 L, 38.26 mol) was added dropwise at -78o C, the resulting solution was stirred for 30 min, the N,N-dimethylformamide (295 g, 4.04 mol, 1.05 equiv) was added dropwise. And the reaction mixture was stirred at -78 °C for 30 min in a liquid nitrogen bath, and then quenched by the addition of 5 L of sat. NH₄Cl aq. The resulting solution was extracted with EtOAc (3 × 5 L). The organic layer was washed with brine, dried over Na₂SO₄, filtered and concentrated under reduced pressure to give the title compound.

### Step 2: 3-bromo-5-formyl-2-methylbenzamide

Into a 3-L 4-necked round-bottom flask purged and maintained with an inert atmosphere of nitrogen, was placed a solution of 5-formyl-2-methylbenzonitrile (245 g, 1.69 mol) in sulfuric acid (980 mL), the reaction mixture was stirred at 60 ° C, 1-bromopyrrolidine-2,5-dione (300 g, 1.69 mol) was added in 3 batches. The resulting solution was stirred at 60°C for 30 min. The reaction was then quenched by the addition of 5 L of water/ice, and stirred for 1 h. The solids were collected by filtration and dried under vacuum to afford the title compound and use as is in the next step.

### Step 3: 3-bromo-5-formyl-2-methylbenzonitrile

Into a 20-L 4-necked round-bottom flask purged and maintained with an inert atmosphere of nitrogen, was placed a solution of 3-bromo-5-formyl-2-methylbenzamide (500 g (crude product from Step 2, 2.07 mol) in dichloromethane (10 L), pyridine (524.5 g, 6.63 mol). While the resulting solution was stirred at 0°C, the 2,2,2-trifluoroacetate (1305 g, 6.21 mol) was added dropwise. The resulting solution was stirred for 30 min at RT, then quenched with water/ice (5 L), and extracted with DCM (3 × 5 L). The organic layers were combined and dried over anhydrous Na₂SO₄, filtered and concentrated under reduced pressure. The residue was purified with flash chromatography on silica (20% EtOAc/hexanes) to afford the title compound.

### Step 4: 3-bromo-5-(difluoromethyl)-2-methylbenzonitrile

To a solution of 3-bromo-5-formyl-2-methylbenzonitrile (360 g, 1.61 mol) in dichloromethane (5.4 L), DAST (260 g, 1.61 mol, 1.00 equiv) was added dropwise atRT. The resulting solution was stirred at RT for 1 h, and then quenched with water/ice (3 L). The resulting solution was extracted with DCM (3 × 5 L). The organic layers were combined and dried over anhydrous Na₂SO₄, filtered and concentrated under reduced pressure to afford the title compound and used as is in the following step.

### Step 5: 5-(difluoromethyl)-3-hydroxy-2-methylbenzonitrile

To a solution of 3-bromo-5-(difluoromethyl)-2-methylbenzonitrile (320 g, 1.30 mol) in 1,4-dioxane (1.6 L), was added solution of KOH (146 g, 2.60 mol) in water (1.6 L), Pd₂(dba)₃ (67 g, 64.92 mmol), and 2-di-tert-butylphosphino-2',4',6'-triisopropylbiphenyl (55 g, 129.53 mmol). The resulting mixture was purged by nitrogen (3 ×) and stirred at 90 °C for 1 h, and then partitioned between ice water (4 L) and EtOAc (3 × 2 L). The combined organic layers were washed with brine, dried over anhydrous Na₂SO₄, filtered and concentrated under reduced pressure. The residue was purified with flash chromatography on silica (33% EtOAc/hexanes) to afford the title compound. MS: 182 (M+1). ¹H NMR (400 MHz, DMSO-*d₆*): δ 10.65 (s, 1H), 7.42 (d, *J*= 1.6 Hz, 1H), 7.26 (d, *J*= 1.5 Hz, 1H), 6.99 (s, 1H), 2.34 (d, *J*= 1.3 Hz, 3H).

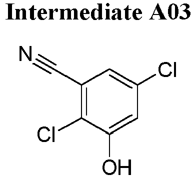

### 2,5-dichloro-3-hydroxybenzonitrile

### Step 1: 2-amino-5-bromo-3-fluorobenzonitrile

To a stirred solution of 2-amino-3-fluorobenzonitrile (5 g, 36.7 mmol) in DMF (50 mL) was added 1-chloropyrrolidine-2,5-dione (5.15 g, 38.6 mmol) and the resulting mixture was stirred at 60°C for 6 h. The mixture was partitioned betweem water (200 mL) and EtOAc (2×150 mL). The combined organic layers were washed with brine (3×300 mL), dried with anhydrous Na₂SO₄, filtered and concentrated under reduced pressure to give the title compound, which was used for the next step without purification. MS: 171.1 (M+1).

### Step 2: 2,5-dichloro-3-fluorobenzonitrile

To a mixture of 2-amino-5-chloro-3-fluorobenzonitrile (6.217 g, 36.4 mmol), copper(I) chloride (10.82 g, 109 mmol) and copper(II) chloride (17.15 g, 128 mmol) in ACN (60 mL), was added tert-butyl nitrite (17.34 ml, 146 mmol) at 25°C for 1 h. The reaction mixture was diluted with water (180 mL) and extracted with EtOAc (3 × 120 mL). The organic layer was washed with brine, dried over anhydrous Na₂SO₄, filtered and concentrated under reduced pressure. The residue was purified by flash chromatography on silica (10 - 100% EtOAc/petroleum ether) to afford the title compound. ¹H NMR (400 MHz, chloroform-*d*): δ ppm 7.51 (dd, *J*=2.26, 1.65 Hz, 1 H), 7.44 - 7.47 (m, 1 H).

### Step 3: 2,5-dichloro-3-((4-methoxybenzyl)oxy)benzonitrile

To a stirred solution of (4-methoxyphenyl)methanol (2.75 g, 19.89 mmol) in DMF (40 mL) was added NaH (1.034 g, 25.9 mmol) at 0°C. The mixtue was stirred at 0°C for 30 min, then was added 2,5-dichloro-3-fluorobenzonitrile (3.78 g, 19.89 mmol), and then stirred at 25 °C for 2 h. The reaction mixture was diluted with water (200 mL) and extracted with EtOAc (3 × 150 mL). The organic layer was washed with brine, dried over anhydroud Na₂SO₄, filtered and concentrated to give the title compound. MS: 308.1 (M+1).

### Step 4: 2,5-dichloro-3-hydroxybenzonitrile

To a stirred solution of 2,5-dichloro-3-((4-methoxybenzyl)oxy)benzonitrile (6.13 g, 19.89 mmol) in DCM (60 mL), TFA (20 mL) was and then stirred at 25 °C for 1 h. Upon the completion, the reaction mixture was poured into sat. NaHCO₃ aq(30 mL), extracted with DCM (3 × 60 mL). The combined organic layers were washed with brine, dried over anhydrous Na₂SO₄, filtered and concentrated under reduced pressure to give the title compound, which was used directly without further purification. MS: 187.9 (M-1).

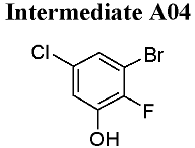

### 3-bromo-5-chloro-2-fluorophenol

### Step 1: 2-(3-bromo-5-chloro-2-fluorophenyl)-4,4,5,5-tetramethyl-1,3,2-dioxaborolane

To a solution of 2-bromo-4-chloro-1-fluorobenzene (300 g, 1.43 mol) in hexane (4.5 L), was added B₂Pin₂ (363.7 g, 1.43 mol), Ir [(Ome) (1,5-COD)]₂ (14.2 g, 21.42 mmol), 4-tert-butyl-2-(4-tert-butylpyridin-2-yl)pyridine (11.5 g, 42.85 mmol) after it was purged with nitrogen three times. The resulting solution was stirred at RT overnight at and then concentrated under vacuum. The residue was purified by flash chromatography on silica (0-100% EtOAc/petroleum ether) to obtain the title compound.

### Step 2: 3-bromo-5-chloro-2-fluorophenol

To a solution of 2-(3-bromo-5-chloro-2-fluorophenyl)-4,4,5,5-tetramethyl-1,3,2-dioxaborolane (385 g, 1.15 mol) in THF (3.85 L), was added a solution of sodium hydroxide (138 g, 3.45 mol) in water (3 L), 35% hydrogen peroxide aq. (390 g, 3.44 mol) at 0°C. The resulting solution was stirred at 0 °C for 2 h, and quenched by with sat. Na₂SO₃ aqueous solution. After the pH of the solution was adjusted to 3-4 with HCl (1 M), the resulting solution was extracted with EtOAc (3 × 3 L). The combined organic layers were washed with brine, then concentrated under reduced pressure. The residue was purified by flash chromatography on silica (0 - 90% EtOAc/petroleum ether) to afford the title compound. ¹H NMR (400 MHz, CDCl₃): δ 11.31 (br. s., 1H), 7.47 (dd, *J*= 2.4, 4.4 Hz, 1H), 7.30 (dd, *J*= 2.4, 7.6 Hz, 1H).

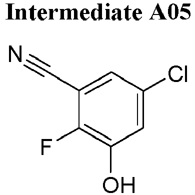

### 5-chloro-2-fluoro-3-hydroxybenzonitrile

To a solution of 3-bromo-5-chloro-2-fluorophenol (86 g, 343 mmol) in NMP (860 mL), was added CuCN (154 g, 1.72 mol) in one portion. The resulting reaction mixture was stirred at 180 °C for 2 h, then partitioned between ice water (1 L) and EtOAc (3 × 800 mL). The combined organic layers were washed with brine, dried over anhydrous Na₂SO₄, filtered and concentrated under reduced pressure. The residue was purified by flash chromatography on silica (2 - 33% EtOAc/petroleum ether) to afford the title compound. ¹H NMR (400 MHz, DMSO-*d₆*): δ 11.33 (br. s., 1H), 7.49 (dd, *J*= 2.5, 4.5 Hz, 1H), 7.30 (dd, *J*= 2.5, 7.6 Hz, 1H).

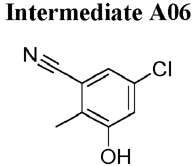

### 5-chloro-3-hydroxy-2-methylbenzonitrile

### Step 1: 2,3-difluorobenzaldehyde

To a solution of 1,2-difluorobenzene (1668 g, 14.62 mol) in THF (16.7 L) cooled at -78 °C, was added a THF solution of n-BuLi (6.44 L, 16.08 mol) dropwise with stirring over 60 min. Then to this mixture, was added DMF (5340 g, 73.06 mol) dropwise with stirring at -78°C over 60 min. The resulting solution was stirred at -78°C for 1h. The reaction was then quenched with sat. NH₄Cl aq (10 L), then extracted with EtOAc (3 × 10 L). The combined organic layers were washed with brine, dried over anhydrous Na₂SO₄, filtered and concentrated under reduced pressure. The residue was purified by flash chromatography on silica (2% EtOAc/petroleum ether) to afford the title compound.

### Step 2: (E)-N-[(2,3-difluorophenyl)methylidene]hydroxylamine

A mixture of 2,3-difluorobenzaldehyde (2410 g, 16.96 mol) in a 70% solution of NH₂OH (672 g, 20.35 mol) in DMF (10 L) was stirred at 20 °C for 3 h, then partitioned between water (6 L) and EtOAc (3 × 8 L). The combined organic layers were washed with brine, dried over anhydrous Na₂SO₄, filtered and concentrated under reduced pressure. The residue was purified by flash chromatography on silica (2% EtOAc/petroleum ether) to afford the title compound.

### Step 3: 2,3-difluorobenzonitrile

A solution of (*E*)-N-[(2,3-difluorophenyl)methylidene]hydroxylamine (2025 g, 12.89 mol) in DMF (11 L) was treated with POCl₃ (5688 g, 37.10 mol). The resulting solution was stirred at 25°C for 3 h, then partitioned between water (6 L) and EtOAc (3 × 6 L). The combined organic layers were washed with brine, dried over anhydrous Na₂SO₄, filtered and concentrated under reduced pressure. The residue was purified by flash chromatography on silica (2% EtOAc/petroleum ether) to afford the title compound.

### Step 4: 2-amino-3-fluorobenzonitrile

In a seal reactor, a solution of 2,3-difluorobenzonitrile (1273 g, 9.15 mol) in EtOH (13 L) was bubbled in NH₃(gas). The resulting solution was stirred at 140°C for 8 h, cooled to RT then concentrated under reduced pressure. The residue was partitioned between water (5 L) and MTBE (3×8L). The combined organic layers were washed with water, brine and dried over anhydrous Na₂SO₄, filtered and concentrated under reduced pressure to afford the title compound.

### Step 5: 2-amino-5-chloro-3-fluorobenzonitrile

A solution of 2-amino-3-fluorobenzonitrile (934 g, 6.86 mol) in DMF (14 L) was treated with NCS (1008 g, 7.55 mol). The resulting solution was stirred at 45°C for 2 h, then partitioned between ice water (20 L) and EtOAc (3×12 L). The organic layer was washed with brine, dried over anhydrous Na₂SO₄, filtered and concentrated under reduced pressure. The residue was purified by flash chromatography on silica (3% EtOAc/petroleum ether) to afford the title compound.

### Step 6: 2-bromo-5-chloro-3-fluorobenzonitrile

To a mixture of *t*-BuONO (815 g, 7.88 mol) and CuBr₂ (1365 g, 6.13 mol) in ACN (8 L), was added a solution of 2-amino-5-chloro-3-fluorobenzonitrile (747 g, 4.38 mol) in ACN (7 L) dropwise with stirring over 90 min. The resulting mixture was stirred at RT for 10 h, and diluted with water (15 L), and then extracted with EtOAc (3 × 10 L). The organic layer was washed with brine, dried over anhydrous Na₂SO₄, filtered and concentrated under reduced pressure. The residue was purified by flash chromatography on silica (3% EtOAc/petroleum ether) to afford the title compound.

### Step 7: 5-chloro-3-fluoro-2-methylbenzonitrile

A mixture of 2-bromo-5-chloro-3-fluorobenzonitrile (768 g, 3.28 mol), 1,4- methylboronic acid (297 g, 4.96 mol), potassium carbonate (913 g, 6.61 mol) and PdCl₂(dppf) (213 g, 0.33 mol) in degassed 1,4-dioxane (4500 mL) and water (450 mL) was stirred at 100°C for 60 min, and then diluted with water (4L), and extrated with EtOAc (3 × 6 L). The organic layer was washed with brine, dried over anhydrous Na₂SO₄, filtered and concentrated under reduced pressure. The residue was purified by flash chromatography on silica (3% EtOAc/petroleum ether) to afford the title compound.

### Step 8: 5-chloro-3-[(4-methoxyphenyl)methoxy]-2-methylbenzonitrile

Under atmosphere of nitrogen, to a solution of 4-Methoxybenzyl alcohol (410 g, 2.97 mol) in DMF (3200 mL), was added sodium hydride (128 g, 5.33 mol), followed by addition of a solution of 5-chloro-3-fluoro-2-methylbenzonitrile (418 g, 2.46 mol) in DMF (800 mL) dropwise. The resulting solution was stirred at 25°C for 12 h, then diluted with ice water (2 L), and extracted with EtOAc (3 × 3L). The organic layer was washed with brine, dried over anhydrous Na₂SO₄, filtered and concentrated under reduced pressure. The resulting solid was washed with petroleum ether and collected on top of a filter to afford the title compound.

### Step 9: 5-chloro-3-hydroxy-2-methylbenzonitrile

A solution of 5-chloro-3-[(4-methoxyphenyl)methoxy]-2-methylbenzonitrile (518 g, 1.80 mol) in DCM (1500 mL) was treated with TFA (500 mL). The resulting solution was stirred at 25°C for 1 h. The solids were collected on top of a filter to afford the title compound. MS: 166 (M-1). ¹H NMR (400 MHz, DMSO-*d₆*): 10.67 (s, 1H), 7.32-7.33 (d, 1H), 7.07-7.08 (d, 1H), 2.24 (s, 3H).

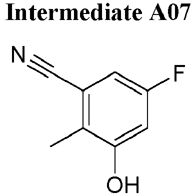

### 5-fluoro-3-hydroxy -2 -methylbenzonitrile

### Step 1 : 3-bromo-5-fluoro-2-methylaniline

To a solution of 1-bromo-5-fluoro-2-methyl-3-nitrobenzene (23g, 98 mmol) in ethanol (390 mL), was added ammonia hydrochloride (26.3 g, 491 mmol) and water (38.3 ml, 2126 mmol) followed by addition of iron (27.4 g, 491 mmol). The resulting mixture was stirred at 90 °C for 6 h. After completion of the reaction monitored by TLC, the reaction mixture was filtered through a sintered funnel. The filtrate was evaporated under reduced pressure. The residue was purified by column chromatography on silica (5% EtOAc/petroleum ether) to afford the title compound. MS: 204.2 and 206.2 (M+1).

### Step2: 3-bromo-5-fluoro-2-methylphenol

To a solution of sulfuric acid (145 mL, 2729 mmol) in water (21.73 mL, 1206 mmol) was added 3-bromo-5-fluoro-2-methylphenol (7g, 31.1 mmol). The resulting mixture was cooled with an ice bath to 0 °C. Then a solution of sodium nitrate (6.66 g, 78 mmol) in water was added dropwise. After stirred at this temperature for 30 minutes, the reaction mixture was heated at 100 °C for 30 minutes, and then diluted with water and extracted with EtOAc. The combined organic layers were washed with brine and dried over anhydrous Na₂SO₄ , filtered and concentrated under reduced pressure. The residue was purified by column chromatography on silica (4 -10% EtOAc/petroleum ether) to afford the title compound. MS: 203.2 and 205.2 (M-1).

### Step3: 5-fluoro-3-hydroxy-2-methylbenzonitrile

To a stirred solution of 3-bromo-5-fluoro-2-methylphenol (7g, 34.1 mmol) in DMA (140 mL), was added zinc dust (4.46 g, 68.3 mmol) and dppf (3.79 g, 6.83 mmol). The resulting mixture was degassed with nitrogen for 10 minutes, and then Pd₂(dba)₃ (1.563 g, 1.707 mmol) and zinc cyanide (8.02 g, 68.3 mmol) was added. The reaction vessle was sealed. And the resulting mixture was heated to 140 °C for 6 h, and then diluted with water (200 mL), and filtered through a CELITE^{®} bed. The filter cake was washed with EtOAc (400 mL). The layers of the filtrate were seperated. The aqueous layer was further extracted with EtOAc (2×200 mL). The combined orgnic layers were washed with brine (2×200 mL) and dried over sodium sulfate, filtered, and concentrated under reduced pressure. The residue was purified by column chromatography over silica (5-15% EtOAc/petroleum ether) to afford the title compound. MS: 150.2 (M-1).

The following intermediates in TABLE 1 were purchased from commercial sources.

**TABLE 1**

| **Intermdiate No.** | **Structure** | **IUPAC Name** | **CAS No.** |
|---|---|---|---|
| **A08** | 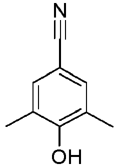 | 4-hydroxy-3,5-dimethylbenzonitrile | 4198-90-7 |
| **A09** | 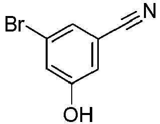 | 3-bromo-5-hydroxybenzonitrile | 770718-92-8 |
| **A10** | 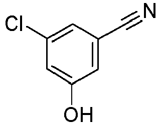 | 3-chloro-5-hydroxybenzonitrile | 473923-97-6 |

### Intermediate B Section

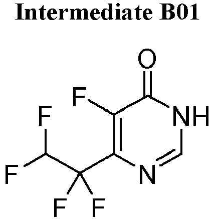

### 5-fluoro-6-(1,1,2,2-tetrafluoroethyl)-3,4-dihydropyrimidin-4-one

### Step 1: 2,2,3,3-tetrafluoropropanoic acid

To a solution of potassium heptaoxodichromium (1782 g, 6.06 mol) in sulfuric acid (2097 g, 21.38 mol) and water(2400 mL), was added of 2,2,3,3-tetrafluoropropan-1-ol (800 g, 6.06 mol) dropwise with stirring at 100 °C. The resulting solution was stirred at 100 °C for 5 h with refluxing, and then extracted with MTBE (4×2L). The organic layer was dried anhydrous sodium sulfate and concentrated under reduced pressure to afford the title compound and use as is for the next step.

### Step 2: 2,2,3,3-tetrafluoro-N-methoxy-N-methylpropanamide

To a solution of 2,2,3,3-tetrafluoropropanoic acid (crude product from previous step, 45% purity,1007g, 3.15 mol) in THF (4600 mL), was added sulfurooyl dichloride (787.2 g, 6.62 mol) dropwise at 0°C, followed by dropwise addition of DMF (753.4 g, 10.31 mol). After stirred at RT for 45 min, the reaction mixture was cooled to 0°C , and then treated with methoxy(methyl)amine hydrochloride (1222.5 g, 12.53 mol), and TEA (1913 g, 18.91 mol) dropwise. The resulting mixture was stirred at RT for 5 h, and then quenched with ice water (2 L), and extracted with MTBE (3 L). The organic layer was washed with sat. NaHCO₃ aq, brine, dried over anhydrous sodium sulfate, filtered and distillated to afford the title compound.

### Step 3: Ethyl 2,4,4,5,5-pentafluoro-3-oxopentanoate

To a solution of 2,2,3,3-tetrafluoro-N-methoxy-N-methylpropanamide (470 g, 2.49 mol) in THF(4700 mL), was added ethyl 2-fluoroacetate (316.2 g, 2.98 mol), 1M solution of LiHMDS in THF(3729 mL, 3.73 mol) at -78°C. The resulting mixture was stirred at -78°C for 2 h, and then quenched with sat. NH₄Cl aq (1 L). After the pH value of the solution was adjusted to 3 with HCl (1M), the reaction mixture was extracted with MTBE (3×2L). The organic layer was washed wahed with brine, dried over anhydrous sodium sulfate, filtered and concentrated under reduced pressure to afford the title compound, which was used for the next step directly.

### Step 4: 5-fluoro-6-(1,1,2,2-tetrafluoroethyl)-3,4-dihydropyrimidin-4-one

To a solution of ethyl 2,4,4,5,5-pentafluoro-3-oxopentanoate (533 g, 2.28 mol) in methanol (5330 mL), was added formamidine acetate (1185.1 g, 11.38 mol), and sodium methoxide (492 g, 9.11 mol). The resulting mixture was stirred at RT for 2 h, then quenched with ice water (1.5 L). After the pH value of the solution was adjusted to 3 with HCl(2M), the resulting mixture was extracted with MTBE (3× 1 L). The combined organic layers was dried over anhydrous sodium sulfate, filtered and concentrated under reduced pressure. The residue was purified by recrystallization from MTBE/PE(1:5) to afford the title compound.MS: 212.9 (M-1). ¹HNMR: (300MHz, CD₃OD): *δ* 8.10 (1H, s), 6.67~6.38(1H, m).

The following intermediates in TABLE **2** were prepared according to scheme C using the procedure outlined in the synthesis intermediate **B01** using a known fluorinated alkyl alcohol or its corresponding acid or methyl ester.

**TABLE 2**

| **Intermediate No.** | **Structure** | **IUPAC Name** | **MS** |
|---|---|---|---|
| **B02** | 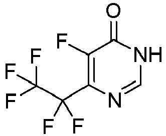 | 5-fluoro-6-(pentafluoroethyl)-3,4-dihydropyrimidin-4-one | 231.0 (M-1) |
| **B03** | 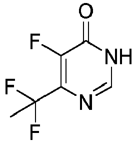 | 6-(1,1-difluoroethyl)-5-fluoropyrimidin-4(3H)-one | 177.1 (M-1) |
| **B04** | 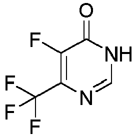 | 5-fluoro-6-(trifluoromethyl)pyrimidin-4(3H)-one | 183.0 (M+1) |

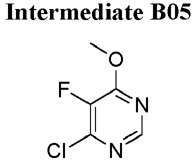

### 4-chloro-5-fluoro-6-methoxypyrimidine

To a solution of 4,6-dichloro-5-fluoropyrimidine (8.0 g, 47.9 mmol) in MeOH (80mL) was added sodium methoxide (3.05g, 56.5 mmol) at 0 °C. The resulting reaction mixture was stirred at 20 °C for 2 h, then quenched with water (20 ml) and then extracted with EA (3×20 mL). The organic layer was washed with brine, dried over anhydrous Na₂SO₄, filtrated and concentrated under reduced pressure to afford the title compound. MS: 163.1 (M+1). ¹H NMR (400 MHz, DMSO-*d₆*): δ 8.45(s, 1H), 4.03(s, 1H).

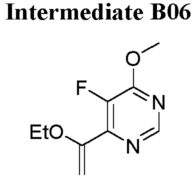

### 4-(1-ethoxyvinyl)-5-fluoro-6-methoxypyrimidine

To a solution of 4-chloro-5-fluoro-6-methoxypyrimidine (5.2g, 28.8 mmol), tributyl (1-ethoxyvinyl) stannane (12.48 g, 34.5 mmol) in DMF (50ml) was added Pd(Ph₃P)₄ (0.5g, 0.433 mmol). The resulting reaction mixture was stirred at 100 °C for 4 h under N₂ atmosphere. Upon completion, the reaction was diluted with water (30 mL), extracted with EtOAc (3 × 100 mL). The combined organic layers was treated with sat. KF aq. (80 mL) and stirred at RT for 0.5 h, then passed through a CELITE^{®} bed filter. The filtrate was washed with water, brine, and dried over anhydrous Na₂SO₄, filtrated and concentrated under reduced pressure. The residue purified by column chromatography on silica (10-90% EtOAc/PE) to to afford the title compound. ¹H NMR (400 MHz, CDCl₃): δ 8.49 (s, 1H), 5.08 (d, *J* = 2.8 Hz, 1H), 4.64 (d, *J* = 3.2 Hz, 1H), 4.06(s, 3H), 3.93 (m, 2H),1.42(m, 3H).

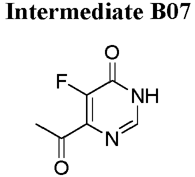

### 6-acetyl-5-fluoropyrimidin-4(3H)-one

To a solution of 4-(1-ethoxyvinyl)-5-fluoro-6-methoxypyrimidine (4.5 g, 20.43 mmol) in THF (30 ml) was added 6M HCl aq. (20 mL, 120 mmol) dropwise at 0 °C. The resulting reaction solution was stirred at 20 °C for 18 h, and then concentrated under reduced pressure while keeping the temperature of the bath at 40 °C to to afford the title compound. ¹H NMR (400 MHz, d₆-DMSO): δ 8.15(s, 1H), 2.50(s, 3H).

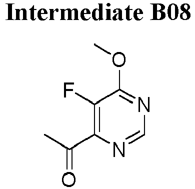

### 1-(5-fluoro-6-methoxypyrimidin-4-yl)ethanone

To a stirred solution of 4-(1-ethoxyvinyl)-5-fluoro-6-methoxypyrimidine, **B06** (12g, 60.5 mmol) in ACN (100 mL) at 0°C, was added 4M solution of hydrochloric acid in 1,4-dioxane (30 mL, 120 mmol) dropwise. The resulting solution was stirred at room temperature for 1 h, and then carefully quenched with ice cold water (200mL), and extracted with EtOAc. The combined organic layers were dried over anhydrous sodium sulfate,filtered and concentrated under reduced pressure. The residue was purified with column chromatography on silica (0-20% EtOAc/petroleum ether) to afford the title compound. MS: 171.4 (M+1).

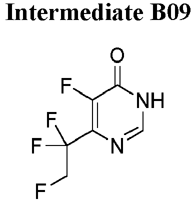

### 5-fluoro-6-(1,1,2-trifluoroethyl)pyrimidin-4(3H)-one

### Step 1: 2-fluoro-1-(5-fluoro-6-methoxypyrimidin-4-yl)ethanone

A solution of 4-(1-ethoxyvinyl)-5-fluoro-6-methoxypyrimidine, **B06** (0.2 g, 0.86 mmol) in ACN (3 mL) was added dropwise to a suspension of 1-chloromethyl-4-fluoro-1,4-diazoniabicyclo[2.2.2]octane bis(tetrafluoroborate) (0.456 g, 1.29 mmol) in ACN (2 mL) and water (1 mL) over 4 h, maintaining the temperature below 15 °C. The reaction mixture was then quenched with sat. NaHCO₃ aq. (10 mL) and left to stir for 10 min. After finished, the mixture was extracted with EA (3 × 10 mL). The combined organic layers was washed with brine, dried over MgSO4, filtered and concentrated under reduced pressure to afford the title compound. MS: 189.1 (M+1).

### Step 2: 5-fluoro-4-methoxy-6-(1,1,2-trifluoroethyl)pyrimidine

To a solution of 2-fluoro-1-(5-fluoro-6-methoxypyrimidin-4-yl)ethanone (0.15 g, 0.60 mmol) in DCM (2 mL) was added diethylaminosulfur trifluoride (0.578 g, 3.59 mmol). The resulting reaction mixture was stirred at 20 °C for 4 h, and then diluated with water, and extracted with EtOAc (3×50 mL). The combined organic layers was concentrated under reduced pressure. The residue was purified by prepative TLC (20% EtOAc/P.E.) to afford the title compound. ¹H NMR (400MHz, CDCl₃) δ 8.56 (s, 1H), 4.99 (t, J=12.0 Hz, 1H), 4.88 (t, J=12.0 Hz, 1H), 4.12 (s, 3H).

### Step 3: 5-fluoro-6-(1,1,2-trifluoroethyl)pyrimidin-4(3H)-one

To a mixture of 5-fluoro-4-methoxy-6-(1,1,2-trifluoroethyl)pyrimidine (0.05 g, 0.238 mmol) and KI (0.119 g, 0.714 mmol) in ACN (15 mL), was added chlorotrimethylsilane (0.078 g, 0.714 mmol) at RT. The resulting mixture was stirred at 80 °C for 4 h, and then diluted with EtOAc, washed with aq. Na₂S₂O₃ and brine, dried over anhydrous Na₂SO₄, filtered and concentrated under reduced pressure to afford the title compound. MS: 197.1 (M+1).

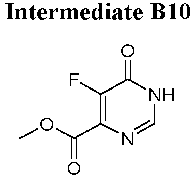

### 5-fluoro-6-methoxypyrimidine-4-carboxylate

Into a 20-L sealed tube purged with N2, was placed a solution of 4-chloro-5-fluoro-6-methoxypyrimidine, **B05** (410 g, 2.52 mol) in MeOH (8.2 L), triethylamine (1.247 kg, 12.32 mol), PdCl₂(dppf) (201 g, 246.32 mmol). Then set up for carbonylation in a bomb with CO at 2 bar and heated at 50 °C for 16 h. The solids were filtered out. The filtrate was concentrated under reduced pressure to remove MeOH, and then diluted with EtOAc (4 L). The solids were filtered out. The filtrate was again concentrated under reduced pressure. The residue was purified with column chromatography on silica (10% EtOAc/hexanes) and then re-crystallized (propan-2-ol:H₂O=2:7) to afford the title compound MS: 187 (M+1). ¹H NMR (400MHz, CDCl₃): δ 5.587 (s, 1H), 4.109-4.075 (s, 3H), 4.003-3.935 (s, 3H).

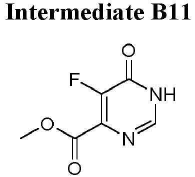

### Methyl 5-fluoro-6-hydroxypyrimidine-4-carboxylate

Trimethylsilyl chloride (25.7 ml, 201 mmol) was slowly added to a stirring slurry of methyl 5-fluoro-6-methoxypyrimidine-4-carboxylate, **B09** (25 g, 134 mmol) and potassium iodide (33.4 g, 201 mmol) in ACN (300 mL) at 25 °C. The resulting mixture was stirred at 25 °C for 2 hr. LCMS indicated reaction was complete. The reaction was quenched with methanol (100 mL) and concentrated to afford the title compound, which was used in the next step without further purification. MS: 173.0 (M+1). ¹H NMR (400 MHz, DMSO-d₆) 8.12 (s, 1H), 2.59 (s, 3H).

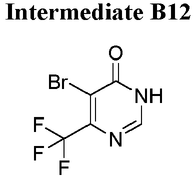

### 5-bromo-6-(trifluoromethyl)pyrimidin-4(3H)-one

This intermediate was made through bromination of 6-(trifluoromethyl)pyrimidin-4(3H)-one using procedures reported in US20140100231(A1).

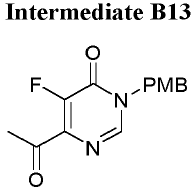

### 6-acetyl-5-fluoro-3-(4-methoxybenzyl)pyrimidin-4(3H)-one

To a solution of 6-acetyl-5-fluoropyrimidin-4(3H)-one, **B07** (1.0 g, 5.44 mmol) in DMF (10 mL) was added K₂CO₃ (2.257 g, 16.33 mmol). The resulting reaction mixture was stirred at 20 °C for 10 min before 1-(chloromethyl)-4-methoxybenzene (1.023 g, 6.53 mmol) was added. The reaction mixture was stirred at 20 °C for another 6 h, and then diluted with water (50 mL) and extracted with EtOAc (3×50 mL). The combined organic layers was washed with water, brine, dried over anhydrous Na₂SO₄, filtrated and concentrated under reduced pressure. The residue was purified by column chromatography on silica (50% EtOAc/PE) to afford the title compound. MS: 277.1 (M+1). ¹H NMR (400 MHz, CDCl₃): δ 8.01 (s, 1H), 7.29 (m, 2H), 6.89 (d, *J*=8.8, 2H), 5.09 (s, 2H), 3.81 (s, 2H), 2.58 (d, *J* =8.8, 3H).

The following intermediates in TABLE **3** were prepared under similar conditions as the above synthetic procedure outlined in the synthesis intermediate **B13** using a corresponding intermediate **B**.

**TABLE 3**

| **Intermediate** | **Starting Material B** | **Structure** | **IUPAC Name** | **MS (M+1)** |
|---|---|---|---|---|
| **B14** | **B03** | 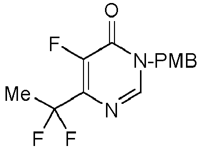 | 6-(1,1-difluoroethyl)-5-fluoro-3-(4-methoxybenzyl)pyrimi din-4(3*H*)-one | 299.0 |
| **B15** | **B11** | 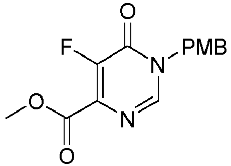 | Methyl 5-fluoro-1-(4-methoxybenzyl)-6-oxo-1,6-dihydropyrimidine-4-carboxylate | 293.0 |
| **B16** | **B01** | 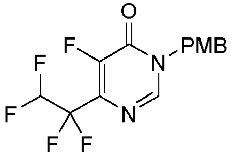 | 5-fluoro-3-(4-methoxybenzyl)-6-(1,1,2,2-tetrafluoroethyl)pyrimi din-4(3H)-one | 335.1 |
| **B17** | **B02** | 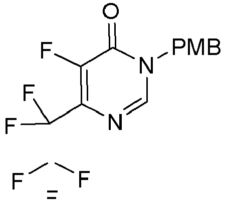 | 5-fluoro-3-(4-methoxybenzyl)-6-(perfluoroethyl)pyrimid in-4(3H)-one | 353.1 |
| **B18** | **B04** | 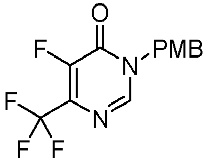 | 5-fluoro-3-(4-methoxybenzyl)-6-(trifluoromethyl)pyrimi din-4(3H)-one | 325.1 (M+23) |

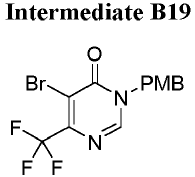

### 5-bromo-3-(4-methoxybenzyl)-6-(trifluoromethyl)pyrimidin-4(3H)-one

This intermediate was made using procedures reported in US20140100231(A1).

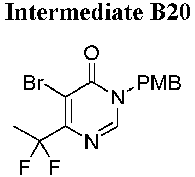

### 5-bromo-6-(1,1-difluoroethyl)-3-(4-methoxybenzyl)pyrimidin-4(3H)-one

This intermediate was made using procedures reported in US20140100231(A1).

### Intermediate C Section

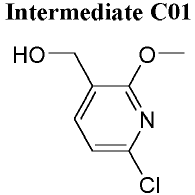

### (6-chloro-2-methoxypyridin-3-yl)methanol

### Step 1: 6-chloro-2-methoxynicotinaldehyde

To a stirred mixture of t-BuLi (23.94 mL, 38.3 mmol) in THF (100 mL) at -78 °C, was added 2-chloro-6-methoxypyridine (5g, 34.8 mmol). The resulting mixture was stirred at -78 °C for 1 hour, and then DMF (3.51 mL, 45.3 mmol) was added and the reaction mixture was stirred at - 78 °C for 1.5 h, and then quenched with acetic acid (3.99 mL, 69.7 mmol) at -78 °C. Next the mixture was warmed to 20 °C over 30 minutes. The mixture was neutralized with saturated aqueous NaHCO₃ solution (200 mL), and extracted with EtOAc (3×50 mL). The combined organic fractions were washed with brine (3× 50 mL), dried over anhydrous Na₂SO₄, filtered and concentrated under reduced pressure. The residue was purified by column chromatography on silica (1-2% EtOAc/isohexane) to afford the title compound.

### Step 2: (6-chloro-2-methoxypyridin-3-yl)methanol

To a suspension of 6-chloro-2-methoxynicotinaldehyde (1 g, 5.83 mmol) in MeOH (12 mL) at 0 °C, was added sodium borohydride (0.140 g, 3.70 mmol) portionwise over a period of 20 minutes. The resulting mixture was allowed to stir at 0 °C for 40 minutes, then quenched with water and extracted with DCM (3 × 5 mL). The combined organic layers were washed with brine, dried over anhydrous MgSO4, filtered and concentrated under reduced pressure to afford the title compound, which was used in the next step as is. MS: 174.1 (M+1).

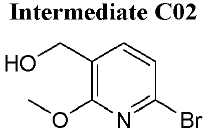

### (6-bromo-2-methoxypyridin-3-yl)methanol

### Step 1: 6-bromo-2-methoxynicotinic acid

To a stirred solution of 2,2,6,6-tetramethylpiperidine (3.76 g, 26.6 mmol) in THF (50 mL) at -78 °C, was added 2.5 M solution of n-BuLi in THE (10.64 mL, 26.6 mmol) dropwise. The resutling mixture was stirred at the same temperature for 60 minutes before a solution of 2-bromo-6-methoxypyridine (5g, 26.6 mmol) in THF was added dropwise. The reaction mixture was stirred at the same temperature for 60 minutess., and treated with crushed dry ice powder was added and stirred for 3 hours at -78 °C, and then allowed to warm up to room temperature slowly and diluted with water. And then the pH of the mixture was adjusted to 3-4 using conc. HCl. The mixture was was extracted with EtOAc (2×250 mL). The combined organic layers were washed with brine, dried over sodium sulphate, filtered and concentrated under reduced pressure. The residue was purified by column chromatography on silica (50% EtOAc/petroleum ether) to afford the title compound. MS: 230.2 and 232.0 (M-1).

### Step 2: (6-bromo-2-methoxypyridin-3-yl)methanol

To a stirred solution of 6-bromo-2-methoxynicotinic acid (2.3g, 9.91 mmol) in THF (20 mL), was added 1M solution of LAH in THF (19.82 mL, 19.82 mmol) dropwise at -20 °C. The resulting mixture was stirred at room temperature for 60 minutes, and then quenched with water (0.5 mL), 10% NaOH aquous solution and water. The resulting mixture was stirred for 30 minutes and filterrred through a CELITE^{®} bed. The filter cake was washed with EtOAc. The filtrate was concentrated under reduced pressure. The residue was purified by column chromatograpy on silica (30% EtOAc/petroleum ether) first, then using reverse phase HPLC (ACN/water with 0.1% TFA modifier) to afford the title compound. MS: 217.4 and 219.4 (M+1).

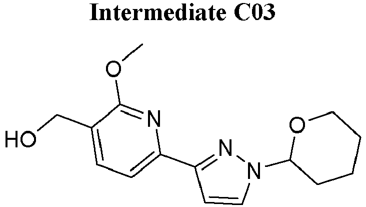

### (2-methoxy-6-(1-(tetrahydro-2H-pyran-2-yl)-1H-pyrazol-3-yl)pyridin-3-yl)methanol

A mixture of 1-(tetrahydro-2h-pyran-2-yl)-5-(4,4,5,5-tetramethyl-1,3,2-dioxaborolan-2-yl)-1h-pyrazole (113 mg, 0.406 mmol), (6-chloro-2-methoxypyridin-3-yl)methanol, **C01** (47 mg, 0.271 mmol), and potassium phosphate tribasic (172 mg, 0.812 mmol in 1,4-dioxane (2 mL) and water (1.0 mL) was purged with nitrogen for 5 minutes and then added [1,1'-bis(diphenyl phosphino)ferrocene]dichloropalladium(II) (19.81 mg, 0.027 mmol). The resulting mixture was stirred at 80 °C for 15 h, and then passed through filter. The filtrate was concentrated under reduced pressure. The residue was putified by column chromatography on silica (0-100% EtOAc/hexanes) to afford the title compound. MS: 290.1 (M+1).

The following intermediates in TABLE **4** were prepared using similar procedure outlined in the synthesis intermediate **C03** using a corresponding commercially available boronic acid or boronate.

**TABLE 4**

| **Intermediate** | **Structure** | **IUPAC Name** | **MS (M+1)** |
|---|---|---|---|
| **C04** | 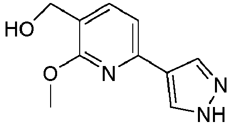 | (2-methoxy-6-(1H-pyrazol-4-yl)pyridin-3 -yl)methanol | 206.2 |
| **C05** | 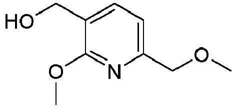 | (2-methoxy-6-(methoxymethyl)pyridin-3-yl)methanol | 184 |
| **C06** | 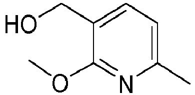 | (2-methoxy-6-methylpyridin-3-yl)methanol | 154.4 |
| **C07** | 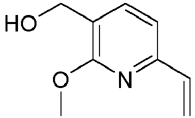 | (2-methoxy-6-vinylpyridin-3-yl)methanol | 166.0 |

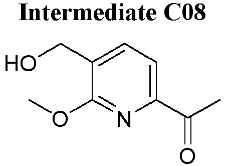

### 1-(5-(chloromethyl)-6-methoxypyridin-2-yl)ethanone

### Step 1: (6-(1-ethoxyvinyl)-2-methoxypyridin-3-yl)methanol and 1-(5-(hydroxymethyl)-6-methoxypyridin-2-yl)ethanone

To a solution of (6-chloro-2-methoxypyridin-3-yl)methanol, **C01** (1g, 5.76 mmol), and tributyl(1-ethoxyvinyl)stannane (4.16 g, 11.52 mmol) in DMF (5 mL) was added Pd(Ph₃P)₄ (0.200 g, 0.173 mmol). The resulting mixture was stirred at 100 °C for 4 h under N₂ atmosphere, and then cooled to room temperature and diluted with water (20 mL), and extracted with EtOAc (3×30 mL). The combined organic layers were treated with saturated KF aqueous solution (45 mL) and 4M HCl aqueous solution, stirred at 20 °C for 0.5 h, then washed with brine (2×75 mL), dried over anhydrous Na₂SO₄, filtrated and concentrated under reduced pressure. The residue was purified by column chromatography on silica (1-25% EtOAc/petroleum ether) to afford the title compound. MS: 182.1 (M+1).

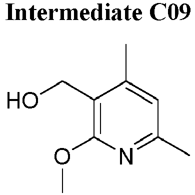

### (4-chloro-6-methoxy-2-methylpyrimidin-5-yl)methanol

### Step 1: methyl 2-chloro-4,6-dimethylnicotinate

To 2-chloro-4,6-dimethylnicotinic acid (2 g, 10.78 mmol) was added slowly sulfurous dichloride (20 mL, 275 mmol). The resulting mixture was stirred at 80°C for 2 h, and then concentrated under reduced pressure. The residue was treated with MeOH (20 mL) at 0 °C, and then the reaction mixture was stirred at 20°C for 16 h, and concentrated under reduced pressure to afford the title compound, which was used in the next step without further purification. MS: 200.1 (M+).

### Step 2: methyl 2-methoxy-4,6-dimethylnicotinate

To a solution of methyl 2-chloro-4,6-dimethylnicotinate (2.206 g, 11.05 mmol) in MeOH (20 mL) was added sodium methanolate (3.58 g, 66.3 mmol) at 0 °C. The reulting mixture was stirred at 60 °C for 24 h, and then quenched with water (20 mL) and then extracted with DCM (3×20 mL). The combined organic layers were washed with brine (3×10 mL), dried over anhdydrous Na₂SO₄, filtrated and concentrated under reduced pressure to afford the title compound, which was used in next step without further purification. MS: 196.1 (M+1).

### Step 3: 2-methoxy-4,6-dimethylpyridin-3-yl)methanol

To a solution of methyl 2-methoxy-4,6-dimethylnicotinate (500 mg, 2.56 mmol) in THF (5 ml) was added LAH (97 mg, 2.56 mmol) at 0 °C. The resulting mixture was stirred at 20°C for 1 hour, and then quenched with saturated ammonium chloride solution and extracted with DCM (3×10 mL). The combined organic layers were washed with brine (20 mL), dried over anhydrous Na₂SO₄, filtered and concentrated under reduced pressure. The residue was purified by preparative TLC plate (25% EtOAc/petroleum ether) to give the title compound. MS: 168.3 (M+1).

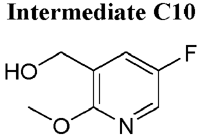

### (5-fluoro-2-methoxypyridin-3-yl)methyl methanesulfonate

### Step 1: 5-fluoro-2-methoxynicotinic acid

A mixture of sodium methoxide (5.70 g, 106 mmol) and methyl 2-chloro-5-fluoronicotinate (2 g, 10.55 mmol) in THF (15 mL) was stirred at 100 °C for 48 h, and then diluted with H₂O (30 mL) and acidified with HCl (1 M) to pH=3-4, and then extracted with EtOAc (3×20 mL). The combined organic layers were dried over anhydrous Na₂SO₄, filtered and concentrated under reduced pressure to give the title compound, which was used directly in the next step without further purification. MS: 170 (M-1).

### Step 2: (5-fluoro-2-methoxypyridin-3-yl)methanol

To a solution of 5-fluoro-2-methoxynicotinic acid (800 mg, 4.67 mmol) in THF (10 ml) was added LAH (266 mg, 7.01 mmol). The resulting mixture was stirred at 25 °C for 5 minutes before it was quenched with water (0.27 mL), and aqueous NaOH solution (0.27 mL). The mixture was filtered. The filtrate was extracted with EtOAc (3×30 mL), dried over anhydrous Na₂SO₄, filtered and concentrated under reduced pressure to give the title compound, which was used for the next step without purification. MS: 158 (M+1).

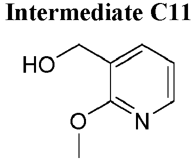

### (2-methoxypyridin-3-yl)methanol

### Step 1: methyl 2-methoxynicotinate

To a stirred solution of 2-methoxynicotinic acid (1 g, 6.53 mmol) in methanol (10 mL), was added H₂SO₄ (0.696 mL, 13.06 mmol) at 0 °C. The resulting mixture was stirred at 65°C for 6 h, and then concentrated under reduced pressure to get the title compound. MS: 168.4 (M+1).

### Step 2: (2-methoxypyridin-3-yl)methanol

To a stirred solution of methyl 2-methoxynicotinate (500 mg, 2.99 mmol) in THF (6 mL), was added 1M solution of LAH in THF (5.98 mL, 5.98 mmol) at 0°C slowly. The resulting mixture was allowed to warm up to room temperature, and stirred for 6 h, and then cooled and quenched with saturated Na₂SO₄ aqueous solution and EtOAc. The reaction mixture was filtered through CELITE^{®} bed. The filter cake was washed with excess of EtOAc. The filterate was seperated. The organic layer was concentrated under reduced pressure to afford the title compound. MS: 140.4 (M+1).

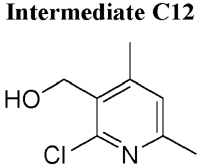

### (2-chloro-4,6-dimethylpyridin-3-yl)methanol

To a solution of 2-chloro-4,6-dimethylnicotinic acid (300 mg, 1.616 mmol) was dissolved in THF (3 mL), was added 1M LAB solution in THF (1.616 mL, 1.616 mmol) dropwise over a period of 10 minutes at 0 °C. The resulting mixture was allowed to stir at 0 °C for 2 h, and then allowed to warm up to ambient temperature and stirred over the weekend. LCMS showed complete conversion to desired product. The reaction mixture was diluted with water (~1 mL) very slowly, and evaporated off organic solvent under reduced pressure, and then partitioned between EtOAc (10 mL) and water (5 mL). The aqueous layer was further extract with EtOAc (2×10 mL). The combined organic layers were dried over anhydrous MgSO₄, filtered and concentrated under reduced pressure. The residue was purified with column chromatography on silica (0-100% EtOAc/hexanes) to afford the title compound. MS: 172.0 (M+1).

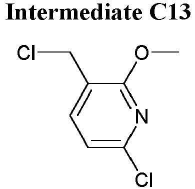

### 6-chloro-3-(chloromethyl)-2-methoxypyridine

A solution of (6-chloro-2-methoxypyridin-3-yl)methanol, **C01** (500 mg, 2.88 mmol) in DCM (14 mL) was treated with triethylamine (0.803 mL, 5.76 mmol) and then Mesyl-Cl (0.247 mL, 3.17 mmol) dropwise. The resulting reaction mixture was allowed to stir at 23 °C overnight, and then partitioned between DCM and saturated aqueous NaHCO₃ solution. The organic layers were separated and dried over anhydrous Na₂SO₄, filtered and concentrated under reduced pressure. The residue was purified with column chromatography on silica (0-20% EtOAc/Hexanes) to afford the title compound. MS: 192.0 (M+1). ¹H NMR (600 MHz, Chloroform-d) δ 7.58 (d, J = 7.7 Hz, 1H), 6.90 (d, J = 7.7 Hz, 1H), 4.53 (s, 2H), 3.99 (s, 3H).

The following intermediates in TABLE 5 were prepared using similar procedure outlined in the synthesis intermediate **C12** with appropriate (6-methoxypyrimidin-5-yl)methanol intermediate **C** as the starting materials described in Table **C12.**

**TABLE 5**

| **Intermediate** | **Starting Material C** | **Structure** | **IUPAC Name** | **MS (M+1)** |
|---|---|---|---|---|
| **C14** | **C03** | 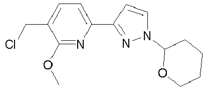 | 3-(chloromethyl)-2-methoxy-6-(1-(tetrahydro-2H-pyran-2-yl)-1H-pyrazol-3-yl)pyridine | 308.1 |
| **C15** | **C05** | 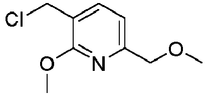 | 3-(chloromethyl)-2-methoxy-6-(methoxymethyl)pyridine | 202.0 |
| **C16** | **C09** | 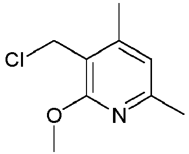 | 3-(chloromethyl)-2-methoxy-4,6-dimethylpyridine | 186.5 |
| **C17** | **C06** | 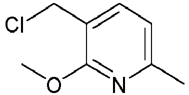 | 3-(chloromethyl)-2-methoxy-6-methylpyridine | 172.1 |
| **C18** | **C11** | 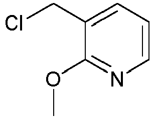 | 3-(chloromethyl)-2-methoxypyridine | 158.2 |
| **C19** | **C07** | 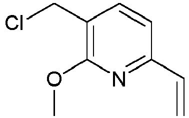 | 3-(chloromethyl)-2-methoxy-6-vinylpyridine | 184.1 |
| **C20** | **C10** | 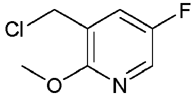 | 3-(chloromethyl)-5-fluoro-2-methoxypyridine | 175.9 |
| **C21** | **C08** | 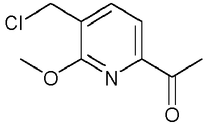 | 2,4-dichloro-5-(chloromethyl)-6-methoxypyrimidine | 200.1 |
| **C23** | **C12** | 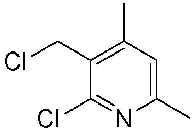 | 2-chloro-3-(chloromethyl)-4,6-dimethylpyridine | 192.1 |

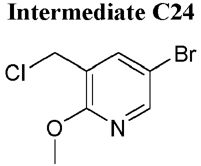

### 5-bromo-3-(chloromethyl)-2-methoxypyridine

Intermediate **C24** was synthesized using similar procedure as intermediate **C12** by replacing intermediate **C01** with commercial available (5-bromo-2-methoxypyridin-3-yl)methanol.

### Intermediate AB Section

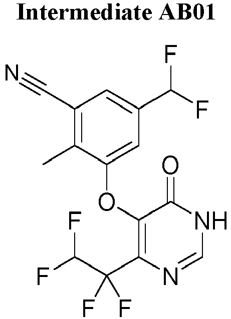

### 5-(difluoromethyl)-2-methyl-3 -((6-oxo-4-(1,1,2,2-tetrafluoroethyl)-1,6-dihydropyrimidin-5-yl)oxy)benzonitrile

### Step 1: 5-(difluoromethyl)-3-((1-(4-methoxybenzyl)-6-oxo-4-(1,1,2,2-tetrafluoroethyl)-1,6-dihydropyrimidin-5-yl)oxy)-2-methylbenzonitrile

To a solution of 5-fluoro-3-(4-methoxybenzyl)-6-(1,1,2,2-tetrafluoroethyl)pyrimidin-4(3H)-one, B16 (7 g, 20.94 mmol) in NMP (46.5 mL) was added 5-(difluoromethyl)-3-hydroxy-2-methylbenzonitrile, **A02** (4.22 g, 23.04 mmol) and K₂CO₃ (8.68 g, 62.8 mmol). The resulting mixture was stirred at 80 °C overnight. LCMS showed complete conversion. The reaction mixture was cooled to room temperature, then diluted with water. The precipitate was collected on top of a filter and washed with water (3×), then air-dried to give the title compound. MS: 498.1 (M+1).

### Step 2: 5-(difluoromethyl)-2-methyl-3-((6-oxo-4-(1,1,2,2-tetrafluoroethyl)-1,6-dihydropyrimidin-5-yl)oxy)benzonitrile

A solution of 5-(difluoromethyl)-3-((1-(4-methoxybenzyl)-6-oxo-4-(1,1,2,2-tetrafluoroethyl)-1,6-dihydropyrimidin-5-yl)oxy)-2-methylbenzonitrile (9.89 g, 19.88 mmol) in TFA (66.3 mL) and TFAA (33.1 mL) was stirred at 50 °C overnight. LCMS showed complete conversion. The reaction mixture was concentrated under reduced pressure. The residue was trituated with hexanes, and the solid was collected on top of a filter, washed with hexanes, then air-dried to afford the title compound, which was used directly in following step with further purification. MS: 378.3 (M+1).

The following intermediates in TABLE 6 were prepared in an analogous manner of intermediate **AB01** that described above: SN_{Ar} reaction using a corresponding intermediate **A** and intermediate **B,** then deprotection to remove PMB group using TFA/TFAA.

**TABLE 6**

| **Intermediate (A No., B No.)** | **Structure** | **IUPAC Name** | **MS (M+1)** |
|---|---|---|---|
| **AB02 (A02, B18)** | 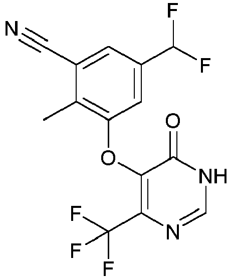 | 5-(difluoromethyl)-2-methyl-3-((6-oxo-4-(trifluoromethyl)-1,6-dihydropyrimidin-5-yl)oxy)benzonitrile | 346.1 |
| **AB03 (A02, B17)** | 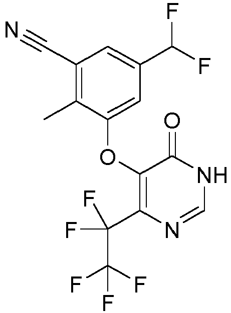 | 5-(difluoromethyl)-2-methyl-3-((6-oxo-4-(perfluoroethyl)-1,6-dihydropyrimidin-5-yl)oxy)benzonitrile | 396.1 |
| **AB04 (A06, B16)** | 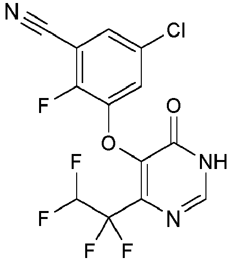 | 5-chloro-2-fluoro-3-((6-oxo-4-(1,1,2,2-tetrafluoroethyl)-1,6-dihydropyrimidin-5-yl)oxy)benzonitrile | 366.1 |
| **AB05 (A08, B16)** | 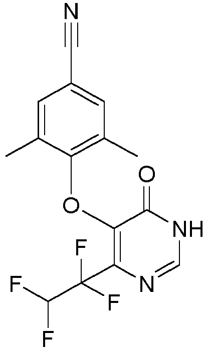 | 3,5-dimethyl-4-((6-oxo-4-(1,1,2,2-tetrafluoroethyl)-1,6-dihydropyrimidin-5-yl)oxy)benzonitrile | 341.9 |
| **AB06 (A05, B17)** | 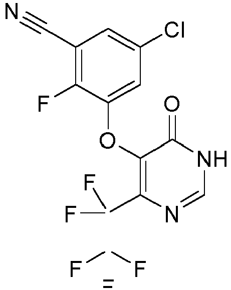 | 5-chloro-2-fluoro-3-((6-oxo-4-(perfluoroethyl)-1,6-dihydropyrimidin-5-yl)oxy)benzonitrile | 384.0 |
| **AB07 (A09, B16)** | 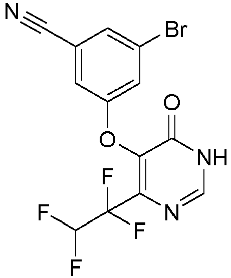 | 3-bromo-5-((6-oxo-4-(1,1,2,2-tetrafluoroethyl)-1,6-dihydropyrimidin-5-yl)oxy)benzonitrile | 392.0 |
| **AB08 (A10, B16)** | 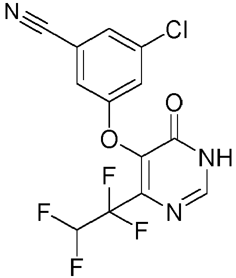 | 3-chloro-5-((6-oxo-4-(1,1,2,2-tetrafluoroethyl)-1,6-dihydropyrimidin-5-yl)oxy)benzonitrile | 346.2 (M-1) |
| **AB09 (A06, B16)** | 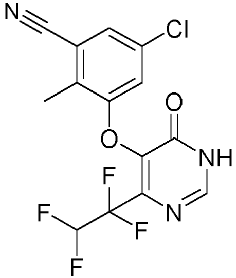 | 5-chloro-2-methyl-3-((6-oxo-4-(1,1,2,2-tetrafluoroethyl)-1,6-dihydropyrimidin-5-yl)oxy)benzonitrile | 362.0 |
| **AB10 (A03, B16)** | 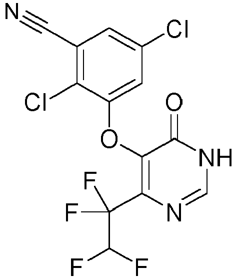 | 2,5-dichloro-3-((6-oxo-4-(1,1,2,2-tetrafluoroethyl)-1,6-dihydropyrimidin-5-yl)oxy)benzonitrile | 382.0 |
| **AB11 (A07, B16)** | 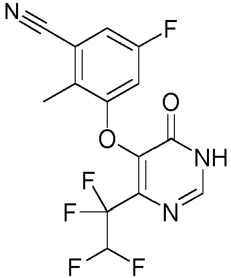 | 5-fluoro-2-methyl-3-((6-oxo-4-(1,1,2,2-tetrafluoroethyl)-1,6-dihydropyrimidin-5-yl)oxy)benzonitrile | 346.1 |

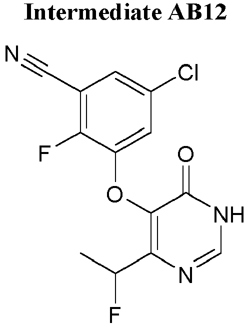

### 5-chloro-2-fluoro-3-((4-(1-fluoroethyl)-6-oxo-1,6-dihydropyrimidin-5-yl)oxy)benzonitrile

### Step 1: 6-acetyl-5-(3-bromo-5-chloro-2-fluorophenoxy)-3-(4-methoxybenzyl)pyrimidin-4(3H)-one

To a solution of 6-acetyl-5-fluoro-3-(4-methoxybenzyl)pyrimidin-4(3H)-one, **B13** (2 g, 7.24 mmol) in DMF (10 mL) was added 3-bromo-5-chloro-2-fluorophenol, **A04** (1.959 g, 8.69 mmol) and K₂CO₃ (2.001 g, 14.48 mmol). The resulting mixture was stirred at 60 °C for 4 h, and then poured into H₂O (100 mL), extracted with EtOAc (3×50 mL). The combined organic layers were dried over anhydrous Na₂SO₄, filtered and concentrated under reduced pressure. The residue was purified by column chromatography on silica (2-3.3% MeOH/DCM) to give the title compound.

### Step 2: 5-(3-bromo-5-chloro-2-fluorophenoxy)-6-(1-hydroxyethyl)-3-(4-methoxybenzyl)pyrimidin-4(3H)-one

To a solution of 6-acetyl-5-(3-bromo-5-chloro-2-fluorophenoxy)-3-(4-methoxybenzyl)pyrimidin-4(3H)-one (300 mg, 0.623 mmol) in THF (2 mL) was added 0.1 M solution of Zn(BH₄)₂ in THF (0.934 mL, 0.093 mmol) at -30°C and the mixture was stirred for 10 min. The reaction mixture was added water (1 drop) and then concentrated under reduced pressure to afford the title compound.

### Step 3: 5-(3-bromo-5-chloro-2-fluorophenoxy)-6-(1-fluoroethyl)-3-(4-methoxybenzyl)pyrimidin-4(3H)-one

To a solution of 5-(3-bromo-5-chloro-2-fluorophenoxy)-6-(1-hydroxyethyl)-3-(4-methoxybenzyl)pyrimidin-4(3H)-one (680 mg, 1.406 mmol) in DCM (10 mL) was added DAST (0.427 mL, 3.23 mmol) at 0 °C. The resulting reaction mixture was stirred at 0 °C for 1 h, and then diluted with water (5 mL), extracted with DCM (3 × 10 mL). The combined organic layers were washed with brine, dried over anhydrous Na₂SO₄, filtrated and concentrated under reduced pressure to give the title compound.

### Step 4: 5-chloro-2-fluoro-3-((4-(1-fluoroethyl)-1-(4-methoxybenzyl)-6-oxo-1,6-dihydropyrimidin-5-yl)oxy)benzonitrile

To a stirred solution of 5-(3-bromo-5-chloro-2-fluorophenoxy)-6-(1-fluoroethyl)-3-(4-methoxybenzyl)pyrimidin-4(3H)-one (340 mg, 0.700 mmol) in NMP (3 mL) was added cyanocopper (217 mg, 2.423 mmol) under N₂. The resulting mixture was stirred at 170 °C for 12 h, and then diluted with H₂O (30 mL) and EtOAc (30 mL). The mixture was filtered through CELITE^{®} funnel. The filtrate was extracted with EA (3×30 mL). The combined organic layers were washed with brine, dried over anhydrous Na₂SO₄, filtered and concentrated under reduced pressure. The residue was triturated with MeOH (20 mL) to afford the title compound.

### Step 5: 5-chloro-2-fluoro-3-((4-(1-fluoroethyl)-6-oxo-1,6-dihydropyrimidin-5-yl)oxy)benzonitrile

To a solution of 5-chloro-2-fluoro-3-((4-(1-fluoroethyl)-1-(4-methoxybenzyl)-6-oxo-1,6-dihydropyrimidin-5-yl)oxy)benzonitrile (0.217 g, 0.503 mmol) in TFA (1.5 mL) was added TFAA (0.75 mL). The resulting mixture was stirred at 100 °C for 6 h, and then diluted with DCM (10 mL), and concentrated under reduced pressure to afford the title compound.

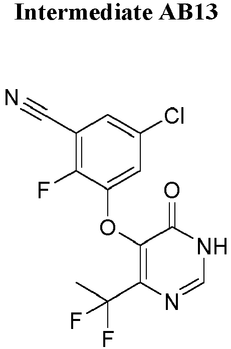

### 5-Chloro-3-((4-(1,1-difluoroethyl)-6-oxo-1,6-dihydropyrimidin-5-yl)oxy)-2-fluorobenzonitrile

### Step 1: Methyl 5-fluoro-1-(4-methoxybenzyl)-6-oxo-1,6-dihydropyrimidine-4-carboxylate

Potassium carbonate (16.11 g, 117 mmol) was added to a mixture of methyl 5-fluoro-1-(4-methoxybenzyl)-6-oxo-1,6-dihydropyrimidine-4-carboxylate, **B15** (21.29 g, 72.9 mmol) and 5-chloro-2-fluoro-3-hydroxybenzonitrile, **A05** (10 g, 58.3 mmol) in dimethylacetamide (350 mL) at 25 °C. The resulting mixture was stirred at 90 °C overnight. LCMS showed desired product and clean reaction. Water (500 mL) was added and a precipitate was formed. The mixture was filtered and the solid was washed with water (100 mL) and hexanes (200 mL) and dried overnight under high vacuum to provide the title compound. MS: 444 (M+1).

### Step 2: 5-(5-Chloro-3-cyano-2-fluorophenoxy)-1-(4-methoxybenzyl)-6-oxo-1,6-dihydropyrimidine-4-carboxylic acid

Potassium trimethylsilanolate (9.97 g, 78 mmol) was added to a stirring mixture of methyl 5-(5-chloro-3-cyano-2-fluorophenoxy)-1-(4-methoxybenzyl)-6-oxo-1,6-dihydropyrimidine-4-carboxylate (23 g, 51.8 mmol) in THF (345 mL). The resulting mixture was stirred at 25 °C for 1hour. LCMS showed only desired product. HCl (1N aq., 300 mL) was added slowly and a white solid was formed. The mixture was concentrated under reduced pressure to remove THF. The mixture was filtered and the white solid was washed with water and dried under vacuum to provide the title compound. MS: 430.0 (M+1).

### Step 3: 5-(5-Chloro-3-cyano-2-fluorophenoxy)-N-methoxy-1-(4-methoxybenzyl)-N-methyl-6-oxo-1,6-dihydropyrimidine-4-carboxamide

To a flask containing 5-(5-chloro-3-cyano-2-fluorophenoxy)-1-(4-methoxybenzyl)-6-oxo-1,6-dihydropyrimidine-4-carboxylic acid (20 g, 46.5 mmol) and *N*,*O*-dimethylhydroxylamine hydrochloride (4.99 g, 51.2 mmol) in DMF (200 mL), was added triethylamine (25.9 ml, 186 mmol) followed by EDC (9.81 g, 51.2 mmol) and HOBt (7.84 g, 51.2 mmol) at 25 °C. The resulting mixture was stirred at 25 °C overnight. LCMS indicated the completion of the reaction. Water (300 mL) was added and the resulting precipitate was filtered and washed with water, dried under vacuum to provide the title compound. MS: 473.1 (M+1).

### Step 4: 3-((4-Acetyl-1-(4-methoxybenzyl)-6-oxo-1,6-dihydropyrimidin-5-yl)oxy)-5-chloro-2-fluorobenzonitrile

Methylmagnesium bromide (3M in THF, 40 mL, 120 mmol) was added dropwise to a cooled (-78 °C) mixture of 5-(5-chloro-3-cyano-2-fluorophenoxy)-N-methoxy-1-(4-methoxybenzyl)-N-methyl-6-oxo-1,6-dihydropyrimidine-4-carboxamide (16.5 g, 34.9 mmol) in THF (350 mL). The resulting mixture was stirred at -78°C for 3 h. LCMs showed desired product but still starting material. The reaction mixture was then warmed up to 0 °C and stirred for 30 min. LCMS indicated completion of the reaction. The reaction was quenched with saturated aqueous NH₄Cl solution (500 mL) and diluted with EtOAc (200 mL). The organic layer was separated and the aqueous layer was extracted with EtOAc (2×200 mL). The combined organic layers were washed with brine (500 mL), dried over anhydrous MgSO₄, filtered and concentrated under reduced pressure. The residue was purified by column chromatography on silica (0-100% EtOAc/hexanes) to afford the title compound. MS: 428.0 (M+1).

### Step 5: 5-Chloro-3-((4-(1,1-difluoroethyl)-1-(4-methoxybenzyl)-6-oxo-1,6-dihydropyrimidin-5-yl)oxy)-2-fluorobenzonitrile

Deoxofluor^{®} (15.08 ml, 82 mmol) was added to a vial containing 3-((4-acetyl-1-(4-methoxybenzyl)-6-oxo-1,6-dihydropyrimidin-5-yl)oxy)-5-chloro-2-fluorobenzonitrile (7 g, 16.36 mmol)and dichloroethane (9.35 ml) at 25 °C. The resulting vial was sealed and stirred at 70 °C for 3 h. LCMS showed desired product. The reaction was cooled to room temperature and poured into a saturated NaHCO₃ solution very slowly and carefully. Then, the organic layer was separated and the aqueous layer was extracted with dichloromethane (2 × 100 mL). The combined organic layers were dried over MgSO4, filtered and concentrated. The residue was purified by column chromatography on silica (0-100% EtOAc/hexanes) to afford the title compound. MS: 450.0 (M+1).

### Step 6: 5-Chloro-3-((4-(1,1-difluoroethyl)-6-oxo-1,6-dihydropyrimidin-5-yl)oxy)-2-fluorobenzonitrile

A flask containing a mixture of 5-chloro-3-((4-(1,1-difluoroethyl)-1-(4-methoxybenzyl)-6-oxo-1,6-dihydropyrimidin-5-yl)oxy)-2-fluorobenzonitrile (7 g, 15.56 mmol) in trifluoroacetic acid (58.4 mL) and trifluoroacetic anhydride (19.45 mL) was stirred at 80 °C for 4 h. After cooling to room temperature, the mixture was concentrated under reduced pressure. The residue was dissolved in dichloromethane (50 mL) and hexanes (100 mL) were added slowly until the formation of a white solid. After stirring for 30 min, the white solid was collected on top of a filter, and dried under vacuum to provide the title compound. MS: 330.0 (M+1).

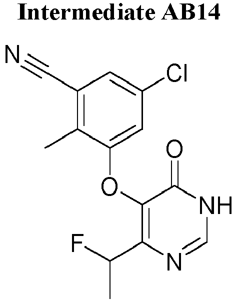

### 5-chloro-3-((4-(1-fluoroethyl)-6-oxo-1,6-dihydropyrimidin-5-yl)oxy)-2-methylbenzonitrile

### Step 1: 3-((4-acetyl-6-methoxypyrimidin-5-yl)oxy)-5-chloro-2-methylbenzonitrile

To the solution of 1-(5-fluoro-6-methoxypyrimidin-4-yl)ethanone, **B08** (2.8g, 16.46 mmol) in DMF (20 mL), was added potassium carbonate (6.82 g, 49.4 mmol) and 5-chloro-3-hydroxy-2-methylbenzonitrile, **A06** (3.31 g, 19.75 mmol) under nitrogen. The resulting mixture was heated at 40 °C for 2 h, and then diluted with water, and extracted with EtOAc. The combined organic layers were washed with brine, dried over anhydrous Na₂SO₄, filtered and concentrated under reduced pressure. The residue was purified by column chromatography on silica (20-60% EtOAc/petroleum ether) to afford the title compound. MS: 318.4 (M+1).

### Step 2: 5-chloro-3-((4-(1-hydroxyethyl)-6-methoxypyrimidin-5-yl)oxy)-2-methylbenzonitrile

To the stirred solution of 3-((4-acetyl-6-methoxypyrimidin-5-yl)oxy)-5-chloro-2-methylbenzonitrile (1.8g, 5.67 mmol) in methanol (50 mL), was added cerium(iii) chloride heptahydrate (3.17 g, 8.50 mmol) followed by slow addition of sodium borohydride (0.214 g, 5.67 mmol). The resulting suspention was allowed to stirred at 0 °C for 1 hour, and then quenched with ice water, and extracted with EtOAc. The combined organic layers were washed with brine solution dried over anhydrous Na₂SO₄, filtered and concentrated under reduced pressure. The residue was purified by column chromatography on silica (40-80% EtOAc/petroleum ether) to afford the title compound. MS: 320.0 (M+1).

### Step 3: 5-chloro-3-((4-(1-fluoroethyl)-6-methoxypyrimidin-5-yl)oxy)-2-methylbenzonitrile

To the stirred solution of 5-chloro-3-((4-(1-hydroxyethyl)-6-methoxypyrimidin-5-yl)oxy)-2-methylbenzonitrile (1.4g, 4.38 mmol) in DCM (5 mL), was added deoxofluor (1.615 mL, 8.76 mmol) at 0 °C. The resulting mixture was allowed to room temprature for 3 h, and then diluted with DCM and washed with saturated NaHCO₃ aqueous solution and brine. The organic layer was dried over anhydrous Na₂SO₄, filtered and concentrated under reduced pressure. The residue was purified by column chromatography on silica (10 40% EtOAc/petroleum ether) to afford the title compound. MS: 322.4 (M+1).

### Step 4: 5-chloro-3-((4-(1-fluoroethyl)-6-oxo-1,6-dihydropyrimidin-5-yl)oxy)-2-methylbenzonitrile

To the stirred solution of 5-chloro-3-((4-(l-fluoroethyl)-6-methoxypyrimidin-5-yl)oxy)-2-methylbenzonitrile (1.2g, 3.73 mmol) in ACN (20 mL) was added sodium iodide (1.118 g, 7.46 mmol) and TMS-Cl (0.953 ml, 7.46 mmol) under nitrogen. The resulting mixture was heated at 80 °c for 2 h, and then concentrated unde reduced pressure. The residue was purified by column chromatography on silica (40- 80 % EtOAc/petroleum ether) to afford the title compound. MS: 306.4 (M-1).

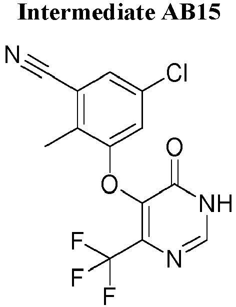

### 5-chloro-2-methyl-3-((6-oxo-4-(trifluoromethyl)-1,6-dihydropyrimidin-5-yl)oxy)benzonitrile

### Step 1: 5-chloro-3-((1-(4-methoxybenzyl)-6-oxo-4-(trifluoromethyl)-1,6-dihydropyrimidin-5-yl)oxy)-2-methylbenzonitrile

To a solution of 5-fluoro-3-(4-methoxybenzyl)-6-(trifluoromethyl)pyrimidin-4(3H)-one, **B18** (9.0g, 29.8 mmol), 5-chloro-3-hydroxy-2-methylbenzonitrile, **A06** (4.99 g, 29.8 mmol) in DMF (60 mL), was added potassium carbonate (8.23 g, 59.6 mmol). The reaction mixture was stirred at 100 °C for 16 h and then diluted with water (85 mL), and extracted with EtOAc (100 mL). The organic layer was washed with brine (40 mL), dried over anhydrous Na₂SO₄ and concentrated under reduced pressure. The residue was purified by column chromatography on silica (0-35% EtOAc/petroleum-ether) to afford the title compound. MS: 450.8 (M+1).

### Step 2: 5-chloro-2-methyl-3-((6-oxo-4-(trifluoromethyl)-1,6-dihydropyrimidin-5-yl)oxy)benzonitrile

To a solution of 5-chloro-3-((1-(4-methoxybenzyl)-6-oxo-4-(trifluoromethyl)-1,6-dihydropyrimidin-5-yl)oxy)-2-methylbenzonitrile (3 g, 6.67 mmol) in ACN (70 mL) and water (5 mL), was added ceric ammonium nitrate (18.28 g, 33.3 mmol). The reaction mixture was stirred at room temperature for 24 h, and then diluted with water (10 mL), and extracted with EtOAc (200 mL). The organic layer was washed with brine (70 mL), dried over anhydrous Na₂SO₄, filtered and concentrated under reduced pressure. The residue was purified by column chromatography on silica (0-35% EtOAc/petroleum ether) to afford the title compound. MS: 328.0 (M-1).

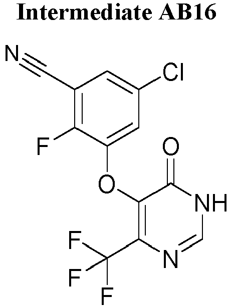

### 5-chloro-2-fluoro-3-((6-oxo-4-(trifluoromethyl)-1,6-dihydropyrimidin-5-yl)oxy)benzonitrile

### Step 1: 5-(3-bromo-5-chloro-2-fluorophenoxy)-3-(4-methoxybenzyl)-6-(trifluoromethyl)pyrimidin-4(3H)-one

To a solution of 5-fluoro-3-(4-methoxybenzyl)-6-(trifluoromethyl)pyrimidin-4(3H)-one, **B18** (1 g, 3.31 mmol) in DMF (10 mL), were added 3-bromo-5-chloro-2-fluorophenol, **A04** (0.821 g, 3.64 mmol) and K₂CO₃ (16.54 mg, 0.120mmol) at 25 °C. The reaction mixture was stirred at 80°C for 1 h, and then diluted with water (3 mL), and extracted with EtOAc (3×2 mL). The combined organic layers were washed with brine (3 mL), dried over anhydrous Na₂SO₄, filtered and concentrated under reduced pressure. The residue was by preparative-TLC plate (25% EtOAc/petroleum ether) to give the title compound. MS: 507.2 (M+1). ¹H NMR (400MHz, Chloroform-d) δ = 8.03 (d, J=4.4 Hz, 1H), 7.34 - 7.24 (m, 3H), 7.04 - 6.89 (m, 2H), 6.86 (dd, J=2.4, 6.6 Hz, 1H), 5.07 (s, 2H), 3.81 (s, 3H).

### Step 2: 5-chloro-2-fluoro-3-((1-(4-methoxybenzyl)-6-oxo-4-(trifluoromethyl)-1,6-dihydropyrimidin-5-yl)oxy)benzonitrile

A stirred solution of a mixture of 5-(3-bromo-5-chloro-2-fluorophenoxy)-3-(4-methoxybenzyl)-6-(trifluoromethyl)pyrimidin-4(3H)-one (300 mg, 0.591 mmol) and copper(I) cyanide (265 mg, 2.95 mmol) in NMP (3 mL) was stirred at 170 °C for 7 h, before diluted with water (30 mL), and then extracted with EtOAc (3×5 mL). The precipitation of solid washed by mixed solvent DCM:MeOH:MeCN=1:1:1 (5 mL). The combined organic layers were washed with brine (2×10 mL), dried over anhydrous Na₂SO₄, filtrated and concentrated under reduced pressure. The residue was purified by preparative-TLC plate (50% EtOAc/petroleum ether) to afford the title compound. ¹H NMR (400MHz, Chloroform-*d*) δ = 8.17 (s, 1H), 7.35 - 7.30 (m, 1H), 7.25 (d, J=8.7 Hz, 2H), 7.14 (dd, J=2.4, 7.0 Hz, 1H), 6.98 - 6.86 (m, 2H), 5.06 (s, 2H), 3.79 (s, 3H).

### Step 3: 5-chloro-2-fluoro-3-((6-oxo-4-(trifluoromethyl)-1,6-dihydropyrimidin-5-yl)oxy)benzonitrile

To a solution of 5-chloro-2-fluoro-3-((1-(4-methoxybenzyl)-6-oxo-4-(trifluoromethyl)-1,6-dihydropyrimidin-5-yl)oxy)benzonitrile (200 mg, 0.176 mmol) in TFA (2 mL), was added TFAA (1 mL). The resulting mixture was stirred at 110 °C for 2 h before quenched by adding saturated aqueous solution of NaHCO₃ (5 mL) and extracted with EtOAc (3×5 mL). The combined organic layers were washed with brine (10 mL), dried over anhydrous Na₂SO₄, filtered and concentrated under reduced pressure to give the title compound, which was used directly in next step. MS: 375.0 (M+ACN+1).

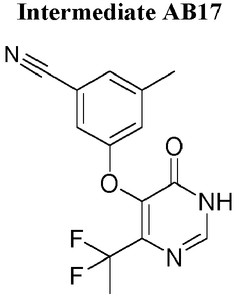

### 3-((4-(1,1-difluoroethyl)-6-oxo-1,6-dihydropyrimidin-5-yl)oxy)-5-methylbenzonitrile

### Step 1: 3-chloro-5-((4-(1,1-difluoroethyl)-1-(4-methoxybenzyl)-6-oxo-1,6-dihydropyrimidin-5-yl)oxy)benzonitrile

To a mixture of compound 5-bromo-6-(1,1-difluoroethyl)-3-(4-methoxybenzyl) pyrimidin-4(3H)-one, **B20** (20 g, 55.7 mmol), 3-chloro-5-hydroxybenzonitrile, **A10** (17.10 g, 111 mmol) in NMP (140 mL), was added K₂CO₃ (15.39 g, 111 mmol). The resulting mixture was stirred at 140 °C for 24 h. To the mixture was added 3-chloro-5-hydroxy benzonitrile (17.10 g, 111 mmol). The resulting mixture was stirred at 140 °C for another 16 h, then diluted with water, extracted with EtOAc. The organic layer was washed with water and brine, dried over anhydrous Na₂SO₄, filtered and concentrated under reduced pressure. The residue was purified by column chromatography on silica (6.25-50% EtOAc/petroleum ether) to afford the title compound. MS: 432.1 (M+1).

### Step 2: 3-((4-(1,1-difluoroethyl)-1-(4-methoxybenzyl)-6-oxo-1,6-dihydropyrimidin-5-yl)oxy)-5-methylbenzonitrile

A mixture of 3-chloro-5-((4-(1,1-difluoroethyl)-1-(4-methoxybenzyl)-6-oxo-1,6-dihydropyrimidin-5-yl)oxy)benzonitrile (1.0 g, 2.316 mmol), chloro[(di(1-adamantyl)-n-butylphosphine)-2-(2-aminobiphenyl)]palladium(II) (155 mg, 0.232 mmol), potassium methyltrifluoroborate (424 mg, 3.47 mmol) and Cs₂CO₃ (2264 mg, 6.95 mmol)) in water (5.79 mL) and 1,4-dioxane (17.4 mL) was purged with N₂, and then heated to 130 °C. After 30 minutes, the mixture was passed through a CELITE^{®} filter bed. The filtrate was diluted with EtOAc (10 mL) and washed with water. The organic layer was dried over anhydrous MgSO₄, filtered and concentrated under reduced pressure. The residue was purifed by column chromatography on silica (0-70% EtOAc/Hexanes) to afford the title compound. MS: 412.1 (M+1). ¹H NMR (600 MHz, Chloroform-d) δ 8.13 (s, 1H), 7.28 (d, J = 8.6 Hz, 2H), 7.17 (s, 1H), 7.01 (s, 1H), 6.89 (d, J = 8.6 Hz, 3H), 5.05 (s, 2H), 3.80 (s, 3H), 2.35 (s, 3H), 1.93 (t, J = 18.8 Hz, 3H).

### Step 3: 3-((4-(1,1-difluoroethyl)-6-oxo-1,6-dihydropyrimidin-5-yl)oxy)-5-methylbenzonitrile

To a solution of 3-((4-(1,1-difluoroethyl)-1-(4-methoxybenzyl)-6-oxo-1,6-dihydropyrimidin-5-yl)oxy)-5-methylbenzonitrile (841.5 mg, 2.045 mmol) in TFA (9297 µL), were added anisole (223uL, 2.045 mmol) and TFAA (930 µL). The resulting mixture was heated to 110 °C for 15 minutes, and concentrated under reduced pressure. The residue was taken up into DCM (20 mL) and washed with saturated aqueous NaHCO₃ solution (2×5 mL) and concentrated under reduced pressure. The residue was purifed by column chromatography on silica (0-10 % MeOH/DCM) to afford the title compound. MS: 292.1 (M+1). ¹H NMR (600 MHz, Chloroform-*d*) δ 8.08 (s, 1H), 7.20 (s, 1H), 7.02 (s, 1H), 6.93 (s, 1H), 2.38 (s, 3H), 1.96 (t, J = 18.8 Hz, 3H).

### Intermediate BC Section

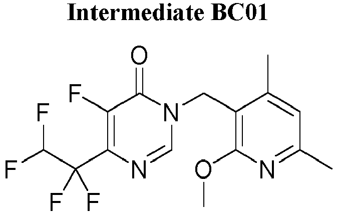

### 5-fluoro-3-((2-methoxy-4,6-dimethylpyridin-3-yl)methyl)-6-(1,1,2,2-tetrafluoroethyl)pyrimidin-4(3H)-one

To a mixture of 5-fluoro-6-(1,1,2,2-tetrafluoroethyl)pyrimidin-4(3H)-one, **B01** (48 mg, 0.224 mmol), lithium bromide (19.47 mg, 0.224 mmol) and K₂CO₃ (77 mg, 0.561 mmol) in DMF (2242 µL), was 3-(chloromethyl)-2-methoxy-4,6-dimethylpyridine, **C16** (41.6 mg, 0.224 mmol). The resulting mixture was heated to 50 °C overnight, and then filtered, and purified with reverse phase HPLC (ACN/water with 0.1% TFA as modifier) to isolate the title compound. MS: 363.9 (M+1). ¹H NMR (500 MHz, Chloroform-*d*) δ 8.18 (s, 1H), 6.65 (s, 1H), 5.08 (s, 2H), 3.94 (s, 3H), 2.46 (s, 3H), 2.40 (s, 3H).

The following intermediates in TABLE 7 were prepared under similar conditions as the above synthetic procedure outlined in the synthesis intermediate **BC01** using a corresponding intermediate **B** and intermediate **C.**

**TABLE 7**

| **Intermediate (B No., C No.)** | **Structure** | **IUPAC Name** | **MS (M+1)** |
|---|---|---|---|
| **BC02 (B04, C23)** | 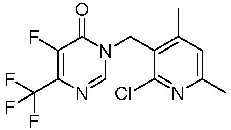 | 3-((2-chloro-4,6-dimethylpyridin-3-yl)methyl)-5-fluoro-6-(trifluoromethyl)pyrimidin-4(3H)-one | 336.0 |
| **BC03 (B01, C17)** | 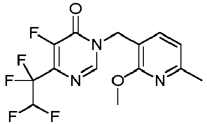 | 5-fluoro-3-((2-methoxy-6-methylpyridin-3-yl)methyl)-6-(1,1,2,2-tetrafluoroethyl)pyrimidin-4(3H)-one | 350.1 |

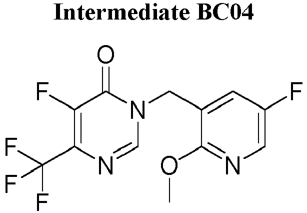

### 5-fluoro-3-((5-fluoro-2-methoxypyridin-3-yl)methyl)-6-(trifluoromethyl)pyrimidin-4(3H)-one

To a stirred solution of 5-fluoro-6-(trifluoromethyl)pyrimidin-4(3H)-one, **B04** (0.207 g, 1.139 mmol) and 3-(chloromethyl)-5-fluoro-2-methoxypyridine, **C20** (0.2 g, 1.139 mmol) in NMP (5.70 mL) at room temperature, was added potassium carbonate (0.315 g, 2.278 mmol). The resulting mixture was stirred at room temperature overnight, and diluted with water (3 mL) and EtOAc (3 mL). The organic layer was separated and concentrated under reduced pressure to afford the title compound, which was used in the next step without further purification. MS: 322.1 (M+1).

The following intermediates in TABLE **8** were prepared under similar conditions as the above synthetic procedure outlined in the synthesis intermediate **BC04** using a corresponding intermediate **B** and intermediate **C.**

**TABLE 8**

| **Intermediate (B No.,C No.)** | **Structure** | **IUPAC Name** | **MS (M+1)** |
|---|---|---|---|
| **BC05 (B02, C20)** | 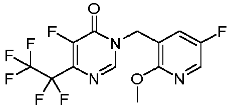 | 5-fluoro-3-((5-fluoro-2-methoxypyridin-3-yl)methyl)-6-(perfluoroethyl)pyrimidin-4(3H)-one | 372.1 |
| **BC06 (B08, C16)** | 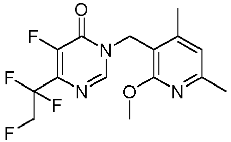 | 5-fluoro-3-((2-methoxy-4,6-dimethylpyridin-3-yl)methyl)-6-(1,1,2-trifluoroethyl)pyrimidin-4(3H)-one | 346.4 |

### EXAMPLE 1

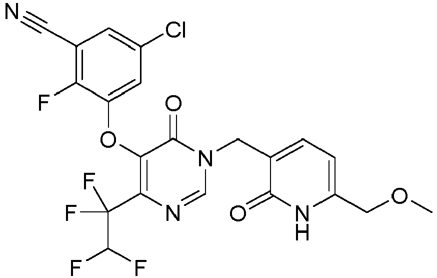

### 5-chloro-2-fluoro-3-((1-((6-(methoxymethyl)-2-oxo-1,2-dihydropyridin-3-yl)methyl)-6-oxo-4-(1,1,2,2-tetrafluoroethyl)-1,6-dihydropyrimidin-5-yl)oxy)benzonitrile

### Step 1: 5-chloro-2-fluoro-3-((1-((2-methoxy-6-(methoxymethyl)pyridin-3-yl)methyl)-6-oxo-4-(1,1,2,2-tetrafluoroeLhyl)-1,6-dihydropyrimidin-5-yl)oxy)benzonitrile

To a stirred solution of 3-(chloromethyl)-2-methoxy-6-(methoxymethyl)pyridine, **C15** (30 mg, 0.149 mmol), 5-chloro-2 -fluoro-3-((6-oxo-4-(1,1,2,2-tetrafluoroethyl)-1,6-dihydropyrimidin-5-yl)oxy)benzonitrile **AB04** (35 mg, 0.086 mmol) in DMF (1 mL), were added K₂CO₃ (23.81 mg, 0.172 mmol) and lithium bromide (11.22 mg, 0.129 mmol). The resulting mixture was stirred at 15 °C for 1 h. TLC showed the completion of the reaction and the mixture was diluted with H₂O (10 mL) and extracted with EtOAc (3×10 mL). The combined organic layers were washed with brine (10 mL), dried over anhydrous Na₂SO₄, filtered and concentrated under reduced pressure. The residue was purified by preparative TLC plate (50% EtOAc/petrlum ether) to give the title compound. MS: 531 (M+1).

### Step 2: 5-chloro-2-fluoro-3-((1-((6-(methoxymethyl)-2-oxo-1,2-dihydropyridin-3-yl)methyl)-6-oxo-4-(1,1,2,2-tetrafluoroethyl)-1,6-dihydropyrimidin-5-yl)oxy)benzonitrile

To a stirred solution of 5-chloro- 2 -fluoro-3-((1-((2-methoxy- 6 -(methoxymethyl) pyridin-3-yl)methyl)-6-oxo-4-(1,1,2,2-tetrafluoroethyl)-1,6-dihydropyrimidin-5-yl)oxy) benzonitrile (25 mg, 0.047 mmol) in ACN (0.5 mL), were added KI (23.45 mg, 0.141 mmol) and TMS-C1 (0.018 ml, 0.141 mmol) under N₂. The resulting mixture was stirred at 70 °C for 1 hour. TLC showed the completion of the reaction. The mixture was purified by reverse phase HPLC (ACN/water with 0.1% TFA as modifier) to isolate the title compound. MS: 517 (M+1). ¹H NMR (400 MHz, CDCl3): δ9.79 (s, 1 H) 8.78 (s, 1 H) 7.69 (d, *J*=6.84 Hz, 1 H) 7.31 (m, *J*=4.41, 2.43 Hz, 1 H) 7.07 (dd, *J*=6.84, 2.43 Hz, 1 H) 6.15 - 6.45 (m, 1 H) 6.10 (d, *J*=6.84 Hz, 1 H) 4.94 (s, 2 H) 4.36 (s, 2 H) 3.45 (s, 3 H).

The following examples in **TABLE 9** were prepared in an analogous manner of that described above for the synthesis of **Example 1** using an appropriate intermediate **AB** and intermediate **C.**

**TABLE 9**

| **EXAMPLE (AB No., C No.)** | **Structure** | **IUPAC Name** | **MS (M+1)** |
|---|---|---|---|
| **2 (AB13, C16)** | 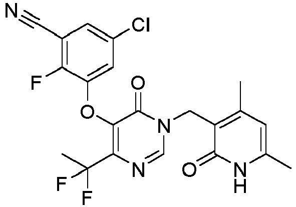 | 5-chloro-3-((4-(1,1-difluoroethyl)-1-((4,6-dimethyl-2-oxo-1,2-dihydropyridin-3-yl)methyl)-6-oxo-1,6-dihydropyrimidin-5-yl)oxy)-2-fluorobenzonitrile | 465.0 |
| **3 (AB13, C17)** | 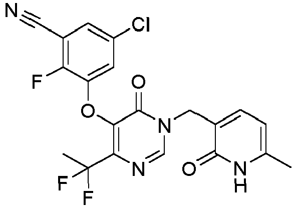 | 5-chloro-3-((4-(1,1-difluoroethyl)-1-((6-methyl-2-oxo-1,2-dihydropyridin-3-yl)methyl)-6-oxo-1,6-dihydropyrimidin-5-yl)oxy)-2-fluorobenzonitrile | 451.1 |
| **4 (AB16, C17)** | 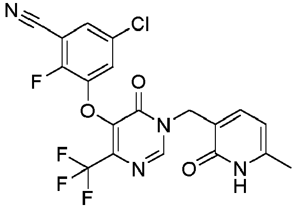 | 5-chloro-2-fluoro-3-((1-((6-methyl-2-oxo-1,2-dihydropyridin-3-yl)methyl)-6-oxo-4-(trifluoromethyl)-1,6-dihydropyrimidin-5-yl)oxy)benzonitrile | 455.0 |
| **5 (AB01, C16)** | 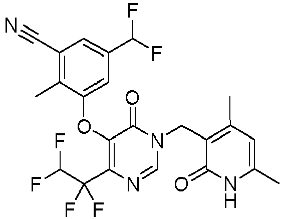 | 5-(difluoromethyl)-3-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile | 513.2 |
| **6 (AB08, C16)** | 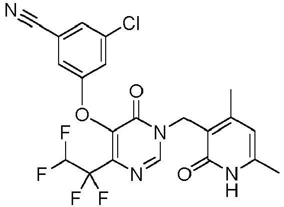 | 3-chloro-5-((1-((4,6-dimethyl-2-oxo-1,2-dihydropyridin-3- yl)methyl)-6-oxo-4-(1,1,2,2-tetrafluoroethyl)-1,6-dihydropyrimidin-5-yl)oxy)benzonitrile | 483.0 |
| **7 (AB07, C16)** | 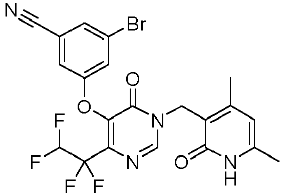 | 3-bromo-5-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)benzonitrile | 527.0 and 529.0 |
| **8 (AB10, C18)** | 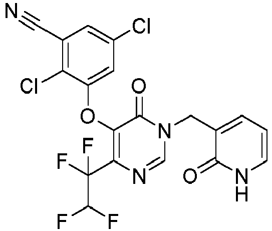 | 2,5-dichloro-3-((6-oxo-1-((2-oxo-1,2-dihydropyridin-3-yl)methyl)-4-(1,1,2,2-tetrafluoroethyl)-1,6-dihydropyrimidin-5-yl)oxy)benzonitrile | 489.0 |
| **9 (AB11, C17)** | 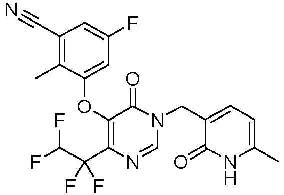 | 5-fluoro-2-methyl-3-((1-((6-methyl-2-oxo-1,2-dihydropyridin-3-yl)methyl)-6-oxo-4-(1,1,2,2-tetrafluoroethyl)-1,6-dihydropyrimidin-5-yl)oxy)benzonitrile | 467.2 |
| **10 (AB05, C16)** | 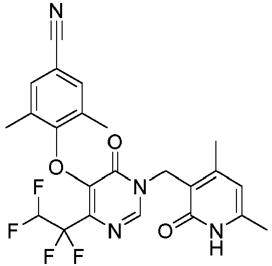 | 4-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)-3,5-dimethylbenzonitrile | 477.4 |
| **11 (AB01, C24)** | 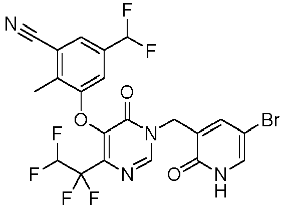 | 3-((1-((5-bromo-2-oxo-1,2-dihydropyridin-3-yl)methyl)-6-oxo-4-(1,1,2,2-tetrafluoroethyl)-1,6-dihydropyrimidin-5-yl)oxy)-5-(difluoromethyl)-2-methylbenzonitrile | 565.2 and 567.2 |
| **12 (AB14, C14)** | 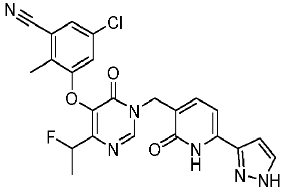 | 5-chloro-3-((4-(1-fluoroethyl)-6-oxo-1-((2-oxo-6-(1H-pyrazol-3-yl)-1,2-dihydropyridin-3-yl)methyl)-1,6-dihydropyrimidin-5-yl)oxy)-2-methylbenzonitrile | 481.1 |
| **13 (AB15, C14)** | 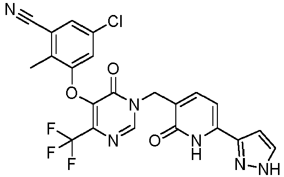 | 5-chloro-2-methyl-3-((6-oxo-1-((2-oxo-6-(1H-pyrazol-3-yl)-1,2-dihydropyridin-3-yl)methyl)-4-(trifluoromethyl)-1,6-dihydropyrimidin-5-yl)oxy)benzonitrile | 503.1 |

### EXAMPLE 14

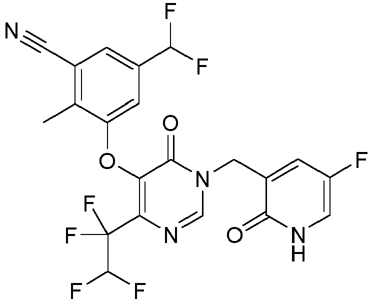

### 5-(difluoromethyl)-3-((1-((5-fluoro-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile

### Step 1: 5-(difluoromethyl)-3-((1-((5-fluoro-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile

To a stirred solution of 5-(difluoromethyl)-2-methyl-3-((6-oxo-4-(1,1,2,2-tetrafluoroethyl)-1,6-dihydropyrimidin-5-yl)oxy)benzonitrile, **AB01** (30 mg, 0.080 mmol) and 3-(chloromethyl)-5-fluoro-2-methoxypyridine, **C20** (15.36 mg, 0.087 mmol) in NMP (795 µL) at room temperature, was added potassium carbonate (21.98 mg, 0.159 mmol). The resulting mixture was stirred at room temperature overnight, and then diluted with water (3 mL) and extracted with EtOAc (3 mL). The organic layer was concentrated under reduced pressure to afford the title compound, which was used in the next step without further purification. MS: 517.1 (M+1).

### Step 2: 5-(difluoromethyl)-3-((1-((5-fluoro-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile

To a solution of 5-(difluoromethyl)-3-((1-((5-fluoro-2-methoxypyridin-3-yl)methyl)-6-oxo-4-(1,1,2,2-tetrafluoroethyl)-1,6-dihydropyrimidin-5-yl)oxy)-2-methylbenzonitrile (40 mg, 0.077 mmol) in ACN (775 µL), was added TMS-I (10.54 µL, 0.077 mmol) at room temperature dropwise. After addition, the resulting mixture was heated to 60 °C for 1 hour, and then diluted with EtOAc (2 mL) and quenched with saturated aqueous NaHCO₃ solution (1 mL) and saturated aqueous Na₂S₂O₃ solution (1 mL). The organic layer was separated and concentrated under reduced pressure. The residue was purified by reverse phase HPLC (ACN/water with 0.1% TFA as modifier) to isolate the title compound. MS: 503.1 (M+1). ¹H NMR (600 MHz, DMSO-*d*₆) δ 8.73 (s, 1H), 7.73 (s, 1H), 7.61 - 7.56 (m, 2H), 7.31 (s, 1H), 6.94 (d, *J* = 16.0 Hz, 1H), 6.86 (s, 1H), 6.77 (s, 1H), 4.87 (s, 2H), 2.47 (s, 3H).

The following examples in **TABLE 10** were prepared in an analogous manner of that described above for the synthesis of **Example 14** using an appropriate Intermediate **AB** and Intermediate **C.**

**TABLE 10**

| **EXAMPLE (AB No., C No.)** | **Structure** | **IUPAC Name** | **MS (M+1)** |
|---|---|---|---|
| **15 (AB17, C16)** | 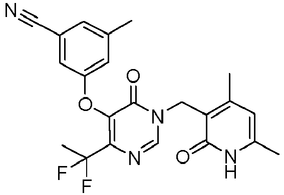 | 3-((4-(1,1-difluoroethyl)-1-((4,6-dimethyl-2-oxo-1,2-dihydropyridin-3-yl)methyl)-6-oxo-1,6-dihydropyrimidin-5-yl)oxy)-5-methylbenzonitrile | 427.2 |
| **16 (AB02, C18)** | 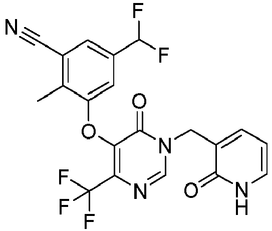 | 5-(difluoromethyl)-2-methyl-3-((6-oxo-1-((2-oxo-1,2-dihydropyridin-3-yl)methyl)-4-(trifluoromethyl)-1,6-dihydropyrimidin-5-yl)oxy)benzonitrile | 453.1 |
| **17 (AB01, C18)** | 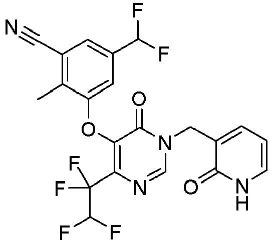 | 5-(difluoromethyl)-2-methyl-3-((6-oxo-1-((2-oxo-1,2-dihydropyridin-3-yl)methyl)-4-(1,1,2,2-tetrafluoroethyl)-1,6-dihydropyrimidin-5-yl)oxy)benzonitrile | 485.1 |
| **18 (AB02, C20)** | 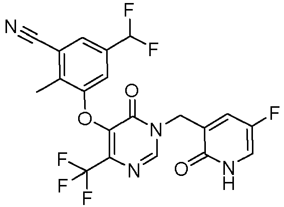 | 5-(difluoromethyl)-3-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile | 471.1 |
| **19 (AB15, C20)** | 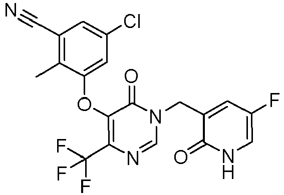 | 5-chloro-3-((1-((5-fluoro-2-oxo-1,2-dihydropyridin-3-yl)methyl)-6-oxo-4-(trifluoromethyl)-1,6-dihydropyrimidin-5-yl)oxy)-2-methylbenzonitrile | 455.1 |
| **20 (AB06, C20)** | 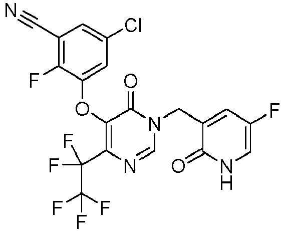 | 5-chloro-2-fluoro-3-((1-((5-fluoro-2-oxo-1,2-dihydropyridin-3-yl)methyl)-6-oxo-4-(perfluoroethyl)-1,6-dihydropyrimidin-5-yl)oxy)benzonitrile | 509.1 |

### EXAMPLE 21

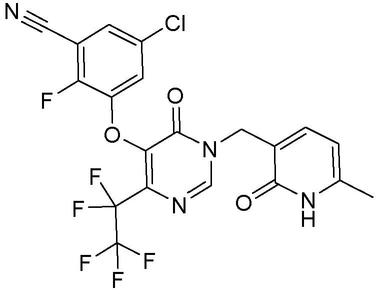

### 5-chloro-2-fluoro-3-((1-((6-methyl-2-oxo-1,2-dihydropyridin-3-yl)methyl)-6-oxo-4-(perfluoroethyl)-1,6-dihydropyrimidin-5-yl)oxy)benzonitrile

### Step 1: 5-chloro-2-fluoro-3-((1-((2-methoxy-6-methylpyridin-3-yl)methyl)-6-oxo-4-(perfluoroethyl)-1,6-dihydropyrimidin-5-yl)oxy)benzonitrile

To an ice cold solution of 5-chloro-2-fluoro-3-((6-oxo-4-(perfluoroethyl)-1,6-dihydropyrimidin-5-yl)oxy)benzonitrile, **AB06** (50 mg, 0.130 mmol) and (2-methoxy-6-methylpyridin-3-yl)methanol, **C17** (20.0 mg, 0.13 mmol) in DCM (1303 µL), was added DIAD (25.3 µL, 0.130 mmol) dropwise. The resulting mixture was allowed to warm to room temperature, and then diluted with saturated aqueous NaHCO₃ solution, and extracted with DCM. Organic layer was dried over anhydrous MgSO₄, filtered and concentrated under reduced pressure. The residue was purified on column chromatography on silica (0-100% EtOAc/ hexanes) to afford the title compound. MS: 519.1 (M+1).

### Step 2: 5-chloro-2-fluoro-3-((1-((6-methyl-2-oxo-1,2-dihydropyridin-3-yl)methyl)-6-oxo-4-(perfluoroethyl)-1,6-dihydropyrimidin-5-yl)oxy)benzonitrile

To a solution of 5-chloro-2-fluoro-3-((1-((2-methoxy-6-methylpyridin-3-yl)methyl)-6-oxo-4-(perfluoroethyl)-1,6-dihydropyrimidin-5-yl)oxy)benzonitrile (39 mg, 0.075 mmol) and potassium iodide (37.4 mg, 0.226 mmol) in ACN (752 µL), was added TMS-Cl (28.8 µl, 0.226 mmol). The resulting mixture was heated to 50 °C for 1 hour, and then cooled to room temperature before diluted with MeOH (1 mL), and concentrated under reduced pressure. The residue was purified by reverse phase HPLC (ACN/water with 0.1% TFA as modifier) to isolate the title compound. MS: 505.0 (M+1). ¹H NMR (500 MHz, Chloroform-*d*) δ 8.78 (s, 1H), 7.68 (d, *J* = 6.9 Hz, 1H), 7.33 (s, 1H), 7.10 (d, *J=* 6.8 Hz, 1H), 6.12 (d, *J* = 7.5 Hz, 1H), 4.94 (s, 2H), 2.36 (s, 3H).

The following example 22 in **TABLE 11** was prepared in an analogous manner of that described above for the synthesis of **Example 21** using an appropriate intermediate **AB** and intermediate **C.**

**TABLE 11**

| **EXAMPLE (AB No., C No.)** | **Structure** | **IUPAC Name** | **MS (M+1)** |
|---|---|---|---|
| **22 (AB01, C17)** | 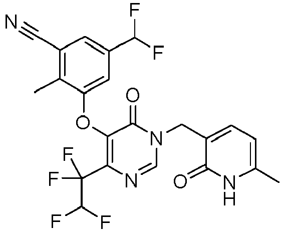 | 5-(difluoromethyl)-2-methyl-3-((1-((6-methyl-2-oxo-1,2-dihydropyridin-3-yl)methyl)-6-oxo-4-(1,1,2,2-tetrafluoroethyl)-1,6-dihydropyrimidin-5-yl)oxy)benzonitrile | 499.1 |

### EXAMPLE 23

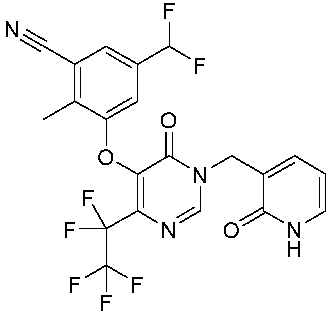

### 5-(difluoromethyl)-2-methyl-3-((6-oxo-1-((2-oxo-1,2-dihydropyridin-3-yl)methyl)-4-(perfluoroethyl)-1,6-dihydropyrimidin-5-yl)oxy)benzonitrile

### Step 1: 5-(difluoromethyl)-3-((1-((2-methoxypyridin-3-yl)methyl)-6-oxo-4-(perfluoroethyl)-1,6-dihydropyrimidin-5-yl)oxy)-2-methylbenzonitrile

To a solution of 5-(difluoromethyl)-2-methyl-3-((6-oxo-4-(perfluoroethyl)-1,6-dihydropyrimidin-5-yl)oxy)benzonitrile, **AB03** (50 mg, 0.127 mmol) in THF (633 µL), was added (2-methoxypyridin-3-yl)methanol, **C18** (19.36 mg, 0.139 mmol) and triphenylphosphine (127 mg, 0.380 mmol), followed by the addition of (E)-di-tert-butyl diazene-1,2-dicarboxylate (64.1 mg, 0.278 mmol). The resulting mixture was stirred overnight, filtered and concentrated under reduced pressure to give the title compound. MS: 517.1 (M+1).

### Step 2: 5-(difluoromethyl)-2-methyl-3-((6-oxo-1-((2-oxo-1,2-dihydropyridin-3-yl)methyl)-4-(perfluoroethyl)-1,6-dihydropyrimidin-5-yl)oxy)benzonitrile

To a solution of 5-(difluoromethyl)-3-((l-((2-methoxypyridin-3-yl)methyl)-6-oxo-4-(perfluoroethyl)-1,6-dihydropyrimidin-5-yl)oxy)-2-methylbenzonitrile (65 mg, 0.126 mmol) in DMA (629 µL), was added pyridine hydrochloride (43.6 mg, 0.378 mmol). The resulting mixture was stirred at 120 °C for 60 minutes, and then purifed by reverse phase HPLC (ACN/water with 0.1% TFA as modifier) to isolate the title compound. MS: 503.1 (M+1). ¹H NMR (500 MHz, DMSO-*d₆*) δ 11.80 (s, 1H), 8.77 (s, 1H), 7.73 (s, 1H), 7.42 (d, *J* = 6.8 Hz, 1H), 7.37 (s, 2H), 6.12 (t, *J* = 6.7 Hz, 1H), 4.85 (s, 2H), 2.44 (s, 3H).

### EXAMPLE 24

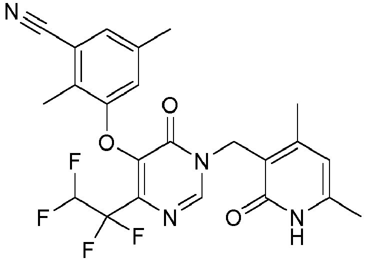

### 3-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)-2,5-dimethylbenzonitrile

### Step 1: 5-chloro-3-((1-((2-methoxy-4,6-dimethylpyridin-3-yl)methyl)-6-oxo-4-(1,1,2,2-tetrafluoroethyl)-1,6-dihydropyrimidin-5-yl)oxy)-2-methylbenzonitrile

To a stirred solution of 5-chloro-2-methyl-3-((6-oxo-4-(1,1,2,2-tetrafluoroethyl)-1,6-dihydropyrimidin-5-yl)oxy)benzonitrile, **AB09** (90 mg, 0.249 mmol) and 3-(chloromethyl)-2-methoxy-4,6-dimethylpyridine, **C16** (46.2 mg, 0.249 mmol) in DMF (3 mL) followed by addition of potassium carbonate (68.8 mg, 0.498 mmol). The resulting mixture was stirred at RT for 5h, and then diluted with water (30 mL) and extracted with EtOAc (2×30 mL). The combined organic layers were washed with brine (20 mL), dried over anhydrous Na₂SO₄, filtered and concentrated under reduced pressure. The residue was purified by flash chromatography on silica (0-60% EtOAc/P.E.) to afford the title compound. MS: 510.8 (M+1).

### Step 2: 3-((1-((2-methoxy-4,6-dimethylpyridin-3-yl)methyl)-6-oxo-4-(1,1,2,2-tetrafluoroethyl)-1,6-dihydropyrimidin-5-yl)oxy)-2,5-dimethylbenzonitrile

To a stirred solution of 5-chloro-3-((1-((2-methoxy-4,6-dimethylpyridin-3-yl)methyl)-6-oxo-4-(1,1,2,2-tetrafluoroethyl)-1,6-dihydropyrimidin-5-yl)oxy)-2-methylbenzonitrile (70 mg, 0.137 mmol) in THF (3 mL), were added cesium carbonate (134 mg, 0.411 mmol) and PdCl₂(dppf) (22.38 mg, 0.027 mmol) and trimethylboroxine (25.8 mg, 0.206 mmol). The resulting mixture was heated to 100°C overnight, and then quenched with water (10mL),and extratced with EtOAc (2×20mL). The combined organic layers were dried over anhydrous Na₂SO₄, filtered and concentrated under reduced pressure. The residue was purified by flash chromatography on silica (0-40% EtOAc/P.E.) to afford the title compound. MS: 490.8 (M+1).

### Step 3: 3-((1-((2-hydroxy-4,6-dimethylpyridin-3-yl)methyl)-6-oxo-4-(1,1,2,2-tetrafluoroethyl)-1,6-dihydropyrimidin-5-yl)oxy)-2,5-dimethylbenzonitrile

To a stirred and ice cold solution of 3-((1-((2-methoxy-4,6-dimethylpyridin-3-yl)methyl)-6-oxo-4-(1,1,2,2-tetrafluoroethyl)-1,6-dihydropyrimidin-5-yl)oxy)-2,5-dimethylbenzonitrile (60 mg, 0.122 mmol) in ACN (3 mL) was added sodium iodide (36.7 mg, 0.245 mmol) and TMS-Cl (0.031 ml, 0.245 mmol). The resulting mixture was heated to 80 °C for 1h, and then quenched with water (10 mL), and extracted with EtOAc (2×20 mL). The combined organic layers were dried over anhydrous Na₂SO₄,filtered and concentrated under reduced pressure. The residue was purified by reverse phase HPLC (ACN/water with 0.1% TFA as modifier) to isolate the title compound. MS: 477.2 (M+1).

### EXAMPLE 25

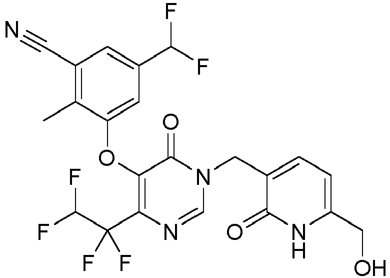

### 5-(difluoromethyl)-3-((1-((6-(hydroxymethyl)-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile

### Step 1: 3-((1-((6-chloro-2-methoxypyridin-3-yl)methyl)-6-oxo-4-(1,1,2,2-tetrafluoroethyl)-1,6-dihydropyrimidin-5-yl)oxy)-5-(difluoromethyl)-2-methylbenzonitrile

To a stirred solution of 6-chloro-3-(chloromethyl)-2-methoxypyridine, **C13** (244 mg, 1.272 mmol) and 5-(difluoromethyl)-2-methyl-3-((6-oxo-4-(1,1,2,2-tetrafluoroethyl)-1,6-dihydropyrimidin-5-yl)oxy)benzonitrile, **AB01** (480 mg, 1.272 mmol) in NMP (3181 µL) at room temperature, was added K₂CO₃ (352 mg, 2.54 mmol). The resulting mixture was stirred at 25°C overnight, and then diluted with water (50 mL) and stirred at room temperature for 30 minutes. The precipitate was collected on top of a filter, washed with water (3×), then air-dried to provide the title compound. MS: 533.3 (M+1).

### Step 2: 5-(difluoromethyl)-3-((1-((6-(hydroxymethyl)-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile

To a N₂ purged mixture of 3-((1-((6-chloro-2-methoxypyridin-3-yl)methyl)-6-oxo-4-(1,1,2,2-tetrafluoroethyl)-1,6-dihydropyrimidin-5-yl)oxy)-5-(difluoromethyl)-2-methylbenzonitrile (20 mg, 0.038 mmol) , potassium trifluoro(((4-methoxybenzyl) oxy)methyl)borate (14.53 mg, 0.056 mmol) and cataCXium^{®} A Pd G2 (5.02 mg, 7.51 µmol) in 1,4-dioxane (1 mL), was added 2 M aqueous solution of cesium carbonate. (0.5 mL, 1.0 mmol). The resulting mixture was heated at 150 °C in microwave oven for 15 minutes, and then extracted with EtOAc. The organic layer was dried over anhydrous Na₂SO₄, filtered and concentrated under reduced pressure. The residue was taken up with ACN (1 mL), and treated with KI (6.23 mg, 0.038 mmol), and TMS-Cl (40 µl, 0.313 mmol). The resulting mixture was heated at 100 °C in microwave oven for 10 minutes, and then purified by reverse phase HPLC (ACN/water with 0.1% TFA as modifier) to isolate the title compound. MS: 515.2 (M+1).

The following examples in **TABLE 12** were prepared in an analogous manner of that described above for the synthesis of **Example 25** using the intermediate of Step 1 in the synthesis of **Example 25** with an appropriate heteroaryl or alkyl boronate in the second column.

**TABLE 12**

| **Example No.** | **Commercial starting material** | **Structure** | **IUPAC Name** | **MS (M+1)** |
|---|---|---|---|---|
| **26** | potassium methoxymethyl trifluoroborate | 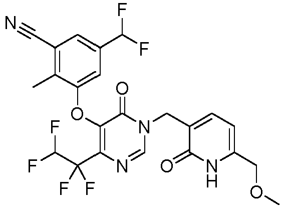 | 5-(difluoromethyl)-3-((1-((6-(methoxymethyl)-2-oxo-1,2-dihydropyridin-3 -yl)methyl)-6-oxo-4-(1,1,2,2-tetrafluoroethyl)-1,6-dihydropyrimidin-5-yl)oxy)-2-methylbenzonitrile | 529.3 |
| **27** | 4-(4,4,5,5-tetramethyl-1,3,2-dioxaborolan-2-yl)-1-((2-(trimethylsilyl) ethoxy)methyl)-1H-pyrazole | 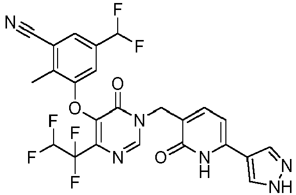 | 5-(difluoromethyl)-3-((1- ((2-hydroxy-6-(1H-pyrazol-4-yl)pyridin-3-yl)methyl)-6-oxo-4-(1,1,2,2-tetrafluoroethyl)-1,6-dihydropyrimidin-5-yl)oxy)-2-methylbenzonitrile | 551.0 |
| **28** | potassium trifluoro(3-hydroxypropyl) borate | 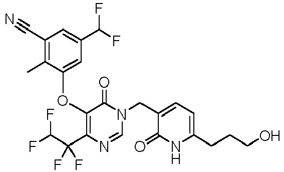 | 5-(difluoromethyl)-3-((1- ((6-(3-hydroxypropyl)-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile | 543.2 |

### EXAMPLE 29

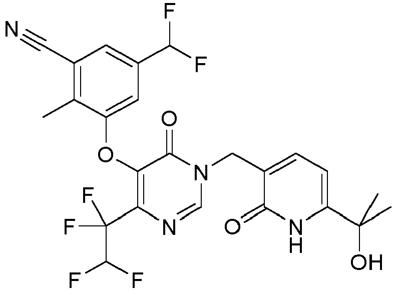

### 5-(difluoromethyl)-3-((1-((6-(2-hydroxypropan-2-yl)-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile

### Step 1: 3-((1-((6-acetyl-2-methoxypyridin-3-yl)methyl)-6-oxo-4-(1,1,2,2-tetrafluoroethyl)-1,6-dihydropyrimidin-5-yl)oxy)-5-(difluoromethyl)-2-methylbenzonitrile

A mixture of 3-((1-((6-chloro-2-methoxypyridin-3-yl)methyl)-6-oxo-4-(1,1,2,2-tetrafluoroethyl)-1,6-dihydropyrimidin-5-yl)oxy)-5-(difluoromethyl)-2-methylbenzonitrile (intermediate from Step 1 of **Example 25**) (100 mg, 0.188 mmol), bis(triphenylphosphine) palladium(II) dichloride (26.3 mg, 0.038 mmol), and tributyl(1-ethoxyvinyl)tin (127 µL, 0.375 mmol) in tol (1877 µL) was stirred at 100 °C overnight, and then diluted with EtOAc and saturated aqueous KF solution, and treated with concentrated HCl (1 mL). The mixture was stirred at r.t overnight, and passed through CELITE^{®} in a funnel. The filtrate was extacted with EtOAc (2X). The combined organic layers was concentrated under reduced pressure. The residue was purified with column chromatography on silica (0-100% EtOAc/hexanes) to afford the title compound. MS: 541.4 (M+1).

### Step 2: 5-(difluoromethyl)-3-((1-((6-(2-hydroxypropan-2-yl)-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile

To a solution of 3-((1-((6-acetyl-2-methoxypyridin-3-yl)methyl)-6-oxo-4-(1,1,2,2-tetrafluoroethyl)-1,6-dihydropyrimidin-5-yl)oxy)-5-(difluoromethyl)-2-methylbenzonitrile (30 mg, 0.056 mmol) in THF (278 µL) at -78 °C, was added a 3 M solution of methylmagnesium bromide in Et₂O (37.0 µL, 0.111 mmol) dropwise. Once LCMS showed complete conversion, the mixture was quenched with MeOH and water, and then partitioned between EtOAc and satureated aqueous NaHCO₃ solution. The organic layer was dried over anhydrous Na₂SO₄, filtered and concentrated under reduced pressure. The residue was taken up with ACN (2 mL), and treated with KI (9.22 mg, 0.056 mmol) and TMS-Cl (20 µL, 0.156 mmol). The resulting mixture was heated at 100 °C in microwave oven for 10 minutes. The mixture was purified with reverse phase HPLC (ACN/water with 0.1% TFA as modifier) to isolate the title compound. MS: 543.4 (M+1). ¹H NMR (600 MHz, Acetone-*d*₆) δ 8.77 (s, 1H), 7.67 - 7.54 (m, 2H), 7.22 (s, 1H), 6.77 (d, *J* = 11.9 Hz, 1H), 6.70 - 6.65 (m, 1H), 6.26 (d, *J* = 7.2 Hz, 1H), 4.94 (s, 2H), 2.03 (s, 3H), 1.62 (s, 1H), 1.53 (s, 6H).

### EXAMPLE 30

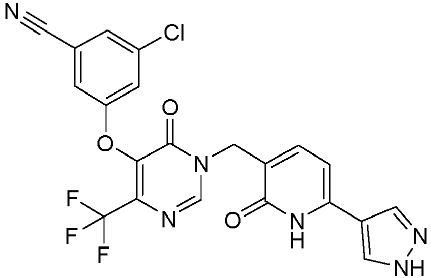

### 3-chloro-5-((6-oxo-1-((2-oxo-6-(1H-pyrazol-4-yl)-1,2-dihydropyridin-3-yl)methyl)-4-(trifluoromethyl)-1,6-dihydropyrimidin-5-yl)oxy)benzonitrile

### Step 1: 3-((1-((6-bromo-2-methoxypyridin-3-yl)methyl)-6-oxo-4-(trifluoromethyl)-1,6-dihydropyrimidin-5-yl)oxy)-5-chlorobenzonitrile

To a stirred solution of 3-chloro-5-((6-oxo-4-(trifluoromethyl)-1,6-dihydropyrimidin-5-yl)oxy)benzonitrile (2.27 g, 7.19 mmol) in toluene (30 mL), was added (6-bromo-2-methoxypyridin-3-yl)methanol **C02** (1.3g, 5.96 mmol), triphenylphosphine (3.15g, 12.01 mmol) and DIAD (2.337 ml, 12.02 mmol). The resulting mixture was stirred at room temperature over night, and then diluted with EtOAc. The organic layer was washed with water and brine, dried over anhydrous Na₂SO₄, filtered and concentrate under reduced pressure. The residue was purified by colunm chromatography on silica (30% EtOAc/P.E.) to afford the title compound.

### Step 2: 3-((1-((6-bromo-2-oxo-1,2-dihydropyridin-3-yl)methyl)-6-oxo-4-(trifluoromethyl)-1,6-dihydropyrimidin-5-yl)oxy)-5-chlorobenzonitrile

To a stirred solution of 3-((1-((6-bromo-2-methoxypyridin-3-yl)methyl)-6-oxo-4-(trifluoromethyl)-1,6-dihydropyrimidin-5-yl)oxy)-5-chlorobenzonitrile (1.8 g, 3.49 mmol) in ACN (20 mL) was added sodium iodide (1.046 g, 6.98 mmol) and TMS-Cl (0.892 ml, 6.98 mmol). The reaction mixture was stirred at 80 °C overnight, then cooled to room temperature and added some more ACN. stirred and filterred. The filter cake was washed with ACN. The filtrate was concentrated under reduced pressure to give the title compound. MS: 501.2 and 503.2 (M+1).

### Step 3: 3-chloro-5-((6-oxo-1-((2-oxo-6-(1H-pyrazol-4-yl)-1,2-dihydropyridin-3-yl)methyl)-4-(trifluoromethyl)-1,6-dihydropyrimidin-5-yl)oxy)benzonitrile

A mixture of 3-((1-((6-bromo-2-oxo-1,2-dihydropyridin-3-yl)methyl)-6-oxo-4-(trifluoromethyl)-1,6-dihydropyrimidin-5-yl)oxy)-5-chlorobenzonitrile (20 mg, 0.040 mmol), 4-(4,4,5,5-tetramethyl-1,3,2-dioxaborolan-2-yl)-1H-pyrazole (9.28 mg, 0.048 mmol), PdCl₂(dppf)-CH₂Cl₂ adduct (3.26 mg, 3.99 µmol), potassium phosphate tribasic (22.85 mg, 0.108 mmol) in 1,4-Dioxane (53.2 µL) and water (26.6 µL) was degassed, and purged with N₂. The resulting mixture was stirred at 80°C for 3h. LCMS showed that the reaction was complete. The solvent was evaporated under reduced pressure. The residue was with reverse phase HPLC (ACN/water with 0.1% TFA as modifier) to isolate the title compound. MS: 489.0 (M+1).

### EXAMPLE 31

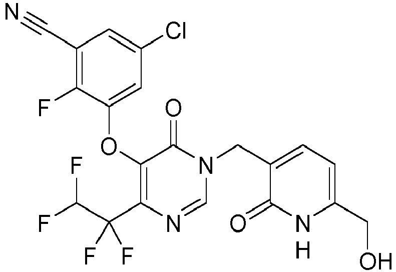

### 5-chloro-2-fluoro-3-((1-((6-(hydroxymethyl)-2-oxo-1,2-dihydropyridin-3-yl)methyl)-6-oxo-4-(1,1,2,2-tetrafluoroethxl)-1,6-dihydropyrimidin-5-yl)oxy)benzonitrile

### Step 1: 5-chloro-2-fluoro-3-((I-((2-metho_{L}cy-6-vinylpyridin-3-yl)methyl)-6-oxo-4-(I,I,2,2-tetrafluoroethyl)-1,6-dihydropyrimidin-5-yl)oxy)benzonitrile

To a solution of 5-chloro-2-fluoro-3-((6-oxo-4-(1,1,2,2-tetrafluoroethyl)-1, 6-dihydropyrimidin-5-yl)oxy)benzonitrile **AB04** (150 mg, 0.410 mmol) and K₂CO₃ (283 mg, 2.051 mmol) in DMF (2 mL), was added 3-(chloromethyl)-2-methoxy-6-vinylpyridine, **C19** (75 mg, 0.410 mmol) and lithium bromide (42.8 mg, 0.492 mmol). The resulting mixture was stirred at 20°C for 20 h, and then quenched with H₂O (5 mL), extracted with EtOAc (3×5 mL). The combined organic layers were dried over anhydrous Na₂SO₄, filtrated and concentrated under reduced pressure. The residue was purified by preparative TLC plate (25% EtOAc/P.E.) to give the title compound. MS: 513.1 (M+1).

### Step 2: 5-chloro-2-fluoro-3-((1-((6-formyl-2-methoxypyridin-3-yl)methyl)-6-oxo-4-(1,1,2,2-tetrafluoroethyl)-1,6-dihydropyrimidin-5-yl)oxy)benzonitrile

To a mixture of 5-chloro-2-fluoro-3-((1-((2-methoxy-6-vinylpyridin-3-yl) methyl)-6-oxo-4-(1,1,2,2-tetrafluoroethyl)-1, 6-dihydropyrimidin-5-yl)oxy)benzonitrile (80 mg, 0.156 mmol) and 2,6-lutidine (0.051 mL, 0.438 mmol) in 1,4-dioxane (3 mL) and H₂O (1 mL), was added osmium tetroxide (1.224 µL, 3.90 µmol) dissolved in t-BuOH (0.1 mL). The resulting mixture was stirred at 20°C for 30 minutes, and to the mixture was added sodium periodate (122 mg, 0.571 mmol). The resulting mixture was stirred at 20°C for 1.5 h, and then diluted with EtOAc (10 mL) and poured in water(10 mL) carefully. The organic layer was washed with saturated aqueous Na₂S₂O₃ (10 mL) and brine(10 mL), dried over anhydrous Na₂SO₄ and concentrated under reduced pressure to afford the title compound. MS: 515.1 (M+1).

### Step 3: 5-chloro-2-fluoro-3-((1-((6-(hydroxymethyl)-2-methoxypyridin-3-yl)methyl)-6-oxo-4-(1,1,2,2-tetrafluoroethyl)-L6-dihydropyrimidin-5-yl)oxy)benzonitrile

To a solution of 5-chloro-2-fluoro-3-((1-((6-formyl-2-methoxypyridin-3-yl)methyl)-6-oxo-4-(1,1,2,2-tetrafluoroethyl)-1,6-dihydropyrimidin-5-yl)oxy)benzonitrile (70 mg, 0.136 mmol) in EtOAc (1 mL) and MeOH (3 mL), was added Zn(BH₄)₂ (0.136 ml, 0.136 mmol). The resulting mixture was stirred at 20 °C for 0.25 h, and then quenched with 1N HCl (10 mL), extracted with EtOAc (3×10 mL). The combined organic layers were dried over anhydrous Na₂SO₄, filtrated and concentrated under reduced pressure to obtain the title compound, which was used directly in the next step. MS: 517.1 (M+1).

### Step 4: 5-chloro-2-fluoro-3-((1-((6-(hydroxymethyl)-2-oxo-1,2-dihydropyridin-3-yl)methyl)-6-oxo-4-(1,1,2,2-tetrafluoroethyl)-1,6-dihydropyrimidin-5-yl)oxy)benzonitrile

To a solution of 5-chloro-2-fluoro-3-((1-((6-(hydroxymethyl)-2-methoxypyridin-3-yl)methyl)-6 -oxo-4-(1,1,2,2-tetrafluoroethyl)-1,6-dihydropyrimidin-5-yl)oxy)benzonitrile (60 mg, 0.116 mmol) in ACN (3.00 mL) was added KI (57.8 mg, 0.348 mmol) and TMS-C1 (0.045 ml, 0.348 mmol). The resulting mixture was stirred at 70°C for 1 hour and then concentrated under reduced pressure. The residue was purified by reverse phase HPLC (ACN/water with 0.1% TFA as modifier) to isolate the title compound. MS: 503.0 (M+1). ¹HNMR (400 MHz, DMSO- *d₆*): δ11.75 (s, 1H), 8.79 (s, 1H), 7.86-7.84 (m, 1 H), 7.69-7.50 (m, 1H), 7.48-7.46 (d, 1H), 6.87-6.74(m, 1H), 6.17-6.15 (m, 1 H), 5.42 (s, 1H), 4.86(s, 2H), 4.27 (s, 2H).

### EXAMPLE 32 (isomerA) and 33 (isomer B)

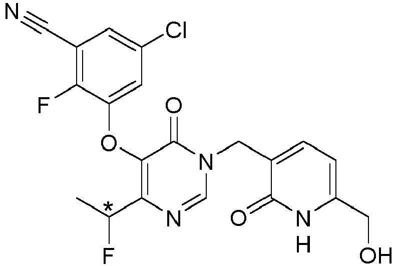

(*S*)-5-chloro-2-fluoro-3-((4-(1-fluoroethyl)-1-((6-(hydroxymethyl)-2-oxo-1,2-dihydropyridin-3-yl)methyl)-6-oxo-1,6-dihydropyrimidin-5-yl)oxy)benzonitrile, and
(*R*)-5-chloro-2-fluoro-3-((4-(1-fluoroethyl)-1-((6-(hydroxymethyl)-2-oxo-1,2-dihydropyridin-3-yl)methyl)-6-oxo-1,6-dihydropyrimidin-5-yl)oxy)benzonitrile

### Step 1: 5-chloro-2-fluoro-3-((4-(1-fluoroethyl)-1-((2-methoxy-6-vinylpyridin-3-yl)methyl)-6-oxo-1,6-dihydropyrimidin-5-yl)oxy)benzonitrile

To a solution of 5-chloro-2-fluoro-3-((4-(1-fluoroethyl)-6-oxo-1,6-dihydropyrimidin-5-yl)oxy)benzonitrile, **AB12** (0.3 g, 0.963 mmol) in DMF (5 mL) was added 3-(chloromethyl)-2-methoxy-6-vinylpyridine, **C19** (0.177 g, 0.963 mmol), K₂CO₃ (0.399 g, 2.89 mmol) and lithium bromide (0.167 g, 1.925 mmol). The resulting mixture was stirred at 20 °C for 1 hour, and then extracted with EtOAc (3×15 mL). The combined organic layers were washed with brine, dried over anhydrous Na₂SO₄, filtrated and concentrated under reduced pressure. The residue was triturated with MeOH to give the title compound, and used directly without further purification.

### Step 2: 5-chloro-2-fluoro-3-((4-(1-fluoroethyl)-1-((6-formyl-2-methoxypyridin-3-yl)methyl)-6-oxo-1,6-dihydropyrimidin-5-yl)oxy)benzonitrile

To a mixture of 5-chloro-2-fluoro-3-((4-(1-fluoroethyl)-1-((2-methoxy-6-vinylpyridin-3-yl)methyl)-6-oxo-1,6-dihydropyrimidin-5-yl)oxy)benzonitrile (200 mg, 0.436 mmol) in 1,4-dioxane (1.5 mL) and water (0.5 mL), were added 2,6-dimethylpyridine (131 mg, 1.220 mmol) and a solution of osmium tetroxide (0.1 mL, 0.436 mmol) in t-BuOH (0.1 mL). The resulting mixture was stirred at 20°C for 15 minutes. Then sodium periodate (336 mg, 1.569 mmol) was added. The reaction was stirred at 20 °C for 1.5 h, and then diluted with water (15 mL) and extracted with EtOAc (3×15 mL). The combined organic layers were dried over anhydrous Na₂SO₄ and concentrated. The residue was purified by column chromatography on silica (0-20% EtOAc/petroleum ether) to give the title compound. MS: 461.1 (M+1).

### Step 3: 5-chloro-2-fluoro-3-((4-(1-fluoroethyl)-1-((6-(hydroxymethyl)-2-methoxypyridin-3-yl)methyl)-6-oxo-1,6-dihydropyrimidin-5-yl)oxy)benzonitrile

To a solution of 5-chloro-2-fluoro-3-((4-(1-fluoroethyl)-1-((6-formyl-2-methoxypyridin-3-yl)methyl)-6-oxo-1,6-dihydropyrimidin-5-yl)oxy)benzonitrile (55 mg, 0.107 mmol) in THF (3 mL) and EtOAc (2 mL), was added 1M Zn(BH₄)₂ solution in THF (0.021 mL, 0.021 mmol) at -30°C. The resulting mixture was stirred at -30°C for 0.5 h. The reaction was quenched with water (1 drop) and then concentrated under reduced pressure to afford the title compound. MS: 463.1 (M+1).

### Step 4: 5-chloro-2-fluoro-3-((4-(1-fluoroethyl)-1-((6-(hydroxymethyl)-2-oxo-1,2-dihydropyridin-3-yl)methyl)-6-oxo-1,6-dihydropyrimidin-5-yl)oxy)benzonitrile;

(S)-5-chloro-2-fluoro-3-((4-(1-fluoroethyl)-1-((6-(hydroxymethyl)-2-oxo-1,2-dihydropyridin-3-yl)methyl)-6-oxo-1,6-dihydropyrimidin-5-yl)oxy)benzonitrile; and
(R)-5-chloro-2-fluoro-3-((4-(1-fluoroethyl)-1-((6-(hydroxymethyl)-2-oxo-1,2-dihydropyridin-3-yl)methyl)-6-oxo-1,6-dihydropyrimidin-5-yl)oxy)benzonitrile

To a solution of 5-chloro-2-fluoro-3-((4-(1-fluoroethyl)-1-((6-(hydroxymethyl)-2-methoxypyridin-3-yl)methyl)-6-oxo-l,6-dihydropyrimidin-5-yl)oxy)benzonitrile ( 49 mg, 0.106 mmol) in ACN (3 mL) was added KI (22.14 mg, 0.133 mmol). TMS-C1 (0.044 ml, 0.347 mmol). The resulting mixture was stirred at 70 °C for 5 h, and concentrated under reduced pressure. The residue was purified by reverse phase HPLC (ACN/water with 0.1% TFA as modifier) to isolate the racemic title compound. MS: 449 (M+1). ¹H NMR (400 MHz, DMSO-*d₆*) δ ppm 4.27 (br d, J=4.63 Hz, 2 H) 4.83 (s, 2 H) 5.46 (s, 1 H) 5.63 - 5.88 (m, 1 H) 6.15 (br d, *J*=7.28 Hz, 1 H) 7.42 (d, *J*=7.06 Hz, 1 H) 7.58 (br d, *J*=5.73 Hz, 1 H) 7.82 (br s, 1 H) 8.68 (s, 1 H). The racemic mixture was purified by chiral SFC (Chiralpak IC-H column, 250 ×30 mm id, 5 µm, IPA/CO₂ = 35%) to give **Example 32** isomer A (faster eluting) and **Example 33** isomer B (slower eluting).

### EXAMPLE 34 (isomer A)

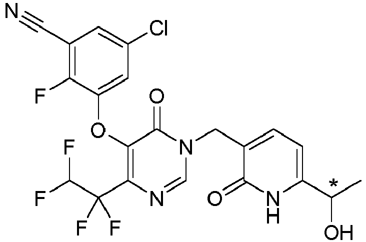

### (S) or (R)-5-chloro-2-fluoro-3-((1-((6-(1-hydroxyethyl)-2-oxo-12-dihydropyridin-3-yl)methyl)-6-oxo-4-(1,1,2,2-tetrafluoroethyl)-1,6-dihydropyrimidin-5-yl)oxy)benzonitrile

### Step 1: 3-((1-((6-acetyl-2-methoxypyridin-3-yl)methyl)-6-oxo-4-(1,1,2,2-tetrafluoroethyl)-1,6-dihydropyrimidin-5-yl)oxy)-5-chloro-2-fluorobenzonitrile

To a solution of 5-chloro-2-fluoro-3-((6-oxo-4-(1,1,2,2-tetrafluoroethyl)-1,6-dihydropyrimidin-5-yl)oxy)benzonitrile, **AB04** (247 mg, 0.676 mmol) in DMF (4 mL), was added 1-(5-(chloromethyl)-6-methoxypyridin-2-yl)ethanone, **C21** (135 mg, 0.676 mmol) and K₂CO₃ (187 mg, 1.352 mmol) and lithium bromide (88 mg, 1.014 mmol). The resulting mixture was stirred at 30°C for 5 h, and diluted with H₂O (15 mL) and extracted with EtOAc (3×30 mL). The combined organic layers were washed with brine (40 mL) and dried over anhydrous Na₂SO₄, filtered and concentrated under reduced pressure. The residue was purified by column chromatography on silica (50% EtOAc/P.E.) to give the title compound. MS: 529.2 (M+1).

### Step 2: 5-chloro-2-fluoro-3-((1-((6-(1-hydroxyethyl)-2-methoxypyridin-3 -yl)methyl)-6-oxo-4-(1,1,2,2-tetrafluoroethyl)-1,6-dihydropyrimidin-5-yl)oxy)benzonitrile

To a solution of 3-((1-((6-acetyl-2-methoxypyridin-3-yl)methyl)-6-oxo-4-(1,1,2,2-tetrafluoroethyl)-1,6-dihydropyrimidin-5-yl)oxy)-5-chloro-2-fluorobenzonitrile (144 mg, 0.272 mmol) in EtOAc (21 mL) was added 1M Zn(BH₄)₂ solution in THF (0.272 ml, 0.272 mmol) at 20 °C. The resulting solution was stirred at 15-20 °C for 0.5 h, and then quenched with 4N HCl aqueous solution (5 mL), and diluted with H₂O (15 mL) and extracted with EtOAc (3×30 mL). The combined organic layers were washed with brine (40 mL) and dried over anhydrous Na₂SO₄, filtered and concentrated under reduced pressure to give the title compound. MS: 531.2 (M+1).

### Step 3: (S) or (R)-5-chloro-2-fluoro-3-((1-((6-(1-hydroxyethyl)-2-oxo-1,2-dihydropyridin-3-yl)methyl)-6-oxo-4-(1,1,2,2-tetrafluoroethyl)-1,6-dihydropyrimidin-5-yl)oxy)benzonitrile

To a solution of 5-chloro-2-fluoro-3-((1-((6-(1-hydroxyethyl)-2-methoxypyridin-3-yl)methyl)-6-oxo-4-(1,1,2,2-tetrafluoroethyl)-1,6-dihydropyrimidin-5-yl)oxy)benzonitrile (185 mg, 0.349 mmol) in ACN (32 mL) was added KI (174 mg, 1.046 mmol), and TMS-Cl (0.134 ml, 1.046 mmol). The resulting mixture was stirred at 70 °C for 2 h, and then diluted with water (15 mL), and extracted with EtOAc (3×30 mL). The combined organic layers were washed with brine (30 mL), dried over anhydrous Na₂SO₄, filtrated and concentrated under reduced pressure. The residue was purified by by reverse phase HPLC (ACN/water with 0.1% TFA as modifier) to isolate the racemic title compound. The racemic mixture was purified by chiral SFC (ChiralPak AD-H column, 250 ×30 mm id, 5 µm, 25% IPA/CO₂ with 0.1% NH₄OH as modifier) to afford **Example 34** (isomer A, faster eluting). MS: 517.0 (M+1). ¹H NMR (400 MHz, DMSO-*d₆*) δ ppm 1.29 (d, *J* = 6.58 Hz, 3 H) 4.49 (br d, *J* = 4.39 Hz, 1 H) 4.86 (s, 2 H) 5.49 (br d, *J* =3.51 Hz, 1 H) 6.17 (d, *J* = 7.45 Hz, 1 H) 6.68 - 7.07 (m, 1 H) 7.47 (d, *J* = 7.02 Hz, 1 H) 7.76 (dd, *J*=7.24, 2.41 Hz, 1 H) 7.85 (dd, *J*=4.38, 2.63 Hz, 1 H) 8.80 (s, 1 H).

### EXAMPLE 35

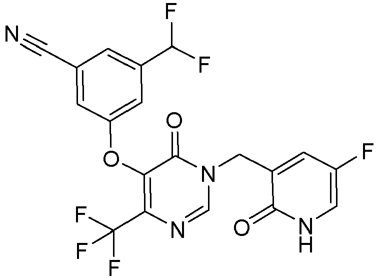

### 3-(difluoromethyl)-5-((1-((5-fluoro-2-oxo-1,2-dihydropyridin-3-yl)methyl)-6-oxo-4-(trifluoromethyl)-1.6-dihydropyrimidin-5-yl)oxy) benzonitrile

Potassium carbonate (34.4 mg, 0.249 mmol) was added to a stirred solution of 5-fluoro-3-((5-fluoro-2-methoxypyridin-3-yl)methyl)-6-(trifluoromethyl)pyrimidin-4(3H)-one, **BC04** (40 mg, 0.125 mmol) and 3-(difluoromethyl)-5-hydroxybenzonitrile, **A01** (25.3 mg, 0.149 mmol) in DMF (623 µL) at room temperature. The resulting mixture was stirred at 100 °C overnight, and then diluted with water (2 mL) and EtOAc (2 mL). The organic layer was separated and concentrated under reduced pressure. The residue was dissolved in ACN (2 mL) and TMS-1 (85 µL, 0.623 mmol) was added at room temperature dropwise. After addition, the resulting mixture was heated to 60 °C for 2 h. The reaction was diluted with EtOAc (2 mL) and quenched with saturated aqueous NaHCO₃ solution (1 mL) and saturated aqueous Na₂S₂O₃ (1 mL). The organic layer was separated and concentrated under reduced pressure. The residue was purified by reverse phase HPLC (ACN/water with 0.1% TFA as modifier) to isolate the title compound. MS: 457.1 (M+1). ¹H NMR (600 MHz, DMSO-*d*₆) δ 8.75 (s, 1H), 7.88 (s, 1H), 7.79 (s, 1H), 7.66 (s, 1H), 7.62 - 7.55 (m, 2H), 7.00 (t, *J* = 55.2 Hz, 1H), 4.88 (s, 2H).

The following examples in **TABLE 13** were prepared in an analogous manner of that described above for the synthesis of **Example 35** using an appropriate intermediate **A** and intermediate **BC.**

**TABLE 13**

| **EXAMPLE (A No., BC No.)** | **Structure** | **IUPAC Name** | **MS (M+1)** |
|---|---|---|---|
| **36 (A03,BC04)** | 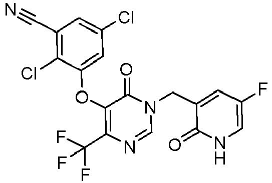 | 2,5-dichloro-3-((1-((5-fluoro-2-oxo-1,2-dihydropyridin-3-yl)methyl)-6-oxo-4-(trifluoromethyl)-1,6-dihydropyrimidin-5-yl)oxy)benzonitrile | 476.0 |
| **37 (A05,BC04)** | 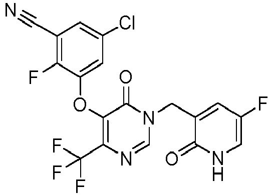 | 5-chloro-2-fluoro-3-((1-((5-fluoro-2-oxo-1,2-dihydropyridin-3-yl)methyl)-6-oxo-4-(trifluoromethyl)-1,6-dihydropyrimidin-5-yl)oxy)benzonitrile | 459.0 |
| **38 (A02,BC05)** | 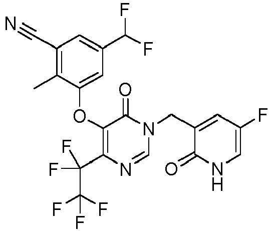 | 5-(difluoromethyl)-3-((1-((5-fluoro-2-oxo-1,2-dihydropyridin-3-yl)methyl)-6-oxo-4-(perfluoroethyl)-1,6-dihydropyrimidin-5-yl)oxy)-2-methylbenzonitrile | 521.1 |

### EXAMPLE 39

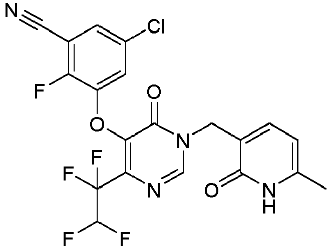

### 5-chloro-2-fluoro-3-((1-((6-methyl-2-oxo-1,2-dihydropyridin-3-yl)methyl)-6-oxo-4-(1,1,2,2-tetrafluoroethyl)-1,6-dihydropyrimidin-5-yl)oxy)benzonitrile

### Step 1: 5-chloro-2-fluoro-3-((1-((2-methoxy-6-methylpyridin-3-yl)methyl)-6-oxo-4-(1,1,2,2-tetrafluoroethyl)-1,6-dihydropyrimidin-5-yl)oxy)benzonitrile

A mixture of 5-fluoro-3-((2-methoxy-6-methylpyridin-3-yl)methyl)-6-(1,1,2,2-tetrafluoroethyl)pyrimidin-4(3H)-one, **BC03** (60 mg, 0.172 mmol), 5-chloro-2-fluoro-3-hydroxybenzonitrile, **A05** (30.9 mg, 0.180 mmol), potassium carbonate (47.5 mg, 0.344 mmol) in DMF (859 µL) was heated to 80 °C overnight and then at 100 °C for the second night. The crude was purified by reverse phase HPLC (ACN/water with 0.1% TFA as modifier) to isolate the title compound. MS: 501.2 (M+1).

### Step 2: 5-chloro-2-fluoro-3-((1-((6-methyl-2-oxo-1,2-dihydropyridin-3-yl)methyl)-6-oxo-4-(1,1,2,2-tetrafluoroethyl)-1,6-dihydropyrimidin-5-yl)oxy)benzonitrile

To a solution of 5-chloro-2-fluoro-3-((1-((2-methoxy-6-methylpyridin-3-yl)methyl)-6-oxo-4-(1,1,2,2-tetrafluoroethyl)-1,6-dihydropyrimidin-5-yl)oxy)benzonitrile (11.5 mg, 0.023 mmol) and potassium iodide (11.44 mg, 0.069 mmol) in ACN (230 µL), was added TMS-Cl (8.80 µL, 0.069 mmol). The resulting mixture was heated to 50 °C for 4 h, and then quenched with MeOH (1 mL). The crude was purified by reverse phase HPLC (ACN/water with 0.1% TFA as modifier) to isolate the title compound. MS: 487.2 (M+1).

The following examples in **TABLE 14** were prepared in an analogous manner of that described above for the synthesis of **Example 39** using an appropriate intermediate **A** and intermediate **BC.**

**TABLE 14**

| **EXAMPLE A No., BC No.** | **Structure** | **IUPAC Name** | **MS (M+1)** |
|---|---|---|---|
| **40 (A01,BC01)** | 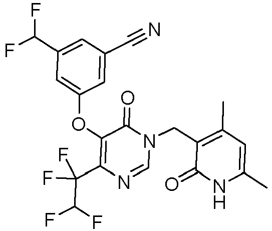 | 3-(difluoromethyl)-5-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)benzonitrile | 499.1 |
| **41 (A02,BC06)** | 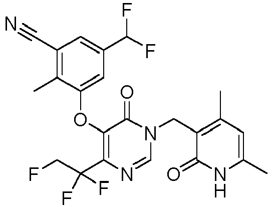 | 5-(difluoromethyl)-3-((1-((4,6-dimethyl-2-oxo-1,2-dihydropyridin-3-yl)methyl)-6-oxo-4-(1,1,2-trifluoroethyl)-1,6-dihydropyrimidin-5-yl)oxy)-2-methylbenzonitrile | 495.4 |

### EXAMPLE 42

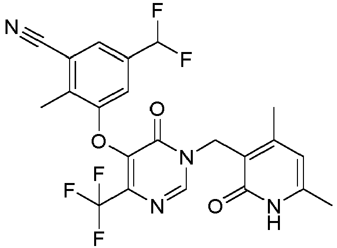

### 5-(difluoromethyl)-3-((1-((4,6-dimethyl-2-oxo-1,2-dihydropyridin-3-yl)methyl)-6-oxo-4-(trifluoromethyl)-1,6-dihydropyrimidin-5-yl)oxy)-2-methylbenzonitrile

### Step 1: 3-((1-((2-chloro-4,6-dimethylpyridin-3-yl)methyl)-6-oxo-4-(trifluoromethyl)-1,6-dihydropyrimidin-5-yl)oxy)-5-(difluoromethyl)-2-methylbenzonitrile

A mixture of 5-(difluoromethyl)-3-hydroxy-2-methylbenzonitrile, **A02** (118 mg, 0.643 mmol), ((2-chloro-4,6-dimethylpyridin-3-yl)methyl)-5-fluoro-6-(trifluoromethyl)pyrimidin-4(3H)-one, **BC02** (180 mg, 0.536 mmol), potassium carbonate (148 mg, 1.072 mmol) in DMF (2 mL) was stirred at 80 °C for 15 h, passed through a filter. The filter cake was washed with EtOAc (10 mL). The filtrate was partitioned between EtOAc and water. The layers were separated. And the aqueoud layer was further extracted with EtOAc (3×10 mL). The combined organic layers were dried over anhydrous MgSO4, filtered and concentrated under reduced pressure. The residue was purified by column chromoatography on silica (0-100% EtOAc/ hexanes) to afford the title compound. MS: 499.1 (M+1).

### Step 2: 5-(difluoromethyl)-3-((1-((4,6-dimethyl-2-oxo-1,2-dihydropyridin-3-yl)methyl)-6-oxo-4-(trifluoromethyl)-1,6-dihydropyrimidin-5-yl)oxy)-2-methylbenzonitrile

A solution of 3-((1-((2-chloro-4,6-dimethylpyridin-3-yl)methyl)-6-oxo-4-(trifluoromethyl)-1,6-dihydropyrimidin-5-yl)oxy)-5-(difluoromethyl)-2-methylbenzonitrile (140 mg, 0.281 mmol) in AcOH (32.100 mL, 561 mmol) was heated at 100 °C for 4 nights until LCMS showed complete conversion. The mixture was concentrated under reduced pressure. The residue was purified by revrse phase HPLC (ACN/water with 0.1% TFA as modifier) to isolate the title compound. MS: 481.1 (M+1). ¹H NMR (500 MHz, Chloroform-*d*) δ 8.77 (s, 1H), 7.45 (s, 1H), 7.26 (d, *J* = 1.8 Hz, 3H), 6.73 (s, 1H), 6.51 (t, *J* = 55.9 Hz, 1H), 6.16 (s, 1H), 5.02 (s, 2H), 2.64 (s, 1H), 2.59 (s, 3H), 2.43 (d, *J* = 1.4 Hz, 3H), 2.35 (s, 3H).

### EXAMPLE 43: Determination of cell kill (SMACK) activity:

PBMCs derived from healthy donors were grown in complete media (RPMI 1640 with L-glutamine; 10% heat inactivated Fetal Bovine Serum; 100 U/mLPenicillin-Streptomycin) containing 5 µg/mL Phytohemagglutinin at about 2.5 × 10⁶ cells/mL for 3 days at 5% CO₂, 37°C, and 90% humidity. On day 4, PHA stimulated cells were washed and resuspended at about 20 × 10⁶ cells/mL in complete media with IL-2 (10 U/mL) with VSV-G pseudotyped HIV virus stock (VSV-G/pNLG1-P2A-ΔEnv - 20 µg/mL p24) and incubated for 4 hours at 37°C, 5% CO₂ and 90% humidity. VSV-G/pNLG1-P2A-ΔEnv is a VSV-G pseudotyped virus derived from pNL43 with *egfp* inserted 5' of *nef* and eGFP expression driven off normal spliced RNA transcripts. Virus contained Vif truncated by 50 amino acids due to deletion of a single nucleotide causing a frameshift and does not express Nef due to a stop codon after *gfp.* HIV Env is not expressed due to a frameshift resulting in multiple stop codons. Infected cells were then washed with complete media plus 10U/mL IL-2 3-times with centrifuging at 200 × g for 3 minutes at 22°C. Cells were resuspended at 5 × 10⁶ cells/mL in complete media plus 10 U/mL IL-2 and incubated overnight at 37°C, 5% CO₂ and 90% humidity. For compound treatment infected PBMCs were diluted to 4 × 10⁵ cells/mL with RPMI 1640 with L-glutamine, 50% Normal Human Serum (NHS), 100 U/mLPenicillin-Streptomycin plus IL-2 (10 U/mL) and 20,000 cells were transferred to each well in a 384-well poly-D-lysine coated compound plate containing compounds with final DMSO <0.5%. Compounds were tested with 10-point 3-fold titration. Plates were analyzed on an Acumen ex3 imager using the Blue Laser 488 nm and the number of GFP positive objects were collected with loss of GFP representing death of infected cells. Titration curves and EC₅₀ values were calculated using a four-parameter logistic fit. Results are shown in Table 15.

**TABLE 15**

| Example | SMACK EC_{50_}50%NHS (nM) | Viking **EC**_{**50**_}50%NHS (nM) |
|---|---|---|
| 1 | 257.7 | 29.55 |
| 2 | 293.5 | 37.37 |
| 3 | 87.63 | 14.05 |
| 4 | 197.3 | 41.15 |
| 5 | 160.8 | 47.11 |
| 6 | 262.7 | 37.51 |
| 7 | 433.3 | 29.11 |
| 8 | 495.4 | 26.93 |
| 9 | 281 | 15.67 |
| 10 | 245.9 | --- |
| 11 | 401.8 | --- |
| 12 | 374.5 | 127.4 |
| 13 | 295.5 | 342.9 |
| 14 | 101.4 | 29.5 |
| 15 | 384.2 | 21.9 |
| 16 | 268.1 | 7.077 |
| 17 | 217.5 | 17.14 |
| 18 | 148.2 | 32.84 |
| 19 | 375.6 | 30.69 |
| 20 | 99.04 | 83.72 |
| 21 | 152 | 76.75 |
| 22 | 131.2 | 48.92 |
| 23 | 233.8 | 53.02 |
| 24 | 346.5 | 168.1 |
| 25 | 72.69 | 19.55 |
| 26 | 90.06 | 29.26 |
| 27 | 292.3 | 77.24 |
| 28 | 73.8 | --- |
| 29 | 187.2 | 29.83 |
| 30 | 414.6 | 30.14 |
| 31 | 86.78 | 20.66 |
| 32 | 377 | 18.25 |
| 33 | 389 | 23.15 |
| 34 | 270.3 | 43.15 |
| 35 | 313.7 | 15.61 |
| 36 | 369.3 | 49.96 |
| 37 | 412.8 | 32.36 |
| 38 | 190.8 | 96.81 |
| 39 | 162.4 | 33.55 |
| 40 | 282.6 | 39.23 |
| 41 | 283.4 | 59.8 |
| 42 | 222.1 | 43.13 |

## Claims

1. A compound of Formula I 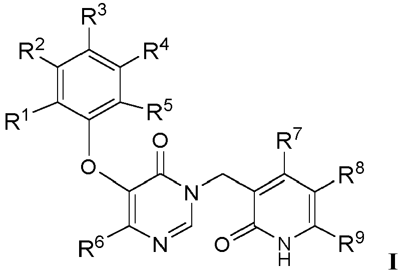 or a pharmaceutically acceptable salt thereof, wherein:
R¹ is H, halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F;
R² is CN and R³ is H; or R² is H and R³ is CN;
R⁴ is -H, halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F;
R⁵ is -H, halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F;
R⁶ is halo or -C₁₋₆alkyl substituted with 1 to 9 of -F;
R⁷ is -H or -C₁₋₃alkyl;
R⁸ is -H, halo or -C₁₋₃alkyl; and
R9 is -H, pyrazolyl or -C₁₋₆alkyl unsubstituted or substituted with -OH or -OC₁₋₃alkyl.

2. The compound of claim 1, or a pharmaceutically acceptable salt thereof, wherein R⁶ is halo or -C₁₋₃alkyl substituted with 1 to 7 of -F.

3. The compound of claim 2, or a pharmaceutically acceptable salt thereof, wherein R⁷ is H or -CH₃.

4. The compound of claim 3, or a pharmaceutically acceptable salt thereof, wherein R⁸ is -H or halo.

5. The compound of claim 4, or a pharmaceutically acceptable salt thereof, wherein R⁹ is -H, pyrazolyl or -C₁₋₄alkyl unsubstituted or substituted with -OH or -OC₁₋₃alkyl.

6. The compound of any one of claims 1 to 5, or a pharmaceutically acceptable salt thereof, having structural Formula II: 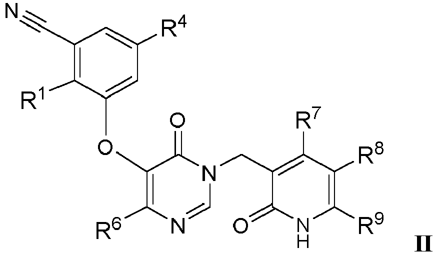 or a pharmaceutically acceptable salt thereof.

7. The compound of claim 6, or a pharmaceutically acceptable salt thereof, wherein R⁴ is halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F.

8. The compound of claim 7, or a pharmaceutically acceptable salt thereof, wherein R⁴ is halo or -C₁₋₃alkyl unsubstituted or substituted with 1 to 7 of F.

9. The compound of claim 7, or a pharmaceutically acceptable salt thereof, wherein R¹ is -H.

10. The compound of claim 7, or a pharmaceutically acceptable salt thereof, wherein R¹ is halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F.

11. The compound of claim 10, or a pharmaceutically acceptable salt thereof, wherein R¹ is halo or -C₁₋₃alkyl unsubstituted or substituted with 1 to 7 of F.

12. The compound of any one of claims 1 to 5, or a pharmaceutically acceptable salt thereof, having structural Formula III: 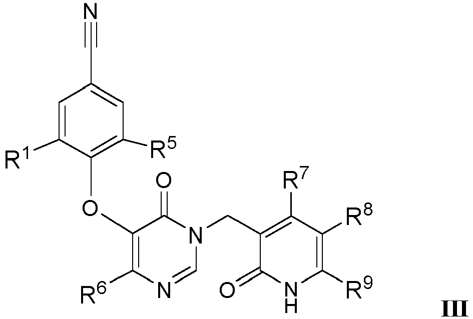 or a pharmaceutically acceptable salt thereof.

13. The compound of claim 12, or a pharmaceutically acceptable salt thereof, wherein R¹ is halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F.

14. The compound of claim 13, or a pharmaceutically acceptable salt thereof, wherein R¹ is halo or -C₁₋₃alkyl unsubstituted or substituted with 1 to 7 of F.

15. The compound of claim 13, or a pharmaceutically acceptable salt thereof, wherein R⁵ is halo, -NH₂, -NH-C₁₋₃alkyl, -N(C₁₋₃alkyl)₂, -C₃₋₆cycloalkyl, -C₁₋₃alkyl unsubstituted or substituted with 1 to 7 of F, or -OC₁₋₃alkyl unsubstituted or substituted with 1 to 7 of F.

16. The compound of claim 15, or a pharmaceutically acceptable salt thereof, wherein R⁵ is halo or -C₁₋₃alkyl unsubstituted or substituted with 1 to 7 of F.

17. The compound of claim 1 that is:
| |
|---|
| 5-chloro-2-fluoro-3-((1-((6-(methoxymethyl)-2-oxo-1,2-dihydropyridin-3-yl)methyl)-6-oxo-4-(1,1,2,2-tetrafluoroethyl)-1,6-dihydropyrimidin-5-yl)oxy)benzonitrile; |
| 5-chloro-3-((4-(1,1-difluoroethyl)-1-((4,6-dimethyl-2-oxo-1,2-dihydropyridin-3-yl)methyl)-6-oxo-1,6-dihydropyrimidin-5-yl)oxy)-2-fluorobenzonitrile; |
| 5-chloro-3-((4-(1,1-difluoroethyl)-1-((6-methyl-2-oxo-1,2-dihydropyridin-3-yl)methyl)-6-oxo-1,6-dihydropyrimidin-5-yl)oxy)-2-fluorobenzonitrile; |
| 5-chloro-2-fluoro-3-((1-((6-methyl-2-oxo-1,2-dihydropyridin-3-yl)methyl)-6-oxo-4-(trifluoromethyl)-1,6-dihydropyrimidin-5-yl)oxy)benzonitrile; |
| 5-(difluoromethyl)-3-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile; |
| 3-chloro-5-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)benzonitrile; |
| 3-bromo-5-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)benzonitrile; |
| 2,5-dichloro-3-((6-oxo-1-((2-oxo-1,2-dihydropyridin-3-yl)methyl)-4-(1,1,2,2-tetrafluoroethyl)-1,6-dihydropyrimidin-5-yl)oxy)benzonitrile; |
| 5-fluoro-2-methyl-3-((1-((6-methyl-2-oxo-1,2-dihydropyridin-3-yl)methyl)-6-oxo-4-(1,1,2,2-tetrafluoroethyl)-1,6-dihydropyrimidin-5-yl)oxy)benzonitrile; |
| 4-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)-3,5-dimethylbenzonitrile; |
| 3-((1-((5-bromo-2-oxo-1,2-dihydropyridin-3-yl)methyl)-6-oxo-4-(1,1,2,2-tetrafluoroethyl)-1,6-dihydropyrimidin-5-yl)oxy)-5-(difluoromethyl)-2-methylbenzonitrile; |
| 5-chloro-3-((4-(1-fluoroethyl)-6-oxo-1-((2-oxo-6-(1H-pyrazol-3-yl)-1,2-dihydropyridin-3-yl)methyl)-1,6-dihydropyrimidin-5-yl)oxy)-2-methylbenzonitrile; |
| 5-chloro-2-methyl-3-((6-oxo-1-((2-oxo-6-(1H-pyrazol-3-yl)-1,2-dihydropyridin-3-yl)methyl)-4-(trifluoromethyl)-1,6-dihydropyrimidin-5-yl)oxy)benzonitrile; |
| 5-(difluoromethyl)-3-((1-((5-fluoro-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile; |
| 3-((4-(1,1-difluoroethyl)-1-((4,6-dimethyl-2-oxo-1,2-dihydropyridin-3-yl)methyl)-6-oxo-1,6-dihydropyrimidin-5-yl)oxy)-5-methylbenzonitrile; |
| 5-(difluoromethyl)-2-methyl-3-((6-oxo-1-((2-oxo-1,2-dihydropyridin-3-yl)methyl)-4-(trifluoromethyl)-1,6-dihydropyrimidin-5-yl)oxy)benzonitrile; |
| 5-(difluoromethyl)-2-methyl-3-((6-oxo-1-((2-oxo-1,2-dihydropyridin-3-yl)methyl)-4-(1,1,2,2-tetrafluoroethyl)-1,6-dihydropyrimidin-5-yl)oxy)benzonitrile; |
| 5-(difluoromethyl)-3-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile; |
| 5-chloro-3-((1-((5-fluoro-2-oxo-1,2-dihydropyridin-3-yl)methyl)-6-oxo-4-(trifluoromethyl)-1,6-dihydropyrimidin-5-yl)oxy)-2-methylbenzonitrile; |
| 5-chloro-2-fluoro-3-((1-((5-fluoro-2-oxo-1,2-dihydropyridin-3-yl)methyl)-6-oxo-4-(perfluoroethyl)-1,6-dihydropyrimidin-5-yl)oxy)benzonitrile; |
| 5-chloro-2-fluoro-3-((1-((6-methyl-2-oxo-1,2-dihydropyridin-3-yl)methyl)-6-oxo-4-(perfluoroethyl)-1,6-dihydropyrimidin-5-yl)oxy)benzonitrile; |
| 5-(difluoromethyl)-2-methyl-3-((1-((6-methyl-2-oxo-1,2-dihydropyridin-3-yl)methyl)-6-oxo-4-(1,1,2,2-tetrafluoroethyl)-1,6-dihydropyrimidin-5-yl)oxy)benzonitrile; |
| 5-(difluoromethyl)-2-methyl-3-((6-oxo-1-((2-oxo-1,2-dihydropyridin-3-yl)methyl)-4-(perfluoroethyl)-1,6-dihydropyrimidin-5-yl)oxy)benzonitrile; |
| 3-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)-2,5-dimethylbenzonitrile; |
| 5-(difluoromethyl)-3-((1-((6-(hydroxymethyl)-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile; |
| 5-(difluoromethyl)-3-((1-((6-(methoxymethyl)-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile; |
| 5-(difluoromethyl)-3-((1-((2-hydroxy-6-(1H-pyrazol-4-yl)pyridin-3-yl)methyl)-6-oxo-4-(1,1,2,2-tetrafluoroethyl)-1,6-dihydropyrimidin-5-yl)oxy)-2-methylbenzonitrile; |
| 5-(difluoromethyl)-3-((1-((6-(3-hydroxypropyl)-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile; |
| 5-(difluoromethyl)-3-((1-((6-(2-hydroxypropan-2-yl)-2-oxo-1,2-dihydropyridin-3-yl)methyl)-6-oxo-4-(1,1,2,2-tetrafluoroethyl)-1,6-dihydropyrimidin-5-yl)oxy)-2-methylbenzonitrile; |
| 3-chloro-5-((6-oxo-1-((2-oxo-6-(1H-pyrazol-4-yl)-1,2-dihydropyridin-3-yl)methyl)-4-(trifluoromethyl)-1,6-dihydropyrimidin-5-yl)oxy)benzonitrile; |
| 5-chloro-2-fluoro-3-((1-((6-(hydroxymethyl)-2-oxo-1,2-dihydropyridin-3-yl)methyl)-6-oxo-4-(1,1,2,2-tetrafluoroethyl)-1,6-dihydropyrimidin-5-yl)oxy)benzonitrile; |
| (*S*) 5-chloro-2-fluoro-3-((4-(1-fluoroethyl)-1-((6-(hydroxymethyl)-2-oxo-1,2-dihydropyridin-3-yl)methyl)-6-oxo-1,6-dihydropyrimidin-5-yl)oxy)benzonitrile; |
| (*R*)-5-chloro-2-fluoro-3-((4-(1-fluoroethyl)-1-((6-(hydroxymethyl)-2-oxo-1,2-dihydropyridin-3-yl)methyl)-6-oxo-1,6-dihydropyrimidin-5-yl)oxy)benzonitrile; |
| 5-chloro-2-fluoro-3-((1-((6-(1-hydroxyethyl)-2-oxo-1,2-dihydropyridin-3-yl)methyl)-6-oxo-4-(1,1,2,2-tetrafluoroethyl)-1,6-dihydropyrimidin-5-yl)oxy)benzonitrile, isomer A; |
| 3-(difluoromethyl)-5-((1-((5-fluoro-2-oxo-1,2-dihydropyridin-3-yl)methyl)-6-oxo-4-(trifluoromethyl)-1,6-dihydropyrimidin-5-yl)oxy)benzonitrile; |
| 2,5-dichloro-3-((1-((5-fluoro-2-oxo-1,2-dihydropyridin-3-yl)methyl)-6-oxo-4-(trifluoromethyl)-1,6-dihydropyrimidin-5-yl)oxy)benzonitrile; |
| 5-chloro-2-fluoro-3-((1-((5-fluoro-2-oxo-1,2-dihydropyridin-3-yl)methyl)-6-oxo-4-(trifluoromethyl)-1,6-dihydropyrimidin-5-yl)oxy)benzonitrile; |
| 5-(difluoromethyl)-3-((1-((5-fluoro-2-oxo-1,2-dihydropyridin-3-yl)methyl)-6-oxo-4-(perfluoroethyl)-1,6-dihydropyrimidin-5-yl)oxy)-2-methylbenzonitrile; |
| 5-chloro-2-fluoro-3-((1-((6-methyl-2-oxo-1,2-dihydropyridin-3-yl)methyl)-6-oxo-4-(1,1,2,2-tetrafluoroethyl)-1,6-dihydropyrimidin-5-yl)oxy)benzonitrile; |
| 3-(difluoromethyl)-5-((1-((4,6-dimethyl-2-oxo-1,2-dihydropyridin-3-yl)methyl)-6-oxo-4-(1,1,2,2-tetrafluoroethyl)-1,6-dihydropyrimidin-5-yl)oxy)benzonitrile; |
| 5-(difluoromethyl)-3-((1-((4,6-dimethyl-2-oxo-1,2-dihydropyridin-3-yl)methyl)-6-oxo-4-(1,1,2-trifluoroethyl)-1,6-dihydropyrimidin-5-yl)oxy)-2-methylbenzonitrile; or |
| 5-(difluoromethyl)-3-((1-((4,6-dimethyl-2-oxo-1,2-dihydropyridin-3-yl)methyl)-6-oxo-4-(trifluoromethyl)-1,6-dihydropyrimidin-5-yl)oxy)-2-methylbenzonitrile; |
or a pharmaceutically acceptable salt thereof.

18. The compound of claims 1 and 17 that is 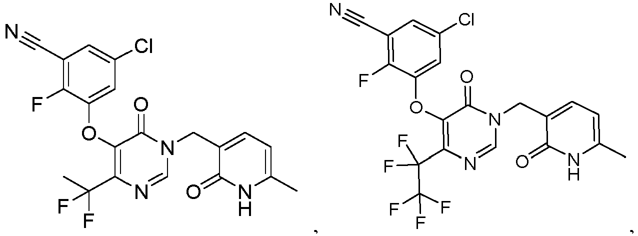 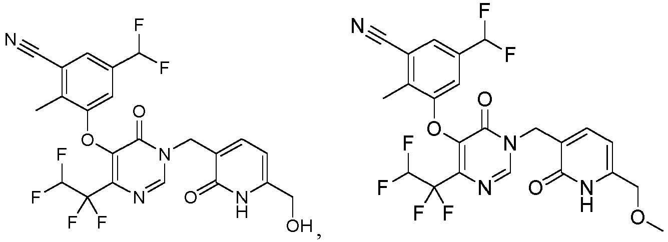 or 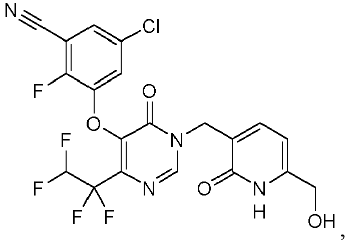 or a pharmaceutically acceptable salt thereof.

19. A pharmaceutical composition comprising an effective amount of the compound of any one of claims 1 to 18, or a pharmaceutically acceptable salt thereof, and a pharmaceutically acceptable carrier.

20. The pharmaceutical composition of claim 19 further comprising an effective amount of one or more additional nucleoside or nucleotide HIV reverse transcriptase inhibitors, nucleoside or nucleotide reverse transcriptase translocation inhibitors, non-nucleoside HIV reverse transcriptase inhibitors, HIV integrase inhibitors, HIV fusion inhibitors, HIV entry inhibitors and HIV maturation inhibitors.

21. A compound according to any one of claims 1 to 18, or a pharmaceutically acceptable salt thereof, for use in a method for treatment of infection by HIV, or for the treatment, prophylaxis or delay in the onset or progression of AIDS or ARC in a human subject.

22. A compound according to any one of claims 1 to 18, or a pharmaceutically acceptable salt thereof, for use in eliciting GAG-POL dimerization in HIV-infected cells in a human subject.

23. A compound according to any one of claims 1 to 18, or a pharmaceutically acceptable salt thereof, for use in selectively killing HIV infected GAG-POL expressing cells without concomitant cytotoxicity to HIV naive cells in a human subject.

24. A compound according to any one of claims 1 to 18, or a pharmaceutically acceptable salt thereof, for use in augmenting the suppression of HIV viremia in a human subject whose viremia is being suppressed by administration of one or more compatible HIV antiviral agents.

25. The compound of any one of claims 1- 18, or a pharmaceutically acceptable salt thereof, for use in therapy.

## Patentansprüche

1. Eine Verbindung der Formel I 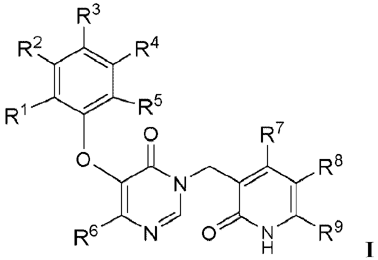 oder ein pharmazeutisch annehmbares Salz davon, wobei:
R¹ H, Halogen, -NH₂, -NH-C₁₋₃-Alkyl, -N(C₁₋₃-Alkyl)₂, -C₃₋₆-Cycloalkyl, -C₁₋₃-Alkyl, unsubstituiert oder substituiert mit 1 bis 7 F, oder -OC₁₋₃-Alkyl, unsubstituiert oder substituiert mit 1 bis 7 F, ist,
R² CN ist und R³ H ist, oder R² H ist und R³ CN ist,
R⁴ -H, Halogen, -NH₂, -NH-C₁₋₃-Alkyl, -N(C₁₋₃-Alkyl)₂, -C₃₋₆-Cyclooalkyl, -C₁₋₃-Alkyl, unsubstituiert oder substituiert mit 1 bis 7 F, oder -OC₁₋₃-Alkyl, unsubstituiert oder substituiert mit 1 bis 7 F, ist,
R⁵ -H, Halogen, -NH₂, -NH-C₁₋₃-Alkyl, -N(C₁₋₃-Alkyl)₂, -C₃₋₆-Cycloalkyl, -C₁₋₃-Alkyl, unsubstituiert oder substituiert mit 1 bis 7 F, oder -OC₁₋₃-Alkyl, unsubstituiert oder substituiert mit 1 bis 7 F, ist,
R⁶ Halogen oder -C₁₋₆-Alkyl, substituiert mit 1 bis 9 -F, ist,
R⁷ -H oder -C₁₋₃-Alkyl ist,
R⁸ -H, Halogen oder -C₁₋₃-Alkyl ist, und
R⁹ -H, Pyrazolyl oder -C₁₋₆-Alkyl, unsubstituiert oder substituiert mit -OH oder -OC₁₋₃-Alkyl, ist.

2. Die Verbindung nach Anspruch 1 oder ein pharmazeutisch annehmbares Salz davon, wobei R⁶ Halogen oder -C₁₋₃-Alkyl, substituiert mit 1 bis 7 -F, ist.

3. Die Verbindung nach Anspruch 2 oder ein pharmazeutisch annehmbares Salz davon, wobei R⁷ H oder -CH₃ ist.

4. Die Verbindung nach Anspruch 3 oder ein pharmazeutisch annehmbares Salz davon, wobei R⁸ -H oder Halogen ist.

5. Die Verbindung nach Anspruch 4 oder ein pharmazeutisch annehmbares Salz davon, wobei R⁹ -H, Pyrazolyl oder -C₁₋₄-Alkyl, unsubstituiert oder substituiert mit -OH oder -OC₁₋₃-Alkyl, ist.

6. Die Verbindung nach einem der Ansprüche 1 bis 5, oder ein pharmazeutisch annehmbares Salz davon, mit der Strukturformel II: 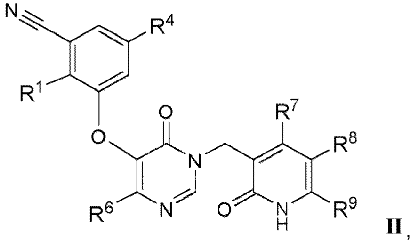 oder ein pharmazeutisch annehmbares Salz davon.

7. Die Verbindung nach Anspruch 6 oder ein pharmazeutisch annehmbares Salz davon, wobei R⁴ Halogen, -NH₂, -NH-C₁₋₃-Alkyl, -N(C₁₋₃-Alkyl)₂, -C₃₋₆-Cycloalkyl, -C₁₋₃-Alkyl, unsubstituiert oder substituiert mit 1 bis 7 F, oder -OC₁₋₃-Alkyl, unsubstituiert oder substituiert mit 1 bis 7 F, ist.

8. Die Verbindung nach Anspruch 7 oder ein pharmazeutisch annehmbares Salz davon, wobei R⁴ Halogen oder -C₁₋₃-Alkyl, unsubstituiert oder substituiert mit 1 bis 7 F, ist.

9. Die Verbindung nach Anspruch 7 oder ein pharmazeutisch annehmbares Salz davon, wobei R¹ -H ist.

10. Die Verbindung nach Anspruch 7 oder ein pharmazeutisch annehmbares Salz davon, wobei R¹ Halogen, -NH₂, -NH-C₁₋₃-Alkyl, -N(C₁₋₃-Alkyl)₂, -C₃₋₆-Cycloalkyl, -C₁₋₃-Alkyl, unsubstituiert oder substituiert mit 1 bis 7 F, oder -OC₁₋₃-Alkyl, unsubstituiert oder substituiert mit 1 bis 7 F, ist.

11. Die Verbindung nach Anspruch 10 oder ein pharmazeutisch annehmbares Salz davon, wobei R¹ Halogen oder -C₁₋₃-Alkyl, unsubstituiert oder substituiert mit 1 bis 7 F, ist.

12. Die Verbindung nach einem der Ansprüche 1 bis 5, oder ein pharmazeutisch annehmbares Salz davon, mit der Strukturformel III: 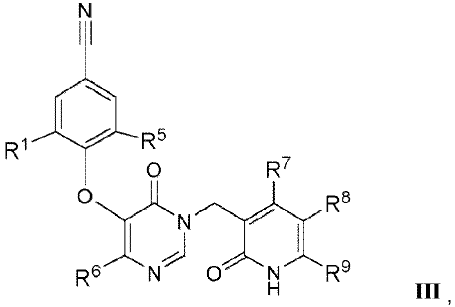 oder ein pharmazeutisch annehmbares Salz davon.

13. Die Verbindung nach Anspruch 12 oder ein pharmazeutisch annehmbares Salz davon, wobei R¹ Halogen, -NH₂, -NH-C₁₋₃-Alkyl, -N(C₁₋₃-Alkyl)₂, -C₃₋₆-Cycloalkyl, -C₁₋₃-Alkyl, unsubstituiert oder substituiert mit 1 bis 7 F, oder -OC₁₋₃-Alkyl, unsubstituiert oder substituiert mit 1 bis 7 F, ist.

14. Die Verbindung nach Anspruch 13 oder ein pharmazeutisch annehmbares Salz davon, wobei R¹ Halogen oder -C₁₋₃-Alkyl, unsubstituiert oder substituiert mit 1 bis 7 F, ist.

15. Die Verbindung nach Anspruch 13 oder ein pharmazeutisch annehmbares Salz davon, wobei R⁵ Halogen, -NH₂, -NH-C₁₋₃-Alkyl, -N(C₁₋₃-Alkyl)₂, -C₃₋₆-Cycloalkyl, -C₁₋₃-Alkyl, unsubstituiert oder substituiert mit 1 bis 7 F, oder -OC₁₋₃-Alkyl, unsubstituiert oder substituiert mit 1 bis 7 F, ist.

16. Die Verbindung nach Anspruch 15 oder ein pharmazeutisch annehmbares Salz davon, wobei R⁵ Halogen oder -C₁₋₃-Alkyl, unsubstituiert oder substituiert mit 1 bis 7 F, ist.

17. Die Verbindung nach Anspruch 1, die ist:
| |
|---|
| 5-Chlor-2-fluor-3-((1-((6-(methoxymethyl)-2-oxo-1,2-dihydropyridin-3-yl)methyl)-6-oxo-4-(1,1,2,2-tetrafluoroethyl)-1,6-dihydropyrimidin-5-yl)oxy)benzonitril, |
| 5-Chlor-3-((4-(1,1-difluorethyl)-1-((4,6-dimethyl-2-oxo-1,2-dihydropyridin-3-yl)methyl)-6-oxo-1,6-dihydropyrimidin-5-yl)oxy)-2-fluorbenzonitril, |
| 5-Chlor-3-((4-(1,1-difluorethyl)-1-((6-methyl-2-oxo-1,2-dihydropyridin-3-yl)methyl)-6-oxo-1,6-dihydropyrimidin-5-yl)oxy)-2-fluorbenzonitril, |
| 5-Chlor-2-fluor-3-((1-((6-methyl-2-oxo-1,2-dihydropyridin-3-yl)methyl)-6-oxo-4-(trifluormethyl)-1,6-dihydropyrimidin-5-yl)oxy)benzonitril, |
| 5-(Difluormethyl)-3-((1-((4,6-dimethyl-2-oxo-1,2-dihydropyridin-3-yl)methyl)-6-oxo-4-(1,1,2,2-tetrafluorethyl)-1,6-dihydropyrimidin-5-yl)oxy)-2-methylbenzonitril, |
| 3-Chlor-5-((1-((4,6-dimethyl-2-oxo-1,2-dihydropyridin-3-yl)methyl)-6-oxo-4-(1,1,2,2-tetrafluorethyl)-1,6-dihydropyrimidin-5-yl)oxy)benzonitril, |
| 3-Brom-5-((1-((4,6-dimethyl-2-oxo-1,2-dihydropyridin-3-yl)methyl)-6-oxo-4-(1,1,2,2-tetrafluorethyl)-1,6-dihydropyrimidin-5-yl)oxy)benzonitril, |
| 2,5-Dichlor-3-((6-oxo-1-((2-oxo-1,2-dihydropyridin-3-yl)methyl)-4-(1,1,2,2-tetrafluorethyl)-1,6-dihydropyrimidin-5-yl)oxy)benzonitril, |
| 5-Fluor-2-methyl-3-((1-((6-methyl-2-oxo-1,2-dihydropyridin-3-yl)methyl)-6-oxo-4-(1,1,2,2-tetrafluorethyl)-1,6-dihydropyrimidin-5-yl)oxy)benzonitril, |
| 4-((1-((4,6-Dimethyl-2-oxo-1,2-dihydropyridin-3-yl)methyl)-6-oxo-4-(1,1,2,2-tetrafluorethyl)-1,6-dihydropyrimidin-5-yl)oxy)-3,5-dimethylbenzonitril, |
| 3-((1-((5-Brom-2-oxo-1,2-dihydropyridin-3-yl)methyl)-6-oxo-4-(1,1,2,2-tetrafluorethyl)-1,6-dihydropyrimidin-5-yl)oxy)-5-(difluormethyl)-2-methylbenzonitril, |
| 5-Chlor-3-((4-(1-fluorethyl)-6-oxo-1-((2-oxo-6-(1H-pyrazol-3-yl)-1,2-dihydropyridin-3-yl)methyl)-1,6-dihydropyrimidin-5-yl)oxy)-2-methylbenzonitril, |
| 5-Chlor-2-methyl-3-((6-oxo-1-((2-oxo-6-(1H-pyrazol-3-yl)-1,2-dihydropyridin-3-yl)methyl)-4-(trifluormethyl)-1,6-dihydropyrimidin-5-yl)oxy)benzonitril, |
| 5-(Difluormethyl)-3-((1-((5-fluor-2-oxo-1,2-dihydropyridin-3-yl)methyl)-6-oxo-4-(1,1,2,2-tetrafluorethyl)-1,6-dihydropyrimidin-5-yl)oxy)-2-methylbenzonitril, |
| 3-((4-(1,1-Difluorethyl)-1-((4,6-dimethyl-2-oxo-1,2-dihydropyridin-3-yl)methyl)-6-oxo-1,6-dihydropyrimidin-5-yl)oxy)-5-methylbenzonitril, |
| 5-(Difluormethyl)-2-methyl-3-((6-oxo-1-((2-oxo-1,2-dihydropyridin-3-yl)methyl)-4-(trifluormethyl)-1,6-dihydropyrimidin-5-yl)oxy)benzonitril, |
| 5-(Difluormethyl)-2-methyl-3-((6-oxo-1-((2-oxo-1,2-dihydropyridin-3-yl)methyl)-4-(1,1,2,2-tetrafluorethyl)-1,6-dihydropyrimidin-5-yl)oxy)benzonitril, |
| 5-(Difluormethyl)-3-((1-((4,6-dimethyl-2-oxo-1,2-dihydropyridin-3-yl)methyl)-6-oxo-4-(1,1,2,2-tetrafluorethyl)-1,6-dihydropyrimidin-5-yl)oxy)-2-methylbenzonitril, |
| 5-Chlor-3-((1-((5-fluor-2-oxo-1,2-dihydropyridin-3-yl)methyl)-6-oxo-4-(trifluormethyl)-1,6-dihydropyrimidin-5-yl)oxy)-2-methylbenzonitril, |
| 5-Chlor-2-fluor-3-((1-((5-fluor-2-oxo-1,2-dihydropyridin-3-yl)methyl)-6-oxo-4-(perfluorethyl)-1,6-dihydropyrimidin-5-yl)oxy)benzonitril, |
| 5-Chlor-2-fluor-3-((1-((6-methyl-2-oxo-1,2-dihydropyridin-3-yl)methyl)-6-oxo-4-(perfluorethyl)-1,6-dihydropyrimidin-5-yl)oxy)benzonitril, |
| 5-(Difluormethyl)-2-methyl-3-((1-((6-methyl-2-oxo-1,2-dihydropyridin-3-yl)methyl)-6-oxo-4-(1,1,2,2-tetrafluorethyl)-1,6-dihydropyrimidin-5-yl)oxy)benzonitril, |
| 5-(Difluormethyl)-2-methyl-3-((6-oxo-1-((2-oxo-1,2-dihydropyridin-3-yl)methyl)-4-(perfluorethyl)-1,6-dihydropyrimidin-5-yl)oxy)benzonitril, |
| 3-((1-((4,6-Dimethyl-2-oxo-1,2-dihydropyridin-3-yl)methyl)-6-oxo-4-(1,1,2,2-tetrafluorethyl)-1,6-dihydropyrimidin-5-yl)oxy)-2,5-dimethylbenzonitril, |
| 5-(Difluormethyl)-3-((1-((6-(hydroxymethyl)-2-oxo-1,2-dihydropyridin-3-yl)methyl)-6-oxo-4-(1,1,2,2-tetrafluorethyl)-1,6-dihydropyrimidin-5-yl)oxy)-2-methylbenzonitril, |
| 5-(Difluormethyl)-3-((1-((6-(methoxymethyl)-2-oxo-1,2-dihydropyridin-3-yl)methyl)-6-oxo-4-(1,1,2,2-tetrafluorethyl)-1,6-dihydropyrimidin-5-yl)oxy)-2-methylbenzonitril, |
| 5-(Difluormethyl)-3-((1-((2-hydroxy-6-(1H-pyrazol-4-yl)pyridin-3-yl)methyl)-6-oxo-4-(1,1,2,2-tetrafluorethyl)-1,6-dihydropyrimidin-5-yl)oxy)-2-methylbenzonitril, |
| 5-(Difluormethyl)-3-((1-((6-(3-hydroxypropyl)-2-oxo-1,2-dihydropyridin-3-yl)methyl)-6-oxo-4-(1,1,2,2-tetrafluorethyl)-1,6-dihydropyrimidin-5-yl)oxy)-2-methylbenzonitril, |
| 5-(Difluormethyl)-3-((1-((6-(2-hydroxypropan-2-yl)-2-oxo-1,2-dihydropyridin-3-yl)methyl)-6-oxo-4-(1,1,2,2-tetrafluorethyl)-1,6-dihydropyrimidin-5-yl)oxy)-2-methylbenzonitril, |
| 3-Chlor-5-((6-oxo-1-((2-oxo-6-(1H-pyrazol-4-yl)-1,2-dihydropyridin-3-yl)methyl)-4-(trifluormethyl)-1,6-dihydropyrimidin-5-yl)oxy)benzonitril, |
| 5-Chlor-2-fluor-3-((1-((6-(hydroxymethyl)-2-oxo-1,2-dihydropyridin-3-yl)methyl)-6-oxo-4-(1,1,2,2-tetrafluorethyl)-1,6-dihydropyrimidin-5-yl)oxy)benzonitril, |
| (*S*)-5-Chlor-2-fluor-3-((4-(1-fluorethyl)-1-((6-(hydroxymethyl)-2-oxo-1,2-dihydropyridin-3-yl)methyl)-6-oxo-1,6-dihydropyrimidin-5-yl)oxy)benzonitril, |
| (*R*)-5-Chlor-2-fluor-3-((4-(1-fluorethyl)-1-((6-(hydroxymethyl)-2-oxo-1,2-dihydropyridin-3-yl)methyl)-6-oxo-1,6-dihydropyrimidin-5-yl)oxy)benzonitril, |
| 5-Chlor-2-fluor-3-((1-((6-(1-hydroxyethyl)-2-oxo-1,2-dihydropyridin-3-yl)methyl)-6-oxo-4-(1,1,2,2-tetrafluorethyl)-1,6-dihydropyrimidin-5-yl)oxy)benzonitril, Isomer A |
| 3-(Difluormethyl)-5-((1-((5-fluor-2-oxo-1,2-dihydropyridin-3-yl)methyl)-6-oxo-4-(trifluormethyl)-1,6-dihydropyrimidin-5-yl)oxy)benzonitril, |
| 2,5-Dichlor-3-((1-((5-fluor-2-oxo-1,2-dihydropyridin-3-yl)methyl)-6-oxo-4-(trifluormethyl)-1,6-dihydropyrimidin-5-yl)oxy)benzonitril, |
| 5-Chlor-2-fluor-3-((1-((5-fluor-2-oxo-1,2-dihydropyridin-3-yl)methyl)-6-oxo-4-(trifluormethyl)-1,6-dihydropyrimidin-5-yl)oxy)benzonitril, |
| 5-(Difluormethyl)-3-((1-((5-fluor-2-oxo-1,2-dihydropyridin-3-yl)methyl)-6-oxo-4-(perfluorethyl)-1,6-dihydropyrimidin-5-yl)oxy)-2-methylbenzonitril, |
| 5-Chlor-2-fluor-3-((1-((6-methyl-2-oxo-1,2-dihydropyridin-3-yl)methyl)-6-oxo-4-(1,1,2,2-tetrafluorethyl)-1,6-dihydropyrimidin-5-yl)oxy)benzonitril, |
| 3-(Difluormethyl)-5-((1-((4,6-dimethyl-2-oxo-1,2-dihydropyridin-3-yl)methyl)-6-oxo-4-(1,1,2,2-tetrafluorethyl)-1,6-dihydropyrimidin-5-yl)oxy)benzonitril, |
| 5-(Difluormethyl)-3-((1-((4,6-dimethyl-2-oxo-1,2-dihydropyridin-3-yl)methyl)-6-oxo-4-(1,1,2-trifluorethyl)-1,6-dihydropyrimidin-5-yl)oxy)-2-methylbenzonitril oder |
| 5-(Difluormethyl)-3-((1-((4,6-dimethyl-2-oxo-1,2-dihydropyridin-3-yl)methyl)-6-oxo-4-(trifluormethyl)-1,6-dihydropyrimidin-5-yl)oxy)-2-methylbenzonitril, |
oder ein pharmazeutisch annehmbares Salz davon.

18. Die Verbindung nach den Ansprüchen 1 und 17, die 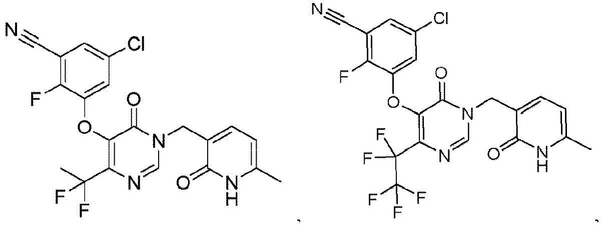 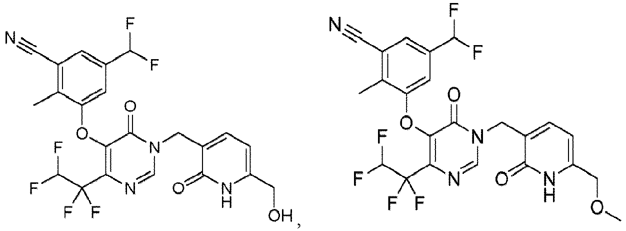 oder 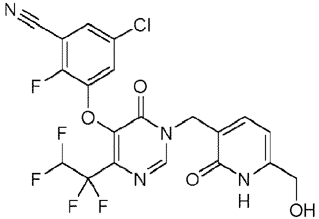 ist, oder ein pharmazeutisch annehmbares Salz davon.

19. Eine pharmazeutische Zusammensetzung, die eine wirksame Menge der Verbindung nach einem der Ansprüche 1 bis 18 oder eines pharmazeutisch annehmbaren Salzes davon und einen pharmazeutisch annehmbaren Träger umfasst.

20. Die pharmazeutische Zusammensetzung nach Anspruch 19, die ferner eine wirksame Menge von einem oder mehreren zusätzlichen nukleosidischen oder nukleotidischen HIV-Reverse-Transkriptase-Inhibitoren, nukleosidischen oder nukleotidischen Reverse-Transkriptase-Translokations-Inhibitoren, nichtnukleosidischen HIV-Reverse-Transkriptase-Inhibitoren, HIV-Integrase-Inhibitoren, HIV-Fusionsinhibitoren, HIV-Entry-Inhibitoren und HIV-Maturationsinhibitoren umfasst.

21. Eine Verbindung gemäß einem der Ansprüche 1 bis 18 oder ein pharmazeutisch annehmbares Salz davon zur Verwendung bei einem Verfahren zur Behandlung einer HIV-Infektion oder zur Behandlung, Prophylaxe oder Verzögerung des Ausbruchs oder Fortschreitens von AIDS oder ARC bei einem menschlichen Subjekt.

22. Eine Verbindung gemäß einem der Ansprüche 1 bis 18 oder ein pharmazeutisch annehmbares Salz davon zur Verwendung zur Herbeiführung einer GAG-POL-Dimerisierung in HIV-infizierten Zellen bei einem menschlichen Subjekt.

23. Eine Verbindung gemäß einem der Ansprüche 1 bis 18 oder ein pharmazeutisch annehmbares Salz davon zur Verwendung zur selektiven Abtötung von HIV-infizierten GAG-POL-exprimierenden Zellen ohne einhergehende Zytotoxizität für HIV-naive Zellen bei einem menschlichen Subjekt.

24. Eine Verbindung gemäß einem der Ansprüche 1 bis 18 oder ein pharmazeutisch annehmbares Salz davon zur Verwendung zur Steigerung der Suppression von HIV-Virämie bei einem menschlichen Subjekt, dessen Virämie durch Verabreichung von einem oder mehreren kompatiblen antiviralen Mitteln gegen HIV supprimiert wird.

25. Die Verbindung nach einem der Ansprüche 1-18 oder ein pharmazeutisch annehmbares Salz davon zur Verwendung in der Therapie.

## Revendications

1. Composé présentant la Formule 1 : 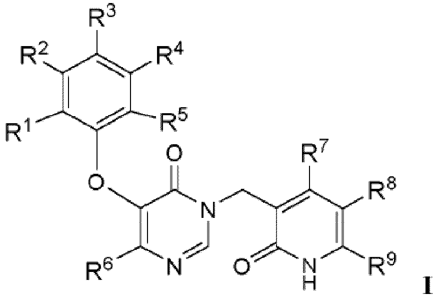 ou un sel pharmaceutiquement acceptable de celui-ci, dans lequel :
R¹ est H, un halo, -NH₂, un -NH-C₁₋₃ alkyle, un -N(C₁₋₃ alkyle)₂, un -C₃₋₆ cycloalkyle, un -C₁₋₃ alkyle non substitué ou substitué par 1 à 7 F ou un -OC₁₋₃ alkyle non substitué ou substitué par 1 à 7 F ;
R² est CN et R³ est H ; ou R² est H et R³ est CN ;
R⁴ est -H, un halo, -NH₂, un -NH-C₁₋₃ alkyle, un -N(C₁₋₃ alkyle)₂, un -C₃₋₆ cycloalkyle, un -C₁₋₃ alkyle non substitué ou substitué par 1 à 7 F ou un -OC₁₋₃ alkyle non substitué ou substitué par 1 à 7 F ;
R⁵ est -H, un halo, -NH₂, un -NH-C₁₋₃ alkyle, un -N(C₁₋₃ alkyle)₂, un -C₃₋₆ cycloalkyle, un -C₁₋₃ alkyle non substitué ou substitué par 1 à 7 F ou un -OC₁₋₃ alkyle non substitué ou substitué par 1 à 7 F ;
R⁶ est un halo ou un -C₁₋₆ alkyle substitué par 1 à 9 -F ;
R⁷ est -H ou un -C₁₋₃ alkyle ;
R⁸ est -H, un halo ou un -C₁₋₃ alkyle ; et
R⁹ est -H, un pyrazolyle ou un -C₁₋₆ alkyle non substitué ou substitué par -OH ou un - OC₁₋₃ alkyle.

2. Composé selon la revendication 1, ou un sel pharmaceutiquement acceptable de celui-ci, dans lequel R⁶ est un halo ou un -C₁₋₃ alkyle substitué par 1 à 7 -F.

3. Composé selon la revendication 2, ou un sel pharmaceutiquement acceptable de celui-ci, dans lequel R⁷ est -H ou -CH₃.

4. Composé selon la revendication 3, ou un sel pharmaceutiquement acceptable de celui-ci, dans lequel R⁸ est -H ou un halo.

5. Composé selon la revendication 4, ou un sel pharmaceutiquement acceptable de celui-ci, dans lequel R⁹ est -H, un pyrazolyle ou un -C₁₋₄ alkyle non substitué ou substitué par -OH ou un -OC₁₋₃ alkyle.

6. Composé selon l'une quelconque des revendications 1 à 5, ou un sel pharmaceutiquement acceptable de celui-ci, présentant la Formule structurale II : 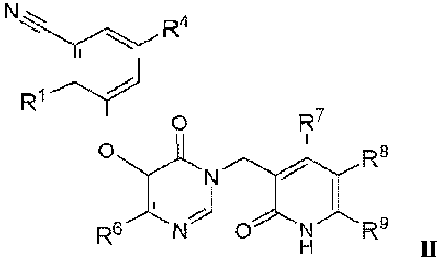 ou un sel pharmaceutiquement acceptable de celui-ci.

7. Composé selon la revendication 6, ou un sel pharmaceutiquement acceptable de celui-ci, dans lequel R⁴ est un halo, -NH₂, un -NH-C₁₋₃ alkyle, un -N(C₁₋₃ alkyle)₂, un -C₃₋₆ cycloalkyle, un -C₁₋₃ alkyle non substitué ou substitué par 1 à 7 F ou un -OC₁₋₃ alkyle non substitué ou substitué par 1 à 7 F.

8. Composé selon la revendication 7, ou un sel pharmaceutiquement acceptable de celui-ci, dans lequel R⁴ est un halo ou un -C₁₋₃ alkyle non substitué ou substitué par 1 à 7 F.

9. Composé selon la revendication 7, ou un sel pharmaceutiquement acceptable de celui-ci, dans lequel R¹ est -H.

10. Composé selon la revendication 7, ou un sel pharmaceutiquement acceptable de celui-ci, dans lequel R¹ est un halo, -NH₂, un -NH-C₁₋₃ alkyle, un -N(C₁₋₃ alkyle)₂, un -C₃₋₆ cycloalkyle, un -C₁₋₃ alkyle non substitué ou substitué par 1 à 7 F ou un -OC₁₋₃ alkyle non substitué ou substitué par 1 à 7 F.

11. Composé selon la revendication 10, ou un sel pharmaceutiquement acceptable de celui-ci, dans lequel R¹ est un halo ou un -C₁₋₃ alkyle non substitué ou substitué par 1 à 7 F.

12. Composé selon l'une quelconque des revendications 1 à 5, ou un sel pharmaceutiquement acceptable de celui-ci, présentant la Formule structurale III : 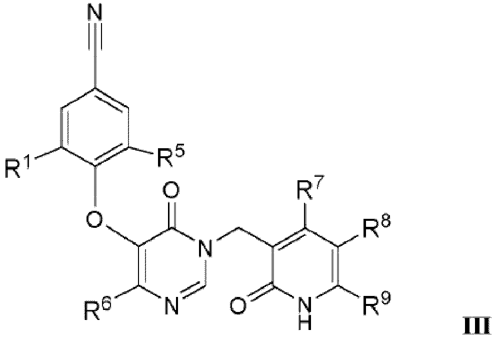 ou un sel pharmaceutiquement acceptable de celui-ci.

13. Composé selon la revendication 12, ou un sel pharmaceutiquement acceptable de celui-ci, dans lequel R¹ est un halo, -NH₂, un -NH-C₁₋₃ alkyle, un -N(C₁₋₃ alkyle)₂, un -C₃₋₆ cycloalkyle, un -C₁₋₃ alkyle non substitué ou substitué par 1 à 7 F ou un -OC₁₋₃ alkyle non substitué ou substitué par 1 à 7 F.

14. Composé selon la revendication 13, ou un sel pharmaceutiquement acceptable de celui-ci, dans lequel R¹ est un halo ou un -C₁₋₃ alkyle non substitué ou substitué par 1 à 7 F.

15. Composé selon la revendication 13, ou un sel pharmaceutiquement acceptable de celui-ci, dans lequel R⁵ est un halo, -NH₂, un -NH-C₁₋₃ alkyle, un -N(C₁₋₃ alkyle)₂, un -C₃₋₆ cycloalkyle, un -C₁₋₃ alkyle non substitué ou substitué par 1 à 7 F ou un -OC₁₋₃ alkyle non substitué ou substitué par 1 à 7 F.

16. Composé selon la revendication 15, ou un sel pharmaceutiquement acceptable de celui-ci, dans lequel R⁵ est un halo ou un -C₁₋₃ alkyle non substitué ou substitué par 1 à 7 F.

17. Composé selon la revendication 1 qui est :
5-chloro-2-fluoro-3-((1-((6-(méthoxyméthyl)-2-oxo-1,2-dihydropyridin-3-yl)méthyl)-6-oxo-4-(1,1,2,2-tétrafluoroéthyl)-1,6-dihydropyrimidin-5-yl)oxy)benzonitrile ;
5-chloro-3-((4-(1,1-difluoroéthyl)-1-((4,6-diméthyl-2-oxo-1,2-dihydropyridin-3-yl)méthyl)-6-oxo-1,6-dihydropyrimidin-5-yl)oxy)-2-fluorobenzonitrile ;
5-chloro-3-((4-(1,1-difluoroéthyl)-1-((6-méthyl-2-oxo-1,2-dihydropyridin-3-yl)méthyl)-6-oxo-1,6-dihydropyrimidin-5-yl)oxy)-2-fluorobenzonitrile ;
5-chloro-2-fluoro-3-((1-((6-méthyl-2-oxo-1,2-dihydropyridin-3-yl)méthyl)-6-oxo-4-(trifluorométhyl)-1,6-dihydropyrimidin-5-yl)oxy)benzonitrile ;
5-(difluorométhyl)-3-((1-((4,6-diméthyl-2-oxo-1,2-dihydropyridin-3-yl)méthyl)-6-oxo-4-(1,1,2,2-tétrafluoroéthyl)-1,6-dihydropyrimidin-5-yl)oxy)-2-méthylbenzonitrile ;
3-chloro-5-((1-((4,6-diméthyl-2-oxo-1,2-dihydropyridin-3-yl)méthyl)-6-oxo-4-(1,1,2,2-tétrafluoroéthyl)-1,6-dihydropyrimidin-5-yl)oxy)benzonitrile ;
3-bromo-5-((1-((4,6-diméthyl-2-oxo-1,2-dihydropyridin-3-yl)méthyl)-6-oxo-4-(1,1,2,2-tétrafluoroéthyl)-1,6-dihydropyrimidin-5-yl)oxy)benzonitrile ;
2,5-dichloro-3-((6-oxo-1-((2-oxo-1,2-dihydropyridin-3-yl)méthyl)-4-(1,1,2,2-tétrafluoroéthyl)-1,6-dihydropyrimidin-5-yl)oxy)benzonitrile ;
5-fluoro-2-méthyl-3-((1-((6-méthyl-2-oxo-1,2-dihydropyridin-3-yl)méthyl)-6-oxo-4-(1,1,2,2-tétrafluoroéthyl)-1,6-dihydropyrimidin-5-yl)oxy)benzonitrile ;
4-((1-((4,6-diméthyl-2-oxo-1,2-dihydropyridin-3-yl)méthyl)-6-oxo-4-(1,1,2,2-tétrafluoroéthyl)-1,6-dihydropyrimidin-5-yl)oxy)-3,5-diméthylbenzonitrile ;
3-((1-((5-bromo-2-oxo-1,2-dihydropyridin-3-yl)méthyl)-6-oxo-4-(1,1,2,2-tétrafluoroéthyl)-1,6-dihydropyrimidin-5-yl)oxy)-5-(difluorométhyl)-2-méthylbenzonitrile ;
5-chloro-3-((4-(1-fluoroéthyl)-6-oxo-1-((2-oxo-6-(1H-pyrazol-3-yl)-1,2-dihydropyridin-3-yl)méthyl)-1,6-dihydropyrimidin-5-yl)oxy)-2-méthylbenzonitrile ;
5-chloro-2-méthyl-3-((6-oxo-1-((2-oxo-6-(1H-pyrazol-3-yl)-1,2-dihydropyridin-3-yl)méthyl)-4-(trifluorométhyl)-1,6-dihydropyrimidin-5-yl)oxy)benzonitrile ;
5-(difluorométhyl)-3-((1-((5-fluoro-2-oxo-1,2-dihydropyridin-3-yl)méthyl)-6-oxo-4-(1,1,2,2-tétrafluoroéthyl)-1,6-dihydropyrimidin-5-yl)oxy)-2-méthylbenzonitrile ;
3-((4-(1,1-difluoroéthyl)-1-((4,6-diméthyl-2-oxo-1,2-dihydropyridin-3-yl)méthyl)-6-oxo-1,6-dihydropyrimidin-5-yl)oxy)-5-méthylbenzonitrile ;
5-(difluorométhyl)-2-méthyl-3-((6-oxo-1-((2-oxo-1,2-dihydropyridin-3-yl)méthyl)-4-(trifluorométhyl)-1,6-dihydropyrimidin-5-yl)oxy)benzonitrile ;
5-(difluorométhyl)-2-méthyl-3-((6-oxo-1-((2-oxo-1,2-dihydropyridin-3-yl)méthyl)-4-(1,1,2,2-tétrafluoroéthyl)-1,6-dihydropyrimidin-5-yl)oxy)benzonitrile ;
5-(difluorométhyl)-3-((1-((4,6-diméthyl-2-oxo-1,2-dihydropyridin-3-yl)méthyl)-6-oxo-4-(1,1,2,2-tétrafluoroéthyl)-1,6-dihydropyrimidin-5-yl)oxy)-2-méthylbenzonitrile ;
5-chloro-3-((1-((5-fluoro-2-oxo-1,2-dihydropyridin-3-yl)méthyl)-6-oxo-4-(trifluorométhyl)-1,6-dihydropyrimidin-5-yl)oxy)-2-méthylbenzonitrile ;
5-chloro-2-fluoro-3-((1-((5-fluoro-2-oxo-1,2-dihydropyridin-3-yl)méthyl)-6-oxo-4-(perfluoroéthyl)-1,6-dihydropyrimidin-5-yl)oxy)benzonitrile ;
5-chloro-2-fluoro-3-((1-((6-méthyl-2-oxo-1,2-dihydropyridin-3-yl)méthyl)-6-oxo-4-(perfluoroéthyl)-1,6-dihydropyrimidin-5-yl)oxy)benzonitrile ;
5-(difluorométhyl)-2-méthyl-3-((1-((6-méthyl-2-oxo-1,2-dihydropyridin-3-yl)méthyl)-6-oxo-4-(1,1,2,2-tétrafluoroéthyl)-1,6-dihydropyrimidin-5-yl)oxy)benzonitrile ;
5-(difluorométhyl)-2-méthyl-3-((6-oxo-1-((2-oxo-1,2-dihydropyridin-3-yl)méthyl)-4-(perfluoroéthyl)-1,6-dihydropyrimidin-5-yl)oxy)benzonitrile ;
3-((1-((4,6-diméthyl-2-oxo-1,2-dihydropyridin-3-yl)méthyl)-6-oxo-4-(1,1,2,2-tétrafluoroéthyl)-1,6-dihydropyrimidin-5-yl)oxy)-2,5-diméthylbenzonitrile ;
5-(difluorométhyl)-3-((1-((6-(hydroxyméthyl)-2-oxo-1,2-dihydropyridin-3-yl)méthyl)-6-oxo-4-(1,1,2,2-tétrafluoroéthyl)-1,6-dihydropyrimidin-5-yl)oxy)-2-méthylbenzonitrile ;
5-(difluorométhyl)-3-((1-((6-(méthoxyméthyl)-2-oxo-1,2-dihydropyridin-3-yl)méthyl)-6-oxo-4-(1,1,2,2-tétrafluoroéthyl)-1,6-dihydropyrimidin-5-yl)oxy)-2-méthylbenzonitrile ;
5-(difluorométhyl)-3-((1-((2-hydroxy-6-(1H-pyrazol-4-yl)pyridin-3-yl)méthyl)-6-oxo-4-(1,1,2,2-tétrafluoroéthyl)-1,6-dihydropyrimidin-5-yl)oxy)-2-méthylbenzonitrile ;
5-(difluorométhyl)-3-((1-((6-(3-hydroxypropyl)-2-oxo-1,2-dihydropyridin-3-yl)méthyl)-6-oxo-4-(1,1,2,2-tétrafluoroéthyl)-1,6-dihydropyrimidin-5-yl)oxy)-2-méthylbenzonitrile ;
5-(difluorométhyl)-3-((1-((6-(2-hydroxypropan-2-yl)-2-oxo-1,2-dihydropyridin-3-yl)méthyl)-6-oxo-4-(1,1,2,2-tétrafluoroéthyl)-1,6-dihydropyrimidin-5-yl)oxy)-2-méthylbenzonitrile ;
3-chloro-5-((6-oxo-1-((2-oxo-6-(1H-pyrazol-4-yl)-1,2-dihydropyridin-3-yl)méthyl)-4-(trifluorométhyl)-1,6-dihydropyrimidin-5-yl)oxy)benzonitrile ;
5-chloro-2-fluoro-3-((1-((6-(hydroxyméthyl)-2-oxo-1,2-dihydropyridin-3-yl)méthyl)-6-oxo-4-(1,1,2,2-tétrafluoroéthyl)-1,6-dihydropyrimidin-5-yl)oxy)benzonitrile ;
(S)-5-chloro-2-fluoro-3-((4-(1-fluoroéthyl)-1-((6-(hydroxyméthyl)-2-oxo-1,2-dihydropyridin-3-yl)méthyl)-6-oxo-1,6-dihydropyrimidin-5-yl)oxy)benzonitrile ;
(R)-5-chloro-2-fluoro-3-((4-(1-fluoroéthyl)-1-((6-(hydroxyméthyl)-2-oxo-1,2-dihydropyridin-3-yl)méthyl)-6-oxo-1,6-dihydropyrimidin-5-yl)oxy)benzonitrile ;
5-chloro-2-fluoro-3-((1-((6-(1-hydroxyéthyl)-2-oxo-1,2-dihydropyridin-3-yl)méthyl)-6-oxo-4-(1,1,2,2-tétrafluoroéthyl)-1,6-dihydropyrimidin-5-yl)oxy)benzonitrile, isomère A ;
3-(difluorométhyl)-5-((1-((5-fluoro-2-oxo-1,2-dihydropyridin-3-yl)méthyl)-6-oxo-4-(trifluorométhyl)-1,6-dihydropyrimidin-5-yl)oxy)benzonitrile ;
2,5-dichloro-3-((1-((5-fluoro-2-oxo-1,2-dihydropyridin-3-yl)méthyl)-6-oxo-4-(trifluorométhyl)-1,6-dihydropyrimidin-5-yl)oxy)benzonitrile ;
5-chloro-2-fluoro-3-((1-((5-fluoro-2-oxo-1,2-dihydropyridin-3-yl)méthyl)-6-oxo-4-(trifluorométhyl)-1,6-dihydropyrimidin-5-yl)oxy)benzonitrile ;
5-(difluorométhyl)-3-((1-((5-fluoro-2-oxo-1,2-dihydropyridin-3-yl)méthyl)-6-oxo-4-(perfluoroéthyl)-1,6-dihydropyrimidin-5-yl)oxy)-2-méthylbenzonitrile ;
5-chloro-2-fluoro-3-((1-((6-méthyl-2-oxo-1,2-dihydropyridin-3-yl)méthyl)-6-oxo-4-(1,1,2,2-tétrafluoroéthy1)-1,6-dihydropyrimidin-5-yl)oxy)benzonitrile ;
3-(difluorométhyl)-5-((1-((4,6-diméthyl-2-oxo-1,2-dihydropyridin-3-yl)méthyl)-6-oxo-4-(1,1,2,2-tétrafluoroéthyl)-1,6-dihydropyrimidin-5-yl)oxy)benzonitrile ;
5-(difluorométhyl)-3-((1-((4,6-diméthyl-2-oxo-1,2-dihydropyridin-3-yl)méthyl)-6-oxo-4-(1,1,2-trifluoroéthyl)-1,6-dihydropyrimidin-5-yl)oxy)-2-méthylbenzonitrile ; ou
5-(difluorométhyl)-3-((1-((4,6-diméthyl-2-oxo-1,2-dihydropyridin-3-yl)méthyl)-6-oxo-4-(trifluorométhyl)-1,6-dihydropyrimidin-5-yl)oxy)-2-méthylbenzonitrile ;
ou un sel pharmaceutiquement acceptable de ceux-ci.

18. Composé selon les revendications 1 et 17 qui est : 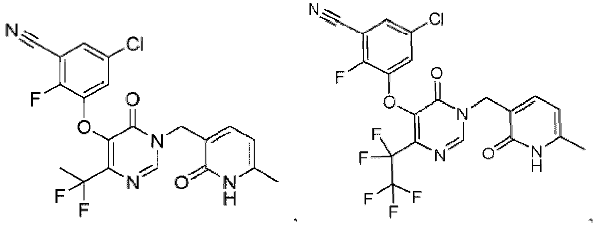 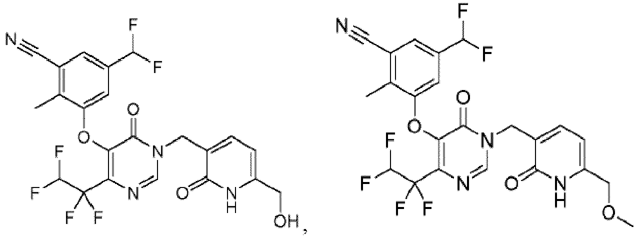 ou 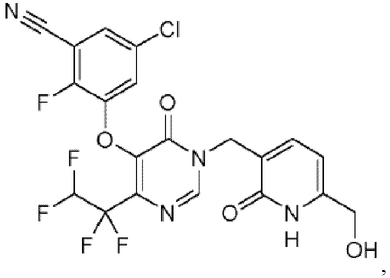 ou un sel pharmaceutiquement acceptable de ceux-ci.

19. Composition pharmaceutique comprenant une quantité efficace du composé selon l'une quelconque des revendications 1 à 18, ou d'un sel pharmaceutiquement acceptable de celui-ci, et un véhicule pharmaceutiquement acceptable.

20. Composition pharmaceutique selon la revendication 19, comprenant en outre une quantité efficace d'un ou de plusieurs inhibiteurs de transcriptase inverse du VIH nucléosidiques ou nucléotidiques supplémentaires, inhibiteurs de translocation de transcriptase inverse nucléosidiques ou nucléotidiques, inhibiteurs de transcriptase inverse du VIH non nucléosidiques, inhibiteurs d'intégrase du VIH, inhibiteurs de fusion du VIH, inhibiteurs d'entrée du VIH et inhibiteurs de maturation du VIH.

21. Composé selon l'une quelconque des revendications 1 à 18, ou un sel pharmaceutiquement acceptable de celui-ci, pour utilisation dans une méthode de traitement d'une infection par le VIH ou de traitement, de prophylaxie ou de retardement de l'apparition ou de la progression du SIDA ou du syndrome pré-sida chez un sujet humain.

22. Composé selon l'une quelconque des revendications 1 à 18, ou un sel pharmaceutiquement acceptable de celui-ci, pour utilisation dans le déclenchement d'une dimérisation GAG-POL dans des cellules infectées par le VIH chez un sujet humain.

23. Composé selon l'une quelconque des revendications 1 à 18, ou un sel pharmaceutiquement acceptable de celui-ci, pour utilisation dans l'élimination sélective de cellules infectées par le VIH, exprimant GAG-POL, sans cytotoxicité concomitante de cellules naïves pour le VIH chez un sujet humain.

24. Composé selon l'une quelconque des revendications 1 à 18, ou un sel pharmaceutiquement acceptable de celui-ci, pour utilisation dans l'augmentation de la suppression d'une virémie de VIH chez un sujet humain dont la virémie est supprimée par l'administration d'un ou de plusieurs agent antiviraux VIH compatibles.

25. Composé selon l'une quelconque des revendications 1 à 18, ou un sel pharmaceutiquement acceptable de celui-ci, pour utilisation en thérapie.
